# EUROPEAN PATENT APPLICATION

(11) **EP 4 253 371 A1**
(43) Date of publication of application: **04.10.2023**
(21) Application number: 21898273.4
(22) Date of filing: 29.07.2021
(51) Int. Cl.: C07D 307/91, C07D 407/04, C07D 407/10, H01L 51/00, H01L 51/50

(54) **NOVEL ORGANIC COMPOUND, AND ORGANIC LIGHT-EMITTING DEVICE COMPRISING SAME**

(30) Priority: 26.11.2020 KR 20200161336; 27.07.2021 KR 20210098698
(71) Applicant: SFC Co., Ltd., Cheongju-si, Chungcheongbuk-do 28122 (KR)
(72) Inventor: KIM, Si-in, Cheongju-si Chungcheongbuk-do 28122 (KR); LEE, Se-jin, Cheongju-si Chungcheongbuk-do 28122 (KR); PARK, Seok-bae, Cheongju-si Chungcheongbuk-do 28122 (KR); KIM, Hee-dae, Cheongju-si Chungcheongbuk-do 28122 (KR); CHOI, Yeong-tae, Cheongju-si Chungcheongbuk-do 28122 (KR); KIM, Ji-yung, Cheongju-si Chungcheongbuk-do 28122 (KR); KIM, Kyungtae, Cheongju-si Chungcheongbuk-do 28122 (KR); KIM, Myeong-jun, Cheongju-si Chungcheongbuk-do 28122 (KR); KIM, Kyeong-hyeon, Cheongju-si Chungcheongbuk-do 28122 (KR); LEE, Yu-rim, Cheongju-si Chungcheongbuk-do 28122 (KR); LEE, Seung-soo, Cheongju-si Chungcheongbuk-do 28122 (KR); LEE, Tae Gyun, Cheongju-si Chungcheongbuk-do 28122 (KR); KIM, Joon-ho, Cheongju-si Chungcheongbuk-do 28122 (KR)
(74) Representative: Epping - Hermann - Fischer
(86) International application number: PCT/KR2021/009864
(87) International publication number: WO 2022/114444

(57) **Abstract**

The present invention relates to a novel heterocyclic compound usable in an organic light-emitting device and to an organic light-emitting device comprising same, wherein [chemical formula A] and [chemical formula B] are as described in the detailed description of the invention.

## Description

### Technical Field

The present disclosure relates to a novel compound useful for an organic light-emitting diode and, more specifically, to a novel compound that can be used as a host material in an organic light-emitting diode and allows for excellent diode characteristics including high luminous efficiency, low driving voltage, and high longevity, and an organic light-emitting diode including same.

### Background Art

Organic light-emitting diodes (OLEDs), based on self-luminescence, enjoy the advantage of having a wide viewing angle and being able to be made thinner and lighter than liquid crystal displays (LCDs). In addition, an OLED display exhibits a very fast response time. Accordingly, OLEDs find applications in the illumination field as well as the full-color display field.

In general, the term "organic light-emitting phenomenon" refers to a phenomenon in which electrical energy is converted to light energy by means of an organic material. An OLED using the organic light-emitting phenomenon has a structure usually comprising an anode, a cathode, and an organic material layer interposed therebetween. In this regard, the organic material layer may be, for the most part, of a multilayer structure consisting of different materials, for example, a hole injecting layer, a hole transport layer, a light-emitting layer, an electron transport layer, and an electron injecting layer, in order to improve the efficiency and stability of the organic light-emitting diode (OLED). In the organic light-emitting diode having such a structure, when a voltage is applied between the two electrodes, a hole injected from the anode migrates to the organic layer while an electron is released from the cathode and moves toward the organic layer. In the luminescent zone, the hole and the electron recombine to produce an exciton. When the exciton returns to the ground state from the excited state, the molecule of the organic layer emits light. Such an organic light-emitting diode is known to have characteristics such as self-luminescence, high luminance, high efficiency, low driving voltage, a wide viewing angle, high contrast, and high-speed response.

Materials used as organic layers in OLEDs may be divided into luminescent materials and charge transport materials, for example, a hole injection material, a hole transport material, an electron injection material, and an electron transport material. As for the luminescent materials, there are two main families of OLED: those based on small molecules and those employing polymers. The light-emitting mechanism forms the basis for classification of the luminescent materials as fluorescent or phosphorescent materials, which use excitons in singlet and triplet states, respectively.

Meanwhile, when a single material is employed as the luminescent material, intermolecular actions cause the wavelength of maximum luminescence to shift toward a longer wavelength, decreasing color purity or attenuating light with consequent reduction in the efficiency of the diode. In this regard, a host-dopant system may be used as a luminescent material so as to increase the color purity and the light emission efficiency through energy transfer.

This is based on the principle whereby, when a dopant is smaller in energy band gap than a host accounting for the light-emitting layer, the addition of a small amount of the dopant to the host generates excitons from the light-emitting layer so that the excitons are transported to the dopant, emitting light at high efficiency. Here, light of desired wavelengths can be obtained depending on the kind of dopant because the wavelength of the host moves to the wavelength range of the dopant.

For use as host compounds in a light-emitting layer, heterocyclic compounds have been recently studied. With regard to related art, reference may be made to Korean Patent No. 10-2016-0089693 A (July 28, 2016), which discloses a compound structured to have a dibenzofuran ring moiety bonded to an anthracene ring, and an organic light-emitting diode including same. In addition, Korean Patent No. 10-2017-0055743 A (May 22, 2017) discloses a compound in which an aryl substituent or a heteroaryl substituent is bonded to a fused fluorene ring bearing a heteroatom such as oxygen, nitrogen, sulfur, etc., and an organic light-emitting diode including same.

Despites a variety of types of compounds prepared for use in light emitting layers in organic light emitting diodes including the related arts, there is still a continuing need to develop a novel compound that allows an OLED to be stably driven at a lower voltage with high efficiency and longevity, and an OLED including same.

### Disclosure

### Technical Problem

Therefore, an aspect of the present disclosure is to provide a novel organic compound which can be used as a host material in a light-emitting layer of an organic light-emitting diode.

In addition, another aspect of the present disclosure is to provide an organic light-emitting diode (OLED) having the organic compound as a host material therein and exhibiting characteristics including high luminous efficiency, low-voltage driving, and high longevity.

### Technical Solution

In order to accomplish the purposes, the present disclosure provides an organic compound represented by the following Chemical Formula A or Chemical Formula B: 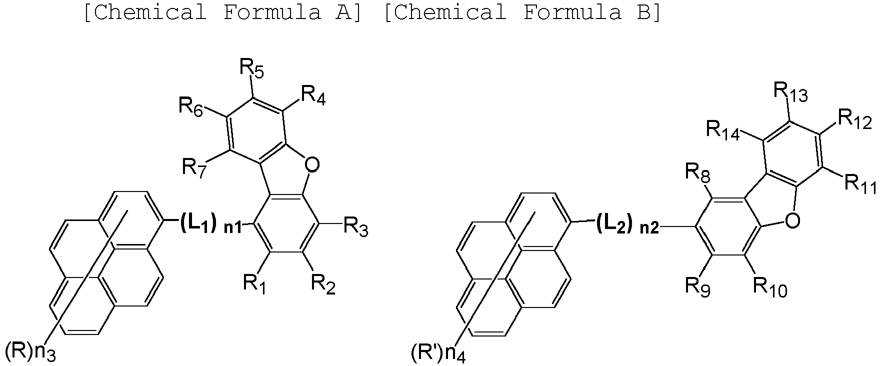 wherein,
R₁ to R₁₄, which are same or different, are each independently at least one selected from a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heterocycloalkyl of 2 to 30 carbon atoms, a substituted or unsubstituted heteroaryl of 2 to 50 carbon atoms, a substituted or unsubstituted alkylamine of 1 to 30 carbon atoms, a substituted or unsubstituted arylamine of 6 to 30 carbon atoms, a substituted or unsubstituted alkylsilyl of 1 to 30 carbon atoms, a substituted or unsubstituted arylsilyl of 6 to 30 carbon atoms, a cyano, a nitro, and a halogen;
linkers L₁ and L₂, which are same or different, are each independently selected from a single bond, a substituted or unsubstituted arylene of 6 to 20 carbon atoms, and a substituted or unsubstituted heteroarylene of 2 to 20 carbon atoms;
n1 and n2, which are same or different, are each independently an integer of 0 to 2 wherein when n1 or n2 is 2, the corresponding linkers L₁'s or L₂'s are same or different,
R and R', which are same or different, are each independently one selected from a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heterocycloalkyl of 2 to 30 carbon atoms, a substituted or unsubstituted heteroaryl of 2 to 50 carbon atoms, a substituted or unsubstituted alkylamine of 1 to 30 carbon atoms, a substituted or unsubstituted arylamine of 6 to 30 carbon atoms, a substituted or unsubstituted alkylsilyl of 1 to 30 carbon atoms, a substituted or unsubstituted arylsilyl of 6 to 30 carbon atoms, a cyano, a nitro, and a halogen;
n3 and n4, which are same or different, are each independently an integer of 1 to 9 where when n3 or n4 are 2 or more, the corresponding Rs or R's are same or different,
wherein the term 'substituted' in the expression "a substituted or unsubstituted" means having at least one substituent selected from the group consisting of a deuterium atom, a cyano, a halogen, a hydroxy, a nitro, an alkyl of 1 to 24 carbon atoms, a hydrogenated alkyl of 1 to 24 carbon atoms, an alkenyl of 1 to 24 carbon atoms, an alkynyl of 1 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 2 to 24 carbon atoms, an alkoxy of 1 to 24 carbon atoms, an alkylamino of 1 to 24 carbon atoms, a diarylamino of 12 to 24 carbon atoms, a diheteroarylamino of 2 to 24 carbon atoms, an aryl(heteroaryl)amino of 7 to 24 carbon atoms, an alkylsilyl of 1 to 24 carbon atoms, an arylsilyl of 6 to 24 carbon atoms, an aryloxy of 6 to 24 carbon atoms, and an arylthionyl of 6 to 24 carbon atoms.

### Advantageous Effects

When used as a host material, the novel compound represented by Chemical Formula A or B according to the present disclosure allows for the provision of an organic light-emitting diode that can be driven at a lower voltage with improved luminous efficiency and longevity, compared to conventional organic light-emitting diodes.

### Description of Drawings

Figure is a schematic diagram of an OLED according to some embodiments of the present disclosure.

### Mode for Invention

Hereinafter, exemplary embodiments which can be easily implemented by those skilled in the art will be described with reference to the accompanying drawing. In each drawing of the present disclosure, sizes or scales of components may be enlarged or reduced from their actual sizes or scales for better illustration, and known components may not be depicted therein to clearly show features of the present disclosure. Therefore, the present disclosure is not limited to the drawings. When describing the principle of the embodiments of the present disclosure in detail, details of well-known functions and features may be omitted to avoid unnecessarily obscuring the presented embodiments.

In drawings, for convenience of description, sizes of components may be exaggerated for clarity. For example, since sizes and thicknesses of components in drawings are arbitrarily shown for convenience of description, the sizes and thicknesses are not limited thereto. Furthermore, throughout the description, the terms "on" and "over" are used to refer to the relative positioning, and mean not only that one component or layer is directly disposed on another component or layer but also that one component or layer is indirectly disposed on another component or layer with a further component or layer being interposed therebetween. Also, spatially relative terms, such as "below", "beneath", "lower", and "between" may be used herein for ease of description to refer to the relative positioning.

Throughout the specification, when a portion may "comprise" or "include" a certain constituent element, unless explicitly described to the contrary, it may not be construed to exclude another constituent element but may be construed to further include other constituent elements. Further, throughout the specification, the word "on" means positioning on or below the object portion, but does not essentially mean positioning on the lower side of the object portion based on a gravity direction.

The present disclosure provides an organic compound represented by the following Chemical Formula A or Chemical Formula B: 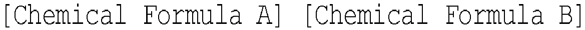 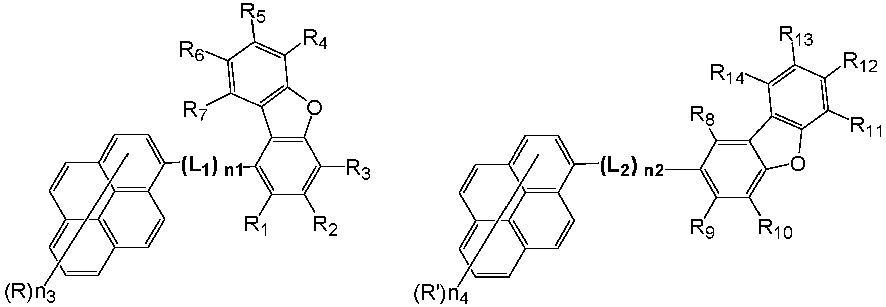 wherein,
R₁ to R₁₄, which are same or different, are each independently at least one selected from a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heterocycloalkyl of 2 to 30 carbon atoms, a substituted or unsubstituted heteroaryl of 2 to 50 carbon atoms, a substituted or unsubstituted alkylamine of 1 to 30 carbon atoms, a substituted or unsubstituted arylamine of 6 to 30 carbon atoms, a substituted or unsubstituted alkylsilyl of 1 to 30 carbon atoms, a substituted or unsubstituted arylsilyl of 6 to 30 carbon atoms, a cyano, a nitro, and a halogen;
linkers L₁ and L₂, which are same or different, are each independently selected from a single bond, a substituted or unsubstituted arylene of 6 to 20 carbon atoms, and a substituted or unsubstituted heteroarylene of 2 to 20 carbon atoms;
n1 and n2, which are same or different, are each independently an integer of 0 to 2 wherein when n1 or n2 is 2, the corresponding linkers L₁'s or L₂'s are same or different,
R and R', which are same or different, are each independently one selected from a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heterocycloalkyl of 2 to 30 carbon atoms, a substituted or unsubstituted heteroaryl of 2 to 50 carbon atoms, a substituted or unsubstituted alkylamine of 1 to 30 carbon atoms, a substituted or unsubstituted arylamine of 6 to 30 carbon atoms, a substituted or unsubstituted alkylsilyl of 1 to 30 carbon atoms, a substituted or unsubstituted arylsilyl of 6 to 30 carbon atoms, a cyano, a nitro, and a halogen;
n3 and n4, which are same or different, are each independently an integer of 1 to 9 where when n3 or n4 are 2 or more, the corresponding Rs or R's are same or different.
wherein the term 'substituted' in the expression "a substituted or unsubstituted" means having at least one substituent selected from the group consisting of a deuterium atom, a cyano, a halogen, a hydroxy, a nitro, an alkyl of 1 to 24 carbon atoms, a hydrogenated alkyl of 1 to 24 carbon atoms, an alkenyl of 1 to 24 carbon atoms, an alkynyl of 1 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 2 to 24 carbon atoms, an alkoxy of 1 to 24 carbon atoms, an alkylamino of 1 to 24 carbon atoms, a diarylamino of 12 to 24 carbon atoms, a diheteroarylamino of 2 to 24 carbon atoms, an aryl(heteroaryl)amino of 7 to 24 carbon atoms, an alkylsilyl of 1 to 24 carbon atoms, an arylsilyl of 6 to 24 carbon atoms, an aryloxy of 6 to 24 carbon atoms, and an arylthionyl of 6 to 24 carbon atoms.

The expression indicating the number of carbon atoms, such as "a substituted or unsubstituted alkyl of 1 to 30 carbon atoms", "a substituted or unsubstituted aryl of 5 to 50 carbon atoms", etc. means the total number of carbon atoms of, for example, the alkyl or aryl radical or moiety alone, exclusive of the number of carbon atoms of substituents attached thereto. For instance, a phenyl group with a butyl at the para position falls within the scope of an aryl of 6 carbon atoms, even though it is substituted with a butyl radical of 4 carbon atoms.

As used herein, the term "aryl" means an organic radical derived from an aromatic hydrocarbon by removing one hydrogen that is bonded to the aromatic hydrocarbon. Further, the aromatic system may include a fused ring that is formed by adjacent substituents on the aryl radical.

Concrete examples of the aryl include phenyl, o-biphenyl, m-biphenyl, p-biphenyl, o-terphenyl, m-terphenyl, p-terphenyl, naphthyl, anthryl, phenanthryl, pyrenyl, indenyl, fluorenyl, tetrahydronaphthyl, perylenyl, chrysenyl, naphthacenyl, and fluoranthenyl at least one hydrogen atom of which may be substituted by a deuterium atom, a halogen atom, a hydroxy, a nitro, a cyano, a silyl, an amino (-NH₂, -NH(R), -N(R') (R") wherein R' and R" are each independently an alkyl of 1 to 10 carbon atoms, in this case, called "alkylamino"), an amidino, a hydrazine, a hydrazone, a carboxyl, a sulfonic acid, a phosphoric acid, an alkyl of 1 to 24 carbon atoms, a halogenated alkyl of 1 to 24 carbon atoms, an alkenyl of 2 to 24 carbon atoms, an alkynyl of 2 to 24 carbon atoms, a heteroalkyl of 1 to 24 carbon atoms, an aryl of 6 to 24 carbon atoms, an arylalkyl of 6 to 24 carbon atoms, a heteroaryl of 2 to 24 carbon atoms, or a heteroarylalkyl of 2 to 24 carbon atoms.

The substituent heteroaryl used in the compound of the present disclosure refers to a cyclic aromatic system of 2 to 24 carbon atoms bearing as ring members one to three heteroatoms selected from among N, O, P, Si, S, Ge, Se, and Te. In the aromatic system, two or more rings may be fused. One or more hydrogen atoms on the heteroaryl may be substituted by the same substituents as on the aryl.

In addition, the term "heteroaromatic ring", as used herein, refers to an aromatic hydrocarbon ring bearing as aromatic ring members 1 to 3 heteroatoms selected particularly from N, O, P, Si, S, Ge, Se, and Te.

As used herein, the term "alkyl" refers to an alkane missing one hydrogen atom and includes linear or branched structures. Examples of the alkyl substituent useful in the present disclosure include methyl, ethyl, propyl, isopropyl, isobutyl, sec-butyl, tert-butyl, pentyl, iso-amyl, and hexyl. At least one hydrogen atom of the alkyl may be substituted by the same substituent as in the aryl.

The term "cyclo" as used in substituents of the present disclosure, such as cycloalkyl, cycloalkoxy, etc., refers to a structure responsible for a mono- or polycyclic ring of saturated hydrocarbons such as alkyl, alkoxy, etc. Concrete examples of cycloalkyl radicals include cyclopropyl, cyclobutyl, cyclopentyl, cyclohexyl, methylcyclopentyl, methylcyclohexyl, ethylcyclopentyl, ethylcyclohexyl, adamantyl, dicyclopentadienyl, decahydronaphthyl, norbornyl, bornyl, and isobornyl. One or more hydrogen atoms on the cycloalkyl may be substituted by the same substituents as on the aryl and it can be applied to cycloalkoxy, as well.

The term "alkoxy" as used in the compounds of the present disclosure refers to an alkyl or cycloalkyl singularly bonded to oxygen. Concrete examples of the alkoxy include methoxy, ethoxy, propoxy, isobutoxy, sec-butoxy, pentoxy, iso-amyloxy, hexyloxy, cyclobutyloxy, cyclopentyloxy, adamantyloxy, dicyclopentyloxy, bornyloxy, and isobornyloxy. One or more hydrogen atoms on the alkoxy may be substituted by the same substituents as on the aryl.

Concrete examples of the arylalkyl used in the compounds of the present disclosure include phenylmethyl(benzyl), phenylethyl, phenylpropyl, naphthylmethyl, and naphthylethyl. One or more hydrogen atoms on the arylalkyl may be substituted by the same substituents as on the aryl.

Concrete examples of the silyl radicals used in the compounds of the present disclosure include trimethylsilyl, triethylsilyl, triphenylsilyl, trimethoxysilyl, dimethoxyphenylsilyl, diphenylmethylsilyl, diphenylvinlysilyl, methylcyclobutylsilyl, and dimethyl furylsilyl. One or more hydrogen atoms on the silyl may be substituted by the same substituents as on the aryl.

As used herein, the term "alkenyl" refers to an unsaturated hydrocarbon group that contains a carbon-carbon double bond between two carbon atoms and the team "alkynyl" refers to an unsaturated hydrocarbon group that contains a carbon-carbon triple bond between two carbon atoms.

As used herein, the term "alkylene" refers to an organic aliphatic radical regarded as derived from a linear or branched saturated hydrocarbon alkane by removal of two hydrogen atoms from different carbon atoms. Concrete examples of the alkylene include methylene, ethylene, propylene, isopropylene, isobutylene, sec-butylene, tert-butylene, pentylene, iso-amylene, hexylene, and so on. One or more hydrogen atoms on the alkylene may be substituted by the same substituents as on the aryl.

Furthermore, as used herein, the term "diarylamino" refers to an amine radical having two identical or different aryl groups bonded to the nitrogen atom thereof, the term "diheteroarylamino" refers to an amine radical having two identical or different heteroaryl groups bonded to the nitrogen atom thereof, and the term "aryl(heteroaryl)amino" refers to an amine radical having an aryl group and a heteroaryl group both bonded to the nitrogen atom thereof.

As more particular examples accounting for the term "substituted" in the expression "substituted or unsubstituted" used for compounds of Chemical Formulas A and B, the compounds may be substituted by at least one substituents selected from the group consisting of a deuterium atom, a cyano, a halogen, a hydroxy, a nitro, an alkyl of 1 to 12 carbon atoms, a halogenated alkyl of 1 to 12 carbon atoms, an alkenyl of 2 to 12 carbon atoms, an alkynyl of 2 to 12 carbon atoms, a cycloalkyl of 3 to 12 carbon atoms, a heteroalkyl of 1 to 12 carbon atoms, an aryl of 6 to 18 carbon atoms, an arylalkyl of 7 to 20 carbon atoms, an alkylaryl of 7 to 20 carbon atoms, a heteroaryl of 2 to 18 carbon atoms, a heteroarylalkyl of 2 to 18 carbon atoms, an alkoxy of 1 to 12 carbon atoms, an alkylamino of 1 to 12 carbon atoms, a diarylamino of 12 to 18 carbon atoms, a diheteroarylamino of 2 to 18 carbon atoms, an aryl(heteroaryl)amino of 7 to 18 carbon atoms, an alkylsilyl of 1 to 12 carbon atoms, an arylsilyl of 6 to 18 carbon atoms, an aryloxy of 6 to 18 carbon atoms, and an arylthionyl of 6 to 18 carbon atoms.

In the present disclosure, the organic compound represented by Chemical Formula A is characterized by the structure in which a linker L₁ is connected to a substituted or unsubstituted pyrene ring moiety at a specific position (see the following Structural Formula C) and to a substituted or unsubstituted dibenzofuran moiety at position 1, and the organic compound represented by Chemical Formula B is characterized by the structure in which a linker L₂ is connected to a substituted or unsubstituted pyrene ring moiety at a specific position (see the following Structural Formula C) and to a substituted or unsubstituted dibenzofuran moiety at position 2. 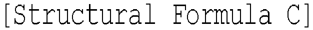 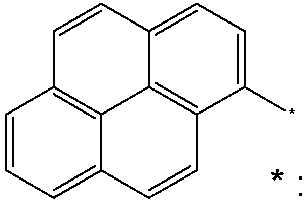 bonding site to linker L₁ or L₂

In the present disclosure, the compound represented by Chemical Formula A may bear at least one deuterium atom and the compound represented by Chemical Formula B may bear at least one deuterium atom.

More specifically, at least one of R₁ to R₇ in Chemical Formula A may be a substituent bearing a deuterium atom and at least one of R₈ to R₁₄ in Chemical Formula B may be a substituent bearing a deuterium atom.

In addition, when the compound represented by Chemical Formula A has at least one deuterium atom or when the compound represented by Chemical Formula B has at least one deuterium atom, at least one R in Chemical Formula A may be a substituent bearing a deuterium atom or at least one R' in Chemical Formula B may be a substituent bearing a deuterium atom.

In an embodiment according to the present disclosure, R₁ to R₁₄, R, and R', which may be same or different, are each independently a substituent selected from a hydrogen atom, a deuterium atom, a substituted or unsubstituted alkyl of 1 to 10 carbon atoms, a substituted or unsubstituted aryl of 6 to 18 carbon atoms, a substituted or unsubstituted heteroaryl of 2 to 18 carbon atoms, a substituted or unsubstituted alkylsilyl of 1 to 15 carbon atoms, a substituted or unsubstituted arylsilyl of 6 to 20 carbon atoms, a cyano, and a halogen.

In an embodiment according to the present disclosure, at least one of R₁ to R₇ in Chemical Formula A may be a substituted or unsubstituted aryl of 6 to 18 carbon atoms and at least one of R₈ to R₁₄ in Chemical Formula B may be a substituted or unsubstituted aryl of 6 to 18 carbon atoms.

In an embodiment according to the present disclosure, the linkers L₂ and L₂ in Chemical Formulas A and B may be each a single bond or any one selected from the following Structural Formulas 1 to 5:

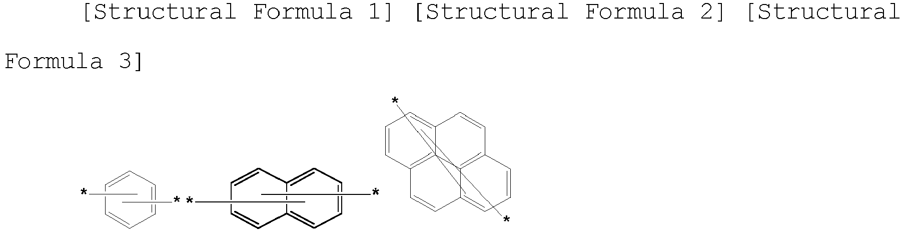

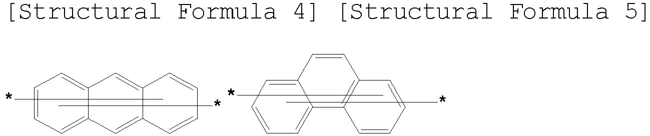

Each of the unsubstituted carbon atoms of the aromatic ring moiety in Structural Formulas 1 to 5 may be bound with a hydrogen atom or a deuterium atom.

In an embodiment according to the present disclosure, the linkers L₁ and L₂ may each be a single bond.

In an embodiment according to the present disclosure, n3 and n4 in Chemical Formulas A and B may each be 1.

In an embodiment according to the present disclosure, at least one R in Chemical Formula A may be a substituted or unsubstituted aryl of 6 to 18 carbon atoms and at least one R' in Chemical Formula B may be a substituted or unsubstituted aryl of 6 to 18 carbon atoms. In this regard, n3 and n4 in Chemical Formula A and Chemical Formula B may each be 1, R in Chemical Formula A may be a substituted or unsubstituted aryl of 6 to 18 carbon atoms, and R' in Chemical Formula B may be a substituted or unsubstituted aryl of 6 to 18 carbon atoms.

In an embodiment according to the present disclosure, the organic compound represented by Chemical Formula A or Chemical Formula B may be a compound represented by the following Chemical Formula A-1 or Chemical Formula B-1: 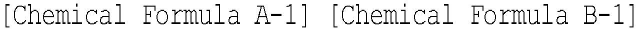 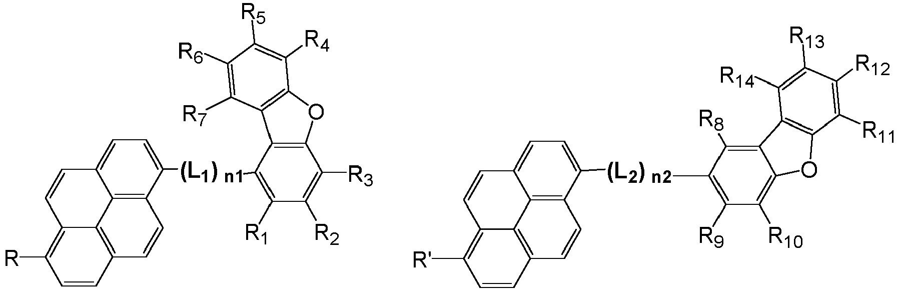
wherein, the substituents R₁ to R₁₄, the linkers L₁ and L₂, and n1 and n2 are as defined in Chemical Formula A or Chemical Formula B, and
R and R' are each a substituted or unsubstituted aryl of 6 to 18 carbon atoms.

In an embodiment according to the present disclosure, n3 and n4 in Chemical Formula A and Chemical Formula B may each be 1, at least one of R₁ to R₇ and R in Chemical Formula A may be a deuterated aryl of 6 to 18 carbon atoms, and at least one of R₈ to R₁₄ and R' in Chemical Formula B may be a deuterated aryl of 6 to 18 carbon atoms.

In an embodiment according to the present disclosure, n3 and n4 in Chemical Formula A and Chemical Formula B may each be 1, R in Chemical Formula A may be a substituted or unsubstituted heteroaryl of 2 to 18 carbon atoms, and R' in Chemical Formula B may be a substituted or unsubstituted heteroaryl of 2 to 18 carbon atoms.

The compound represented by Chemical Formula A or Chemical Formula B according to the present disclosure may be any one selected from Chemical Formula 1 to Chemical Formula 240:

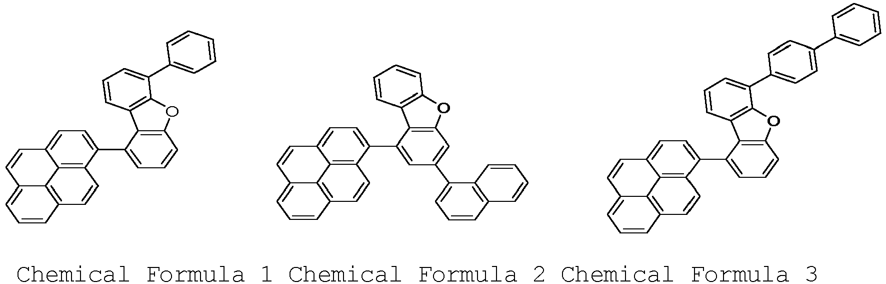

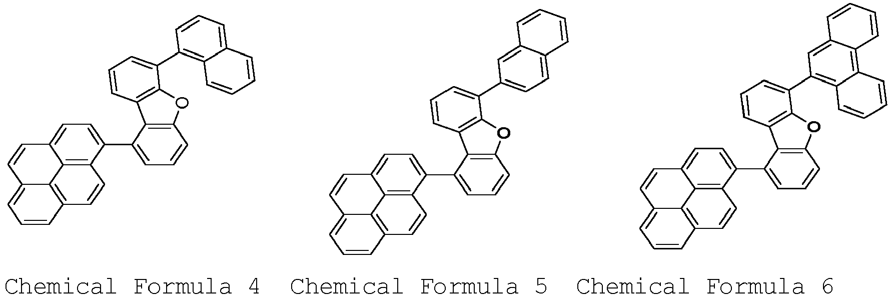

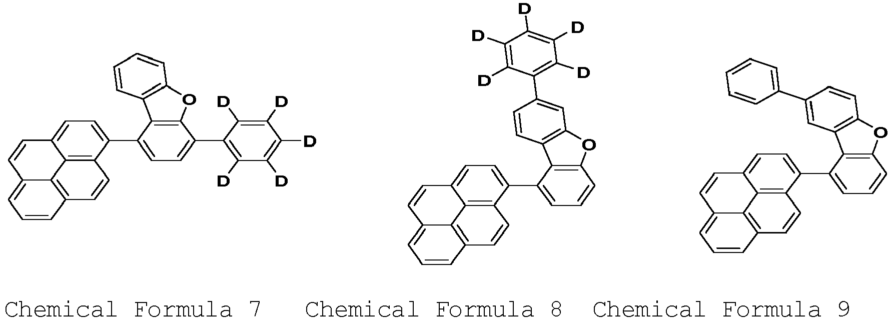

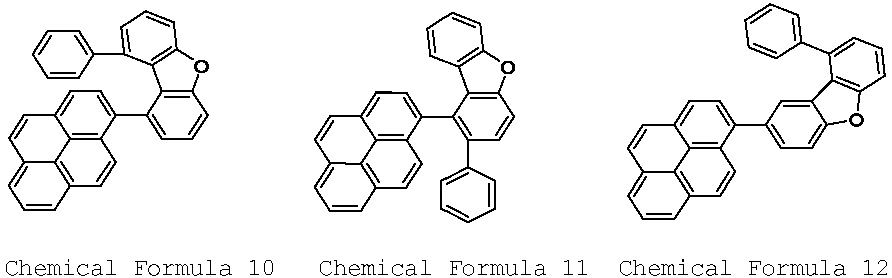

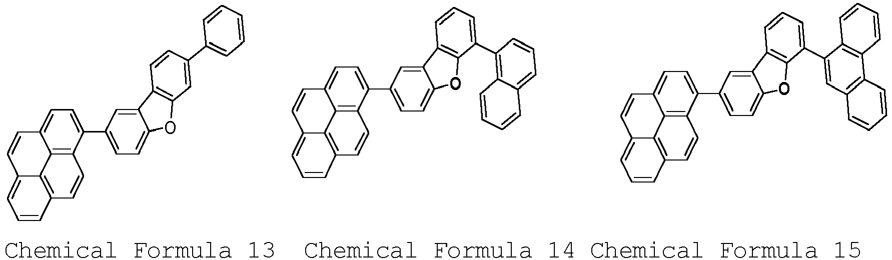

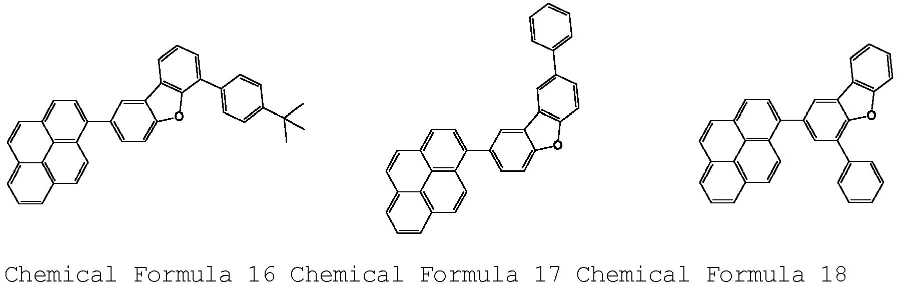

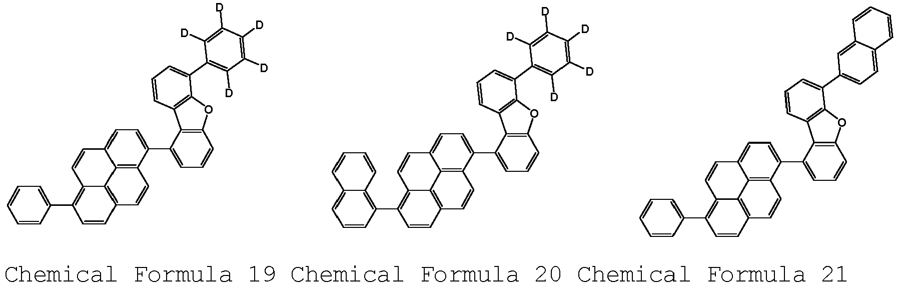

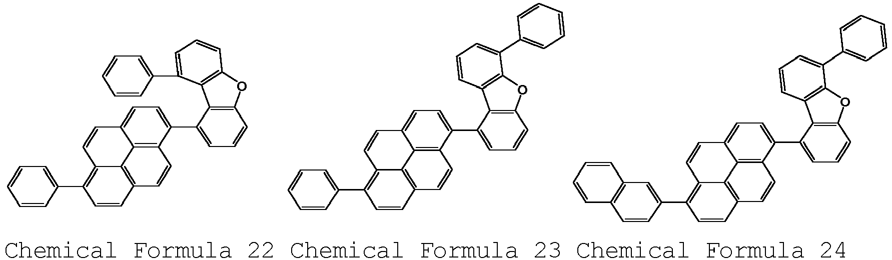

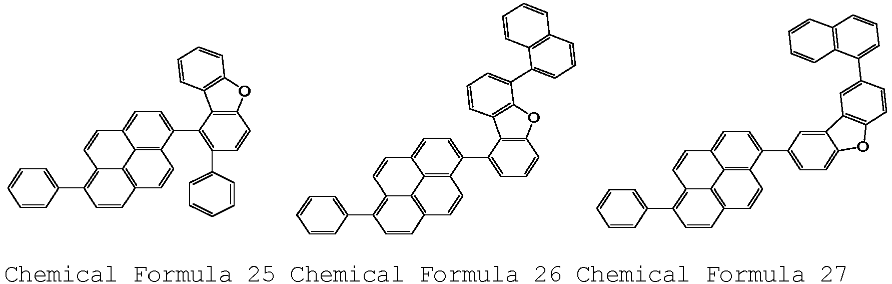

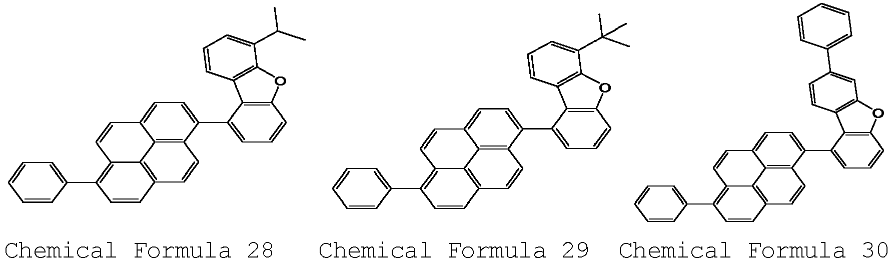

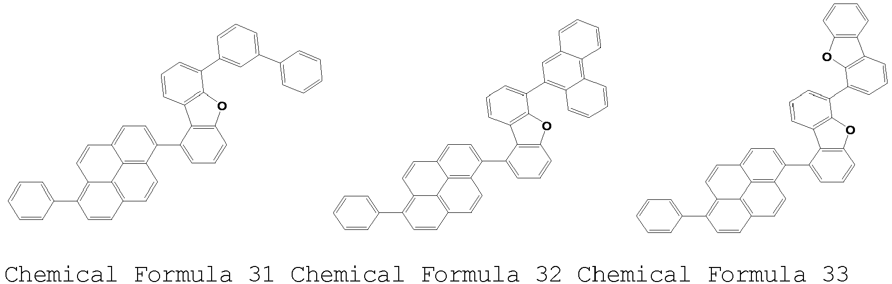

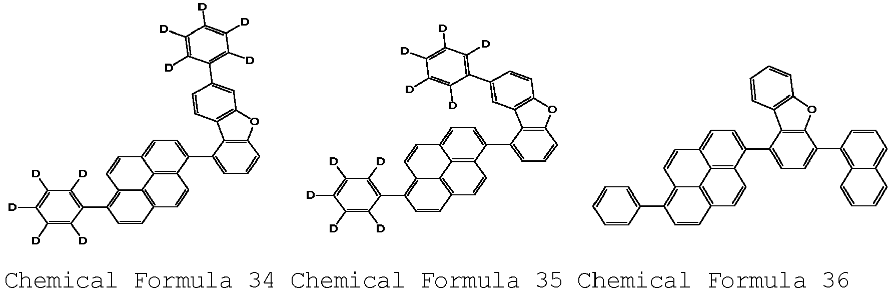

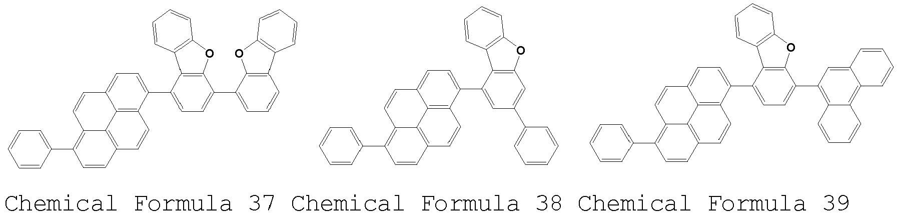

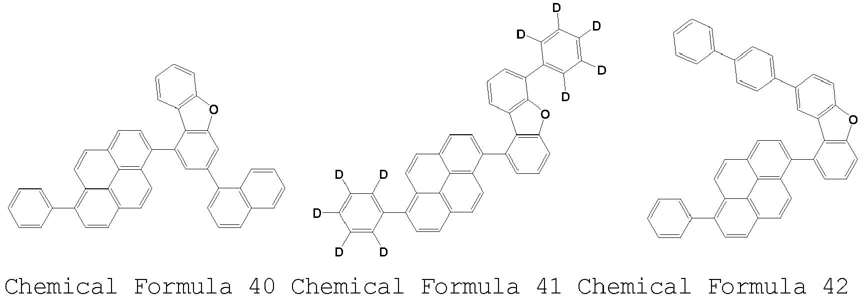

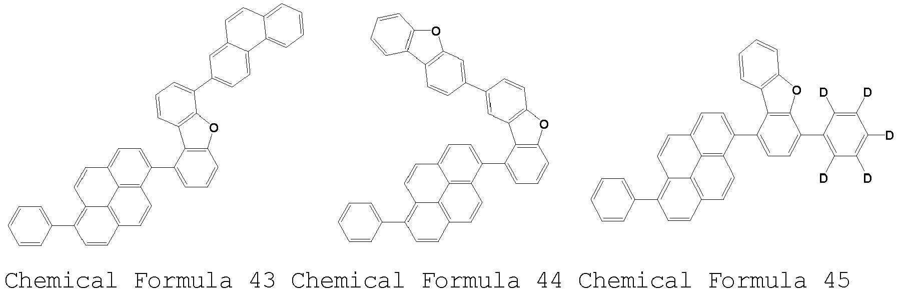

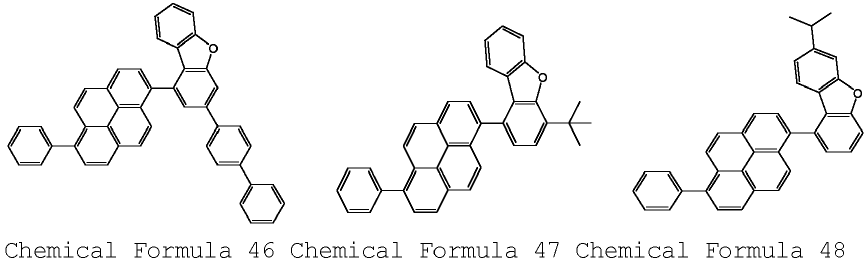

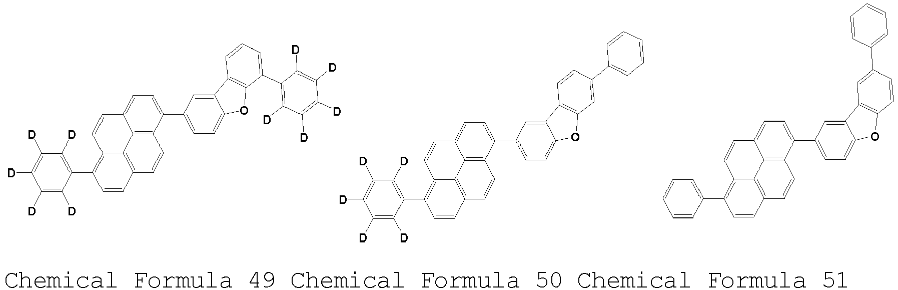

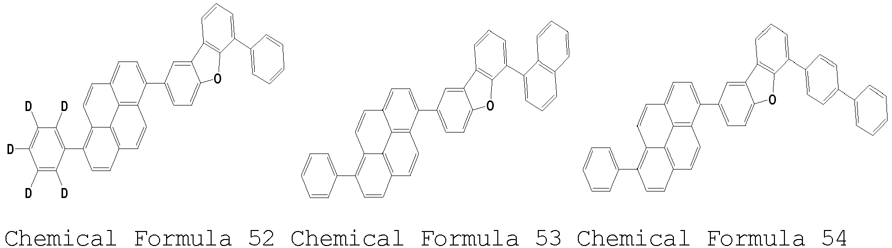

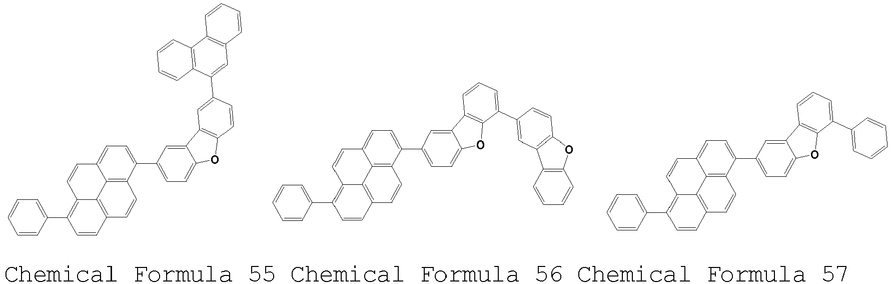

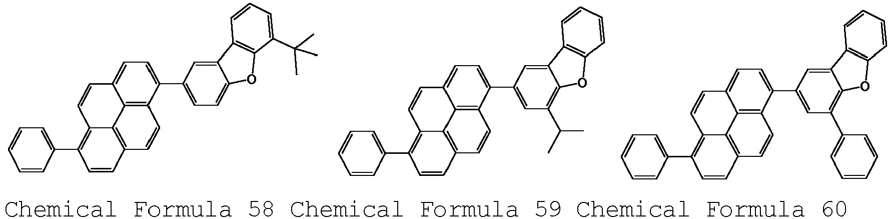

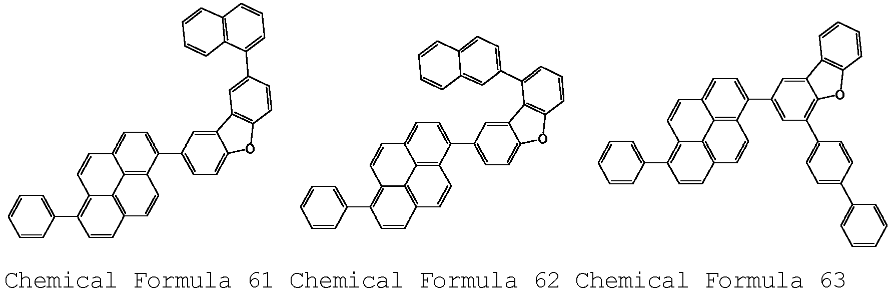

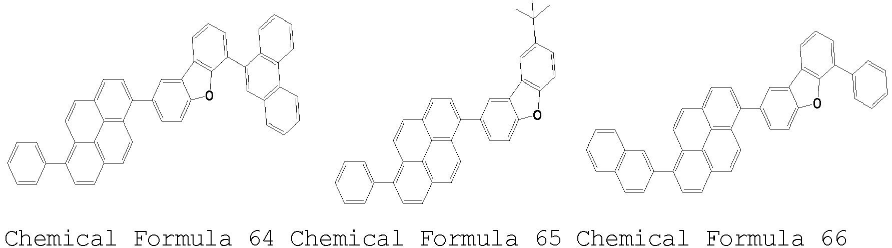

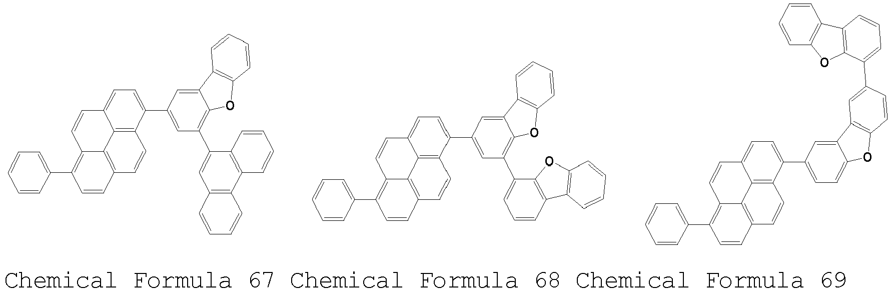

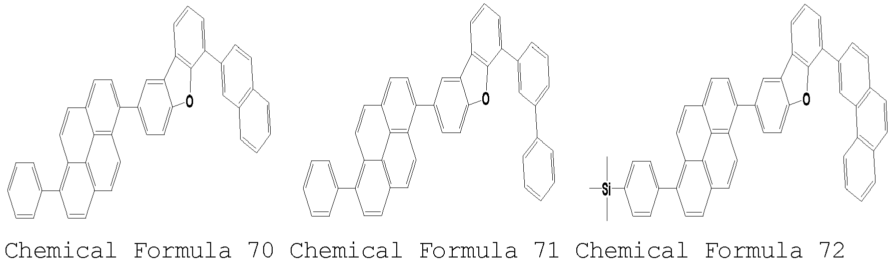

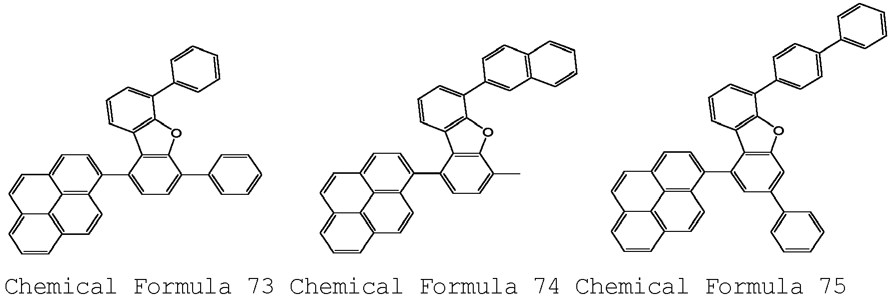

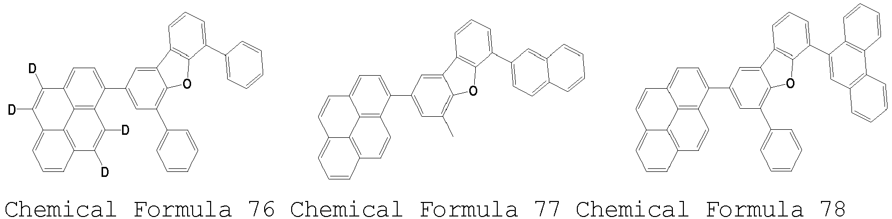

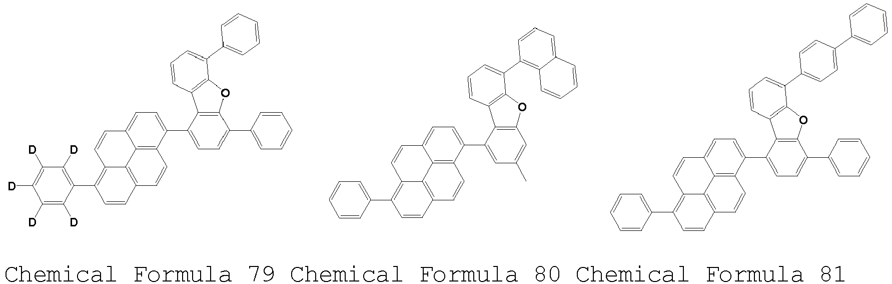

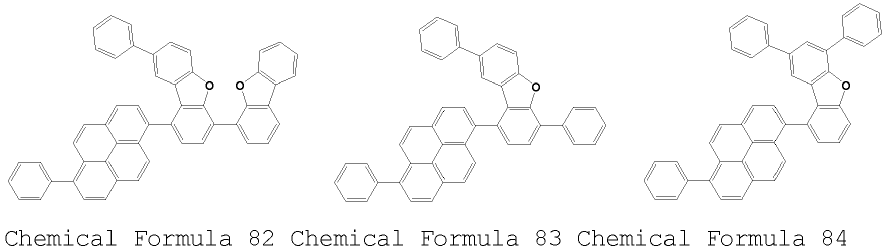

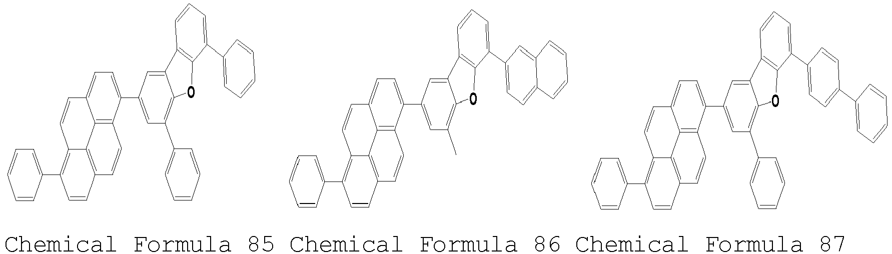

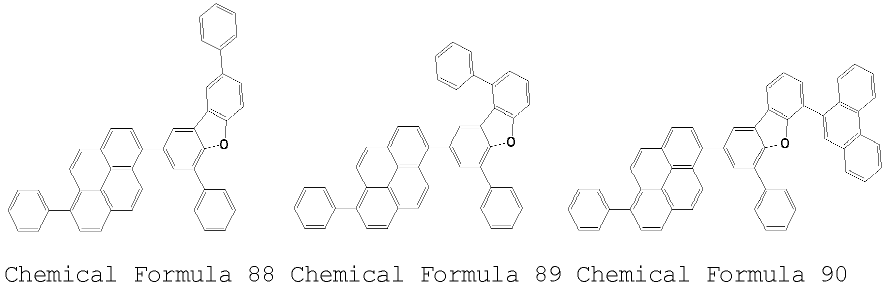

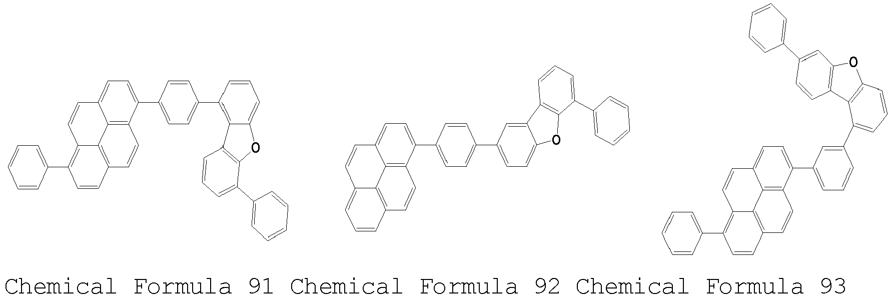

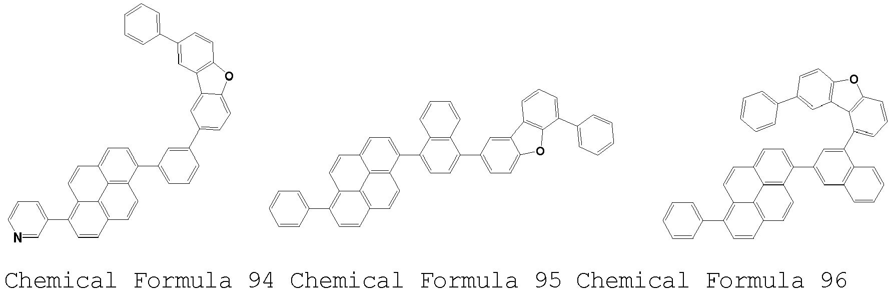

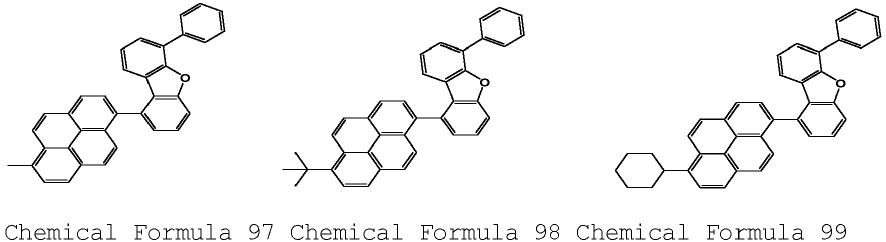

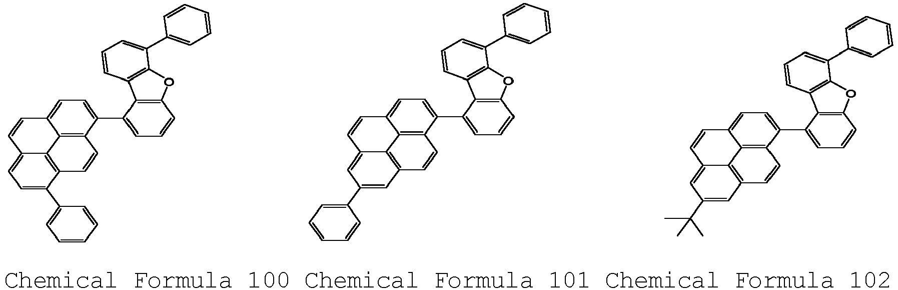

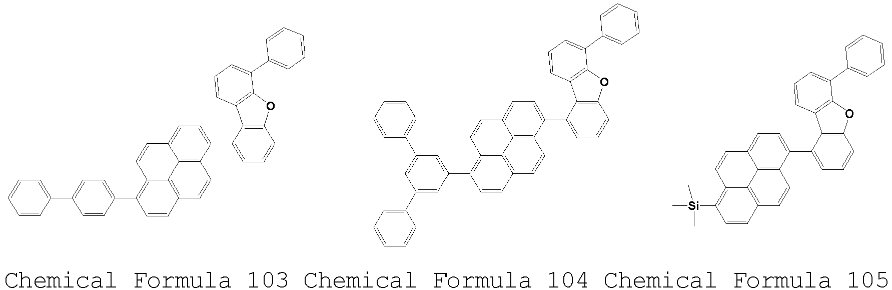

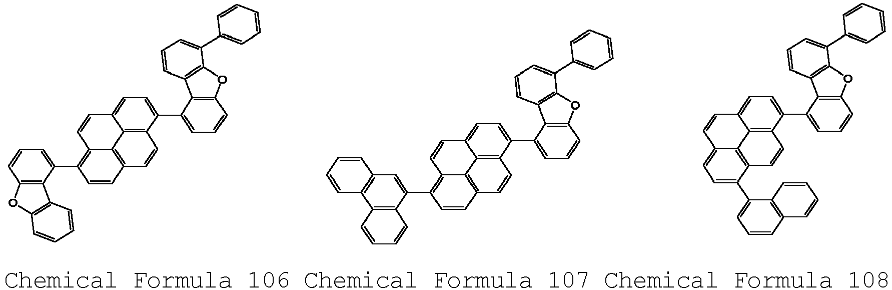

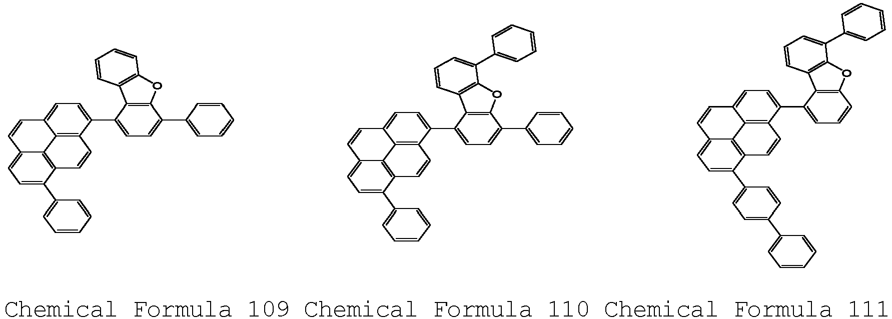

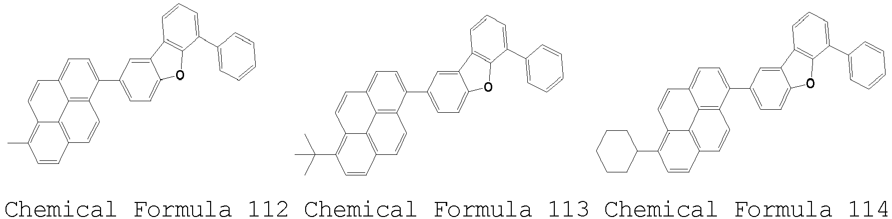

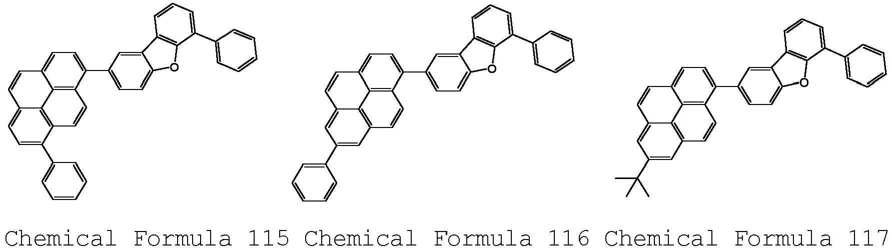

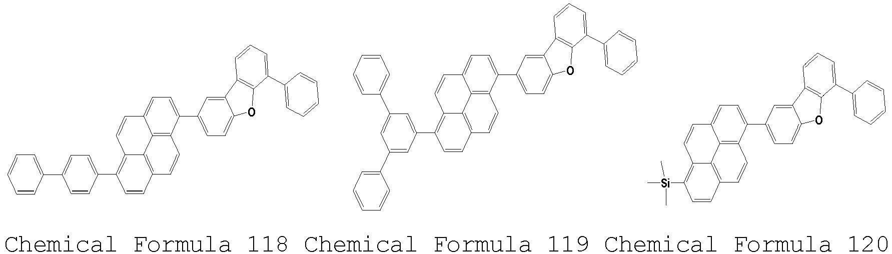

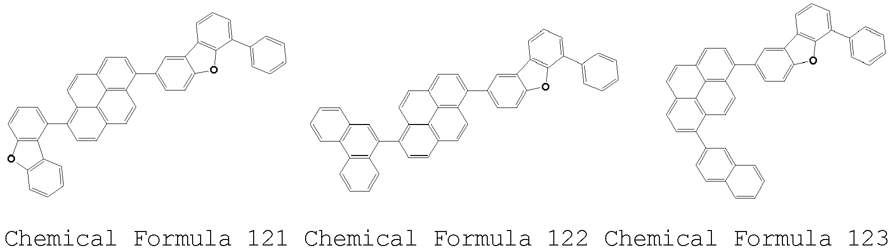

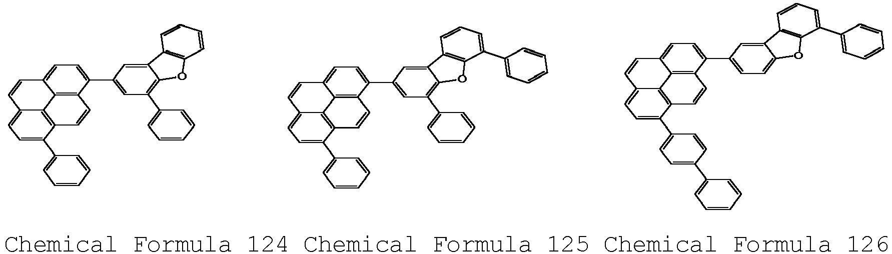

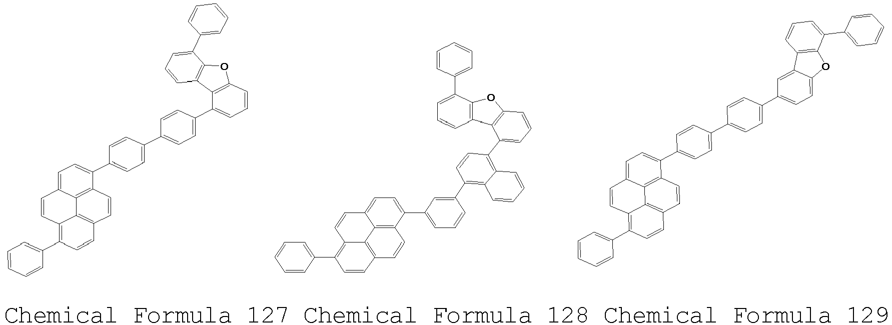

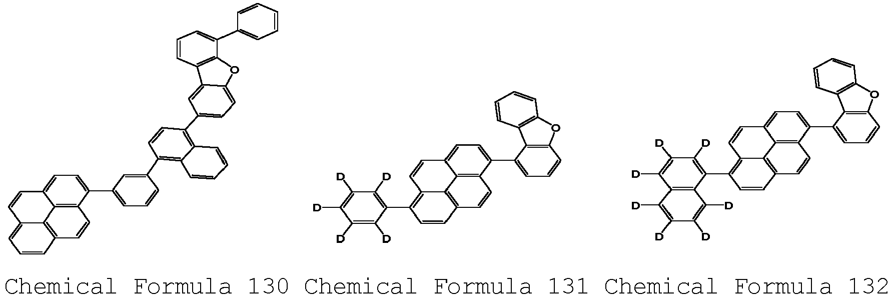

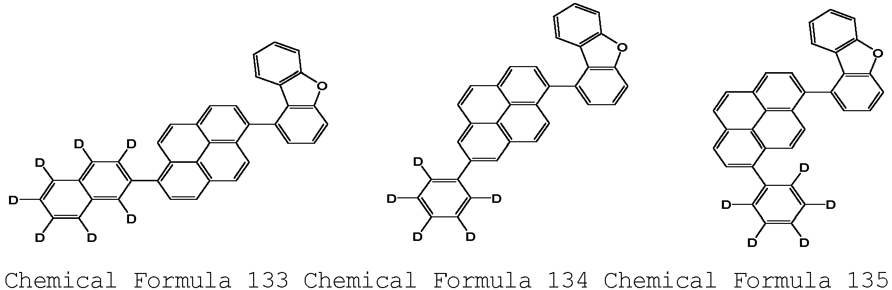

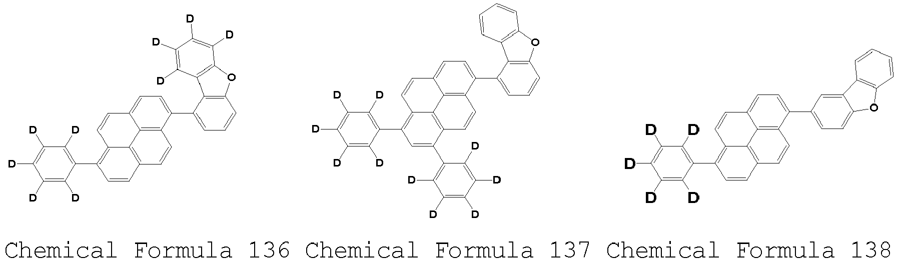

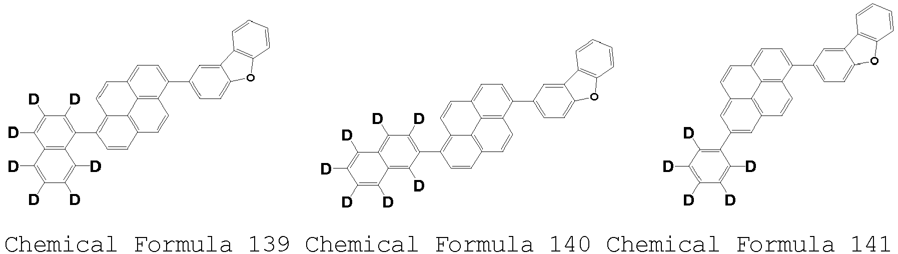

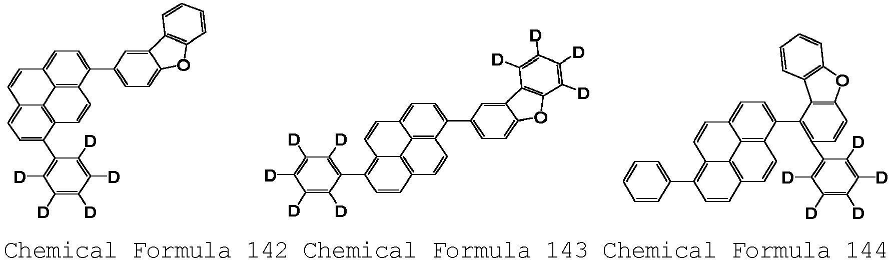

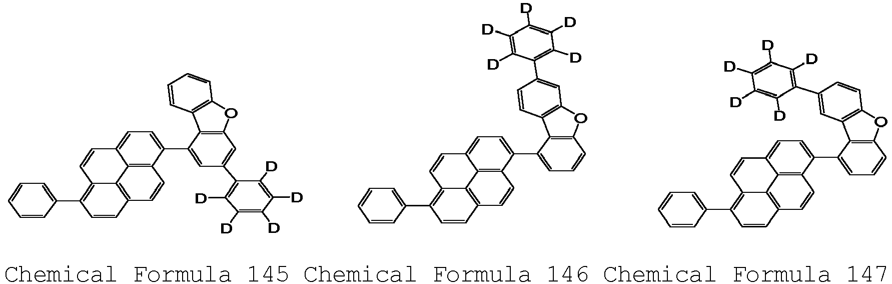

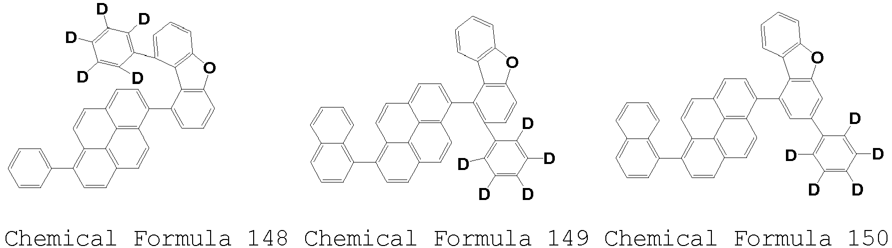

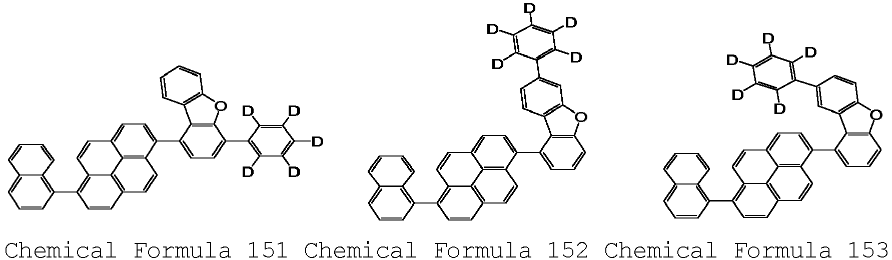

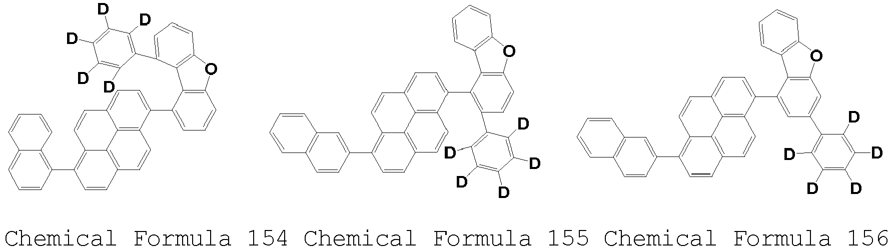

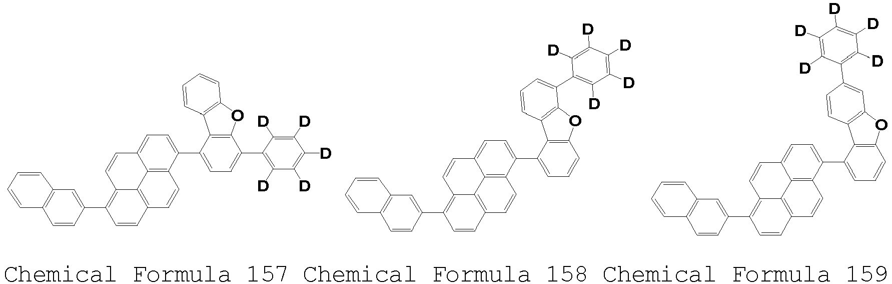

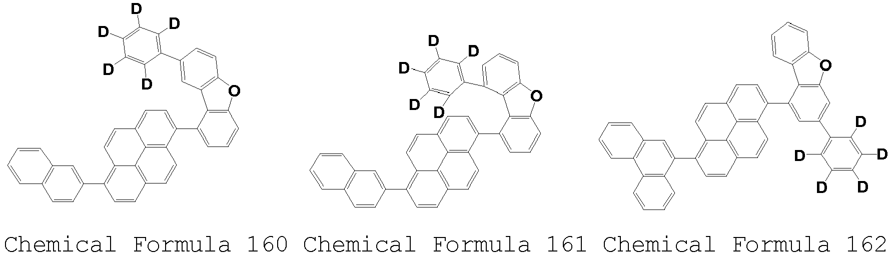

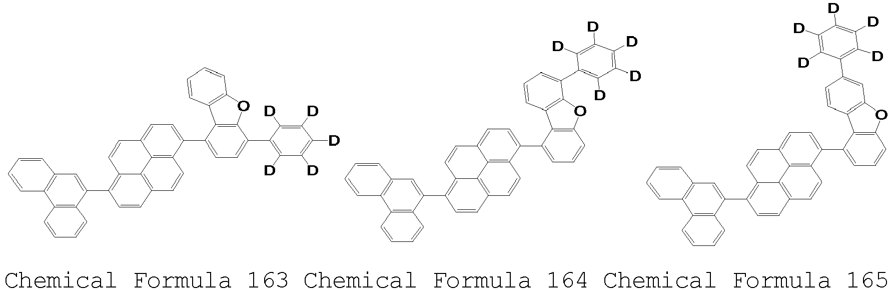

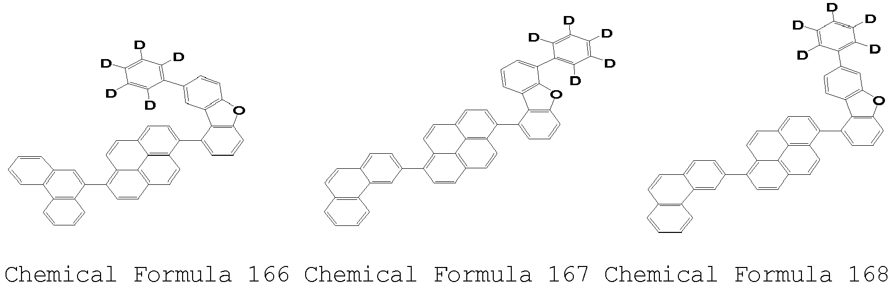

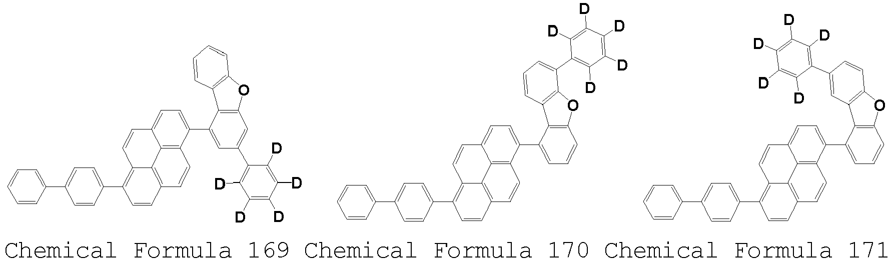

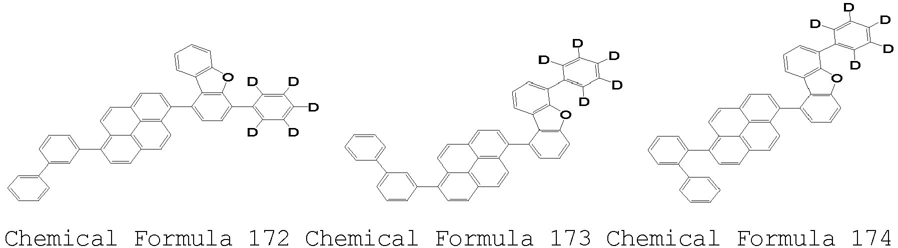

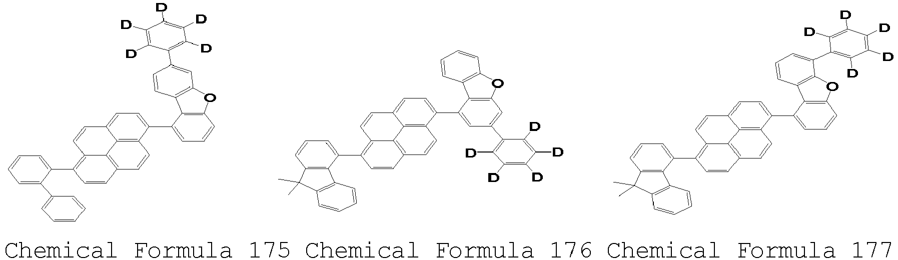

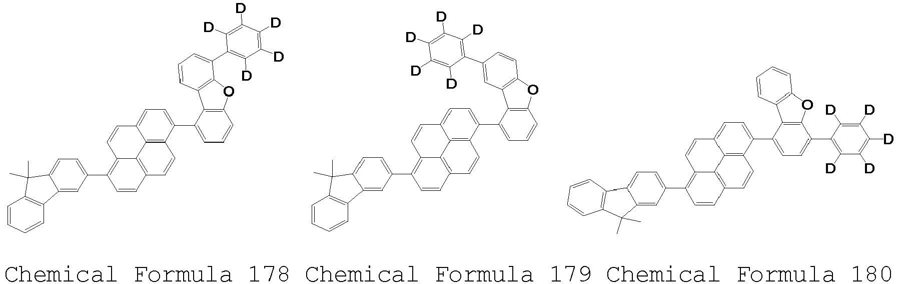

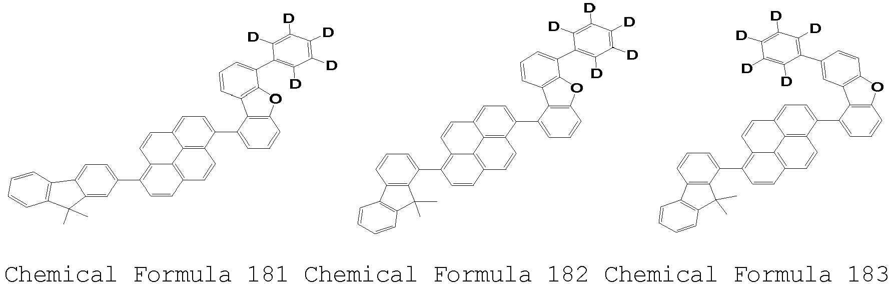

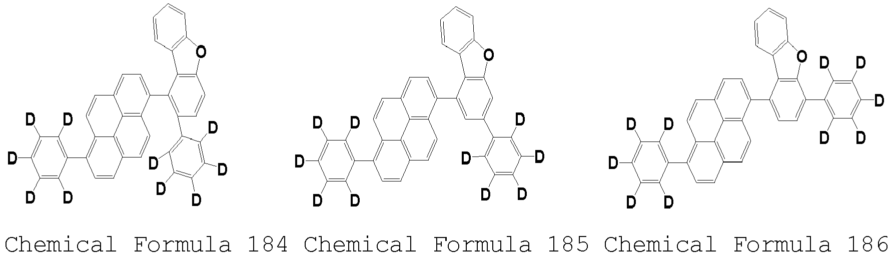

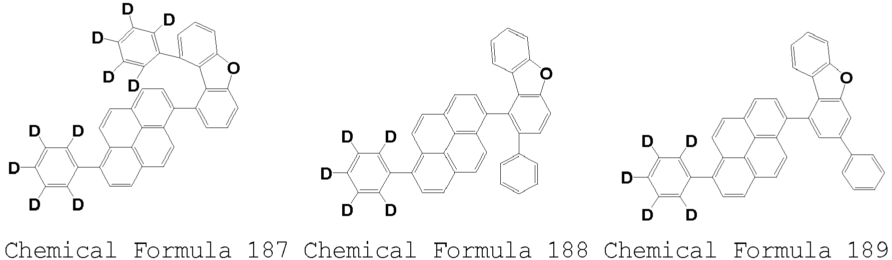

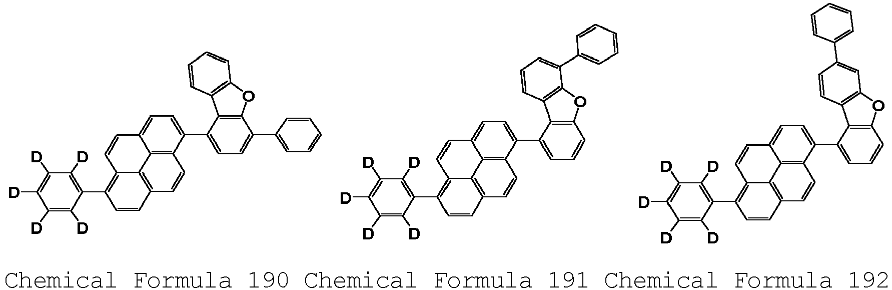

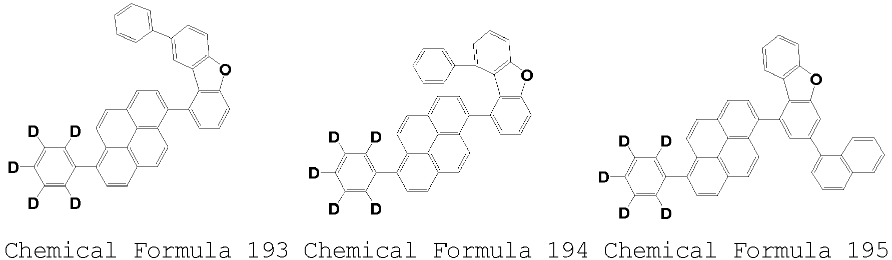

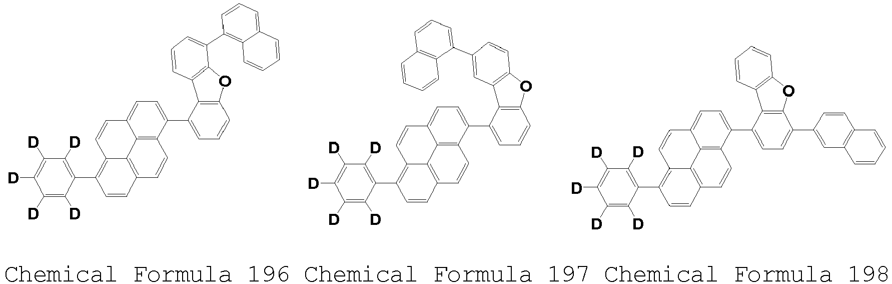

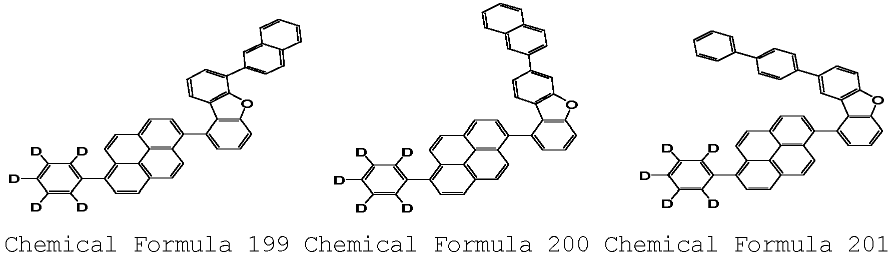

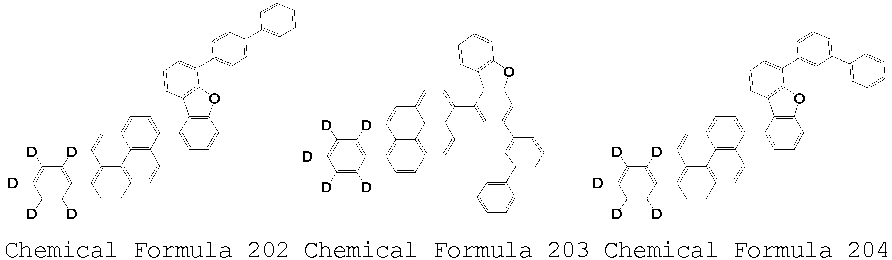

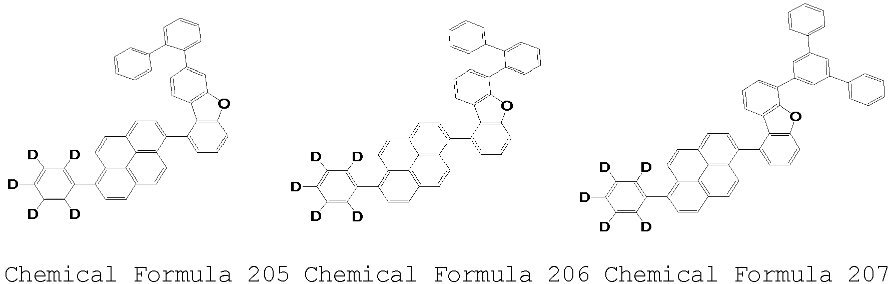

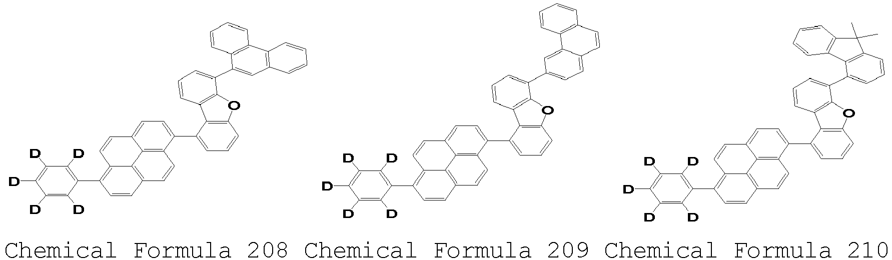

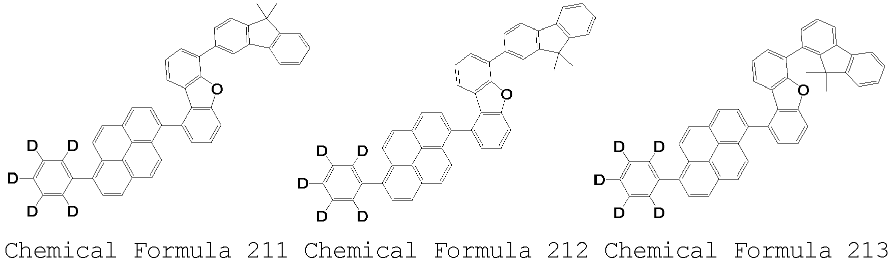

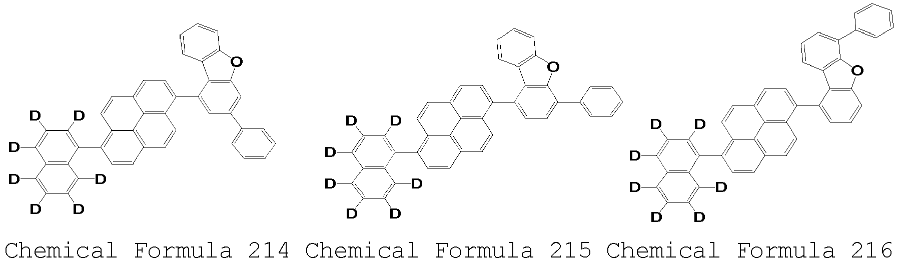

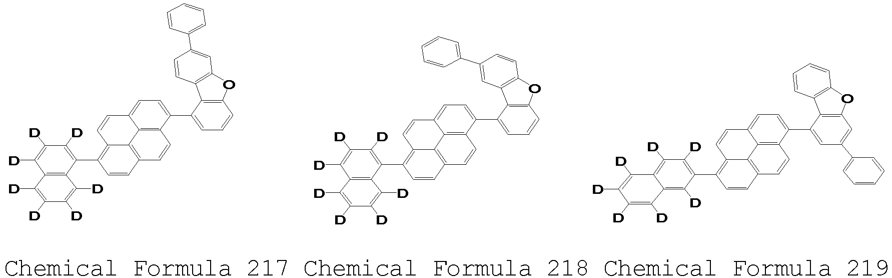

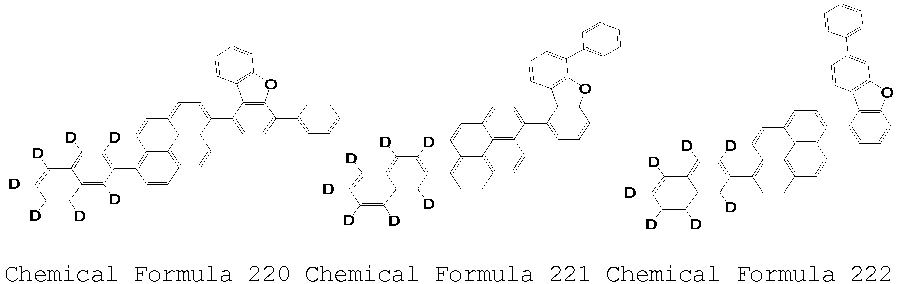

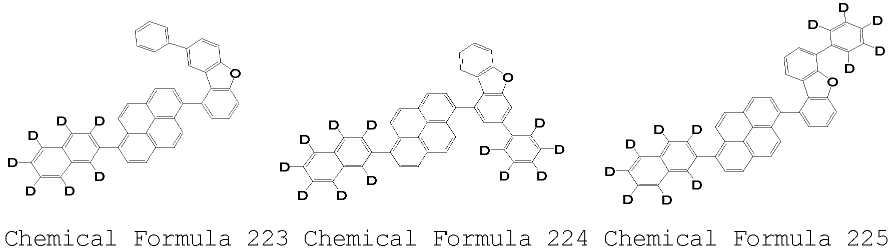

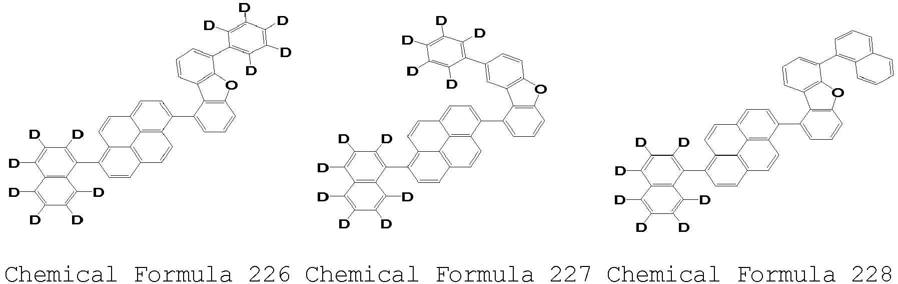

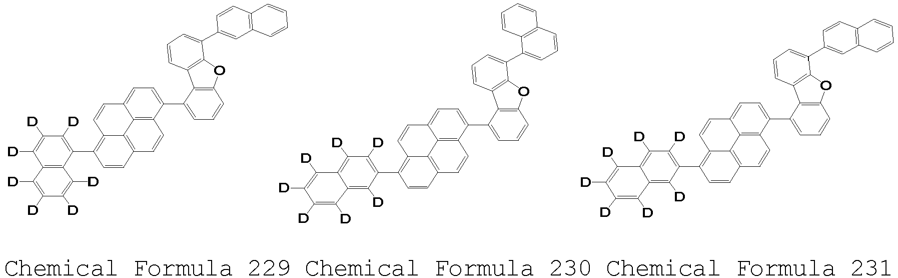

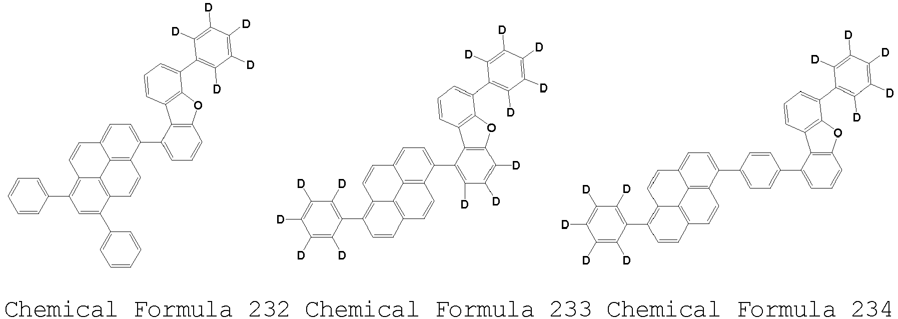

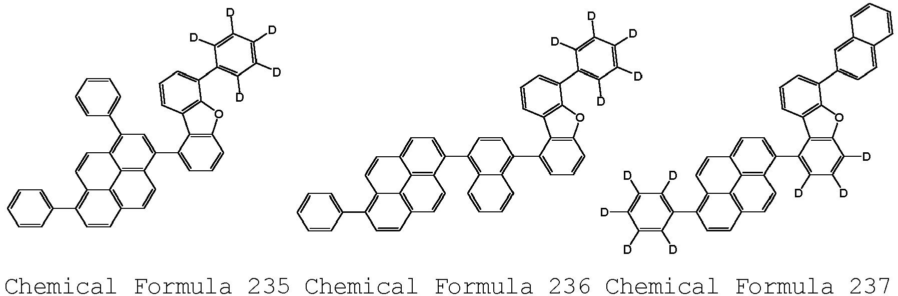

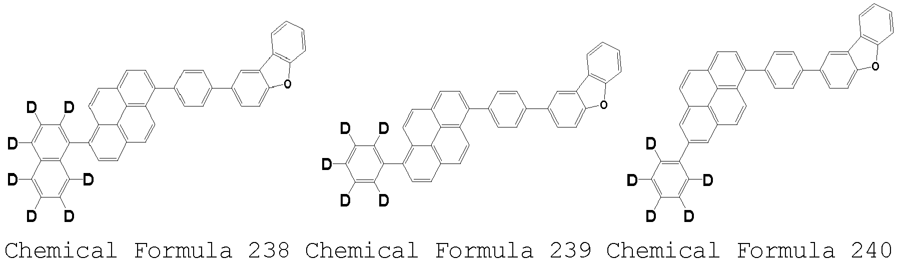

In addition, the present disclosure provides an organic light-emitting diode comprising: a first electrode: a second electrode facing the first electrode; and a light-emitting layer disposed between the first electrode and the second electrode, wherein the light-emitting layer comprises at least one of the compounds represented by Chemical Formula A or Chemical Formula B.

Throughout the description of the present disclosure, the phrase "(an organic layer) includes at least one organic compound" may be construed to mean that "(an organic layer) may include a single organic compound species or two or more different species of organic compounds falling within the scope of the present disclosure".

In this regard, the organic light-emitting diode according to the present disclosure may include at least one of a hole injection layer, a hole transport layer, a functional layer capable of both hole injection and hole transport, a light-emitting layer, an electron transport layer, and an electron injection layer.

In more particular embodiments of the present disclosure, the organic layer disposed between the first electrode and the second electrode includes a light-emitting layer composed of a host and a dopant, wherein the compound represented by Chemical Formula A or Chemical Formula B serves as a host in the light-emitting layer.

In an embodiment, the organic light-emitting diode according to the present disclosure may employ a compound represented by the following Chemical Formulas D1 to Chemical Formula D10 as a dopant compound in the light-emitting layer:

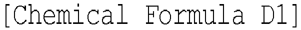

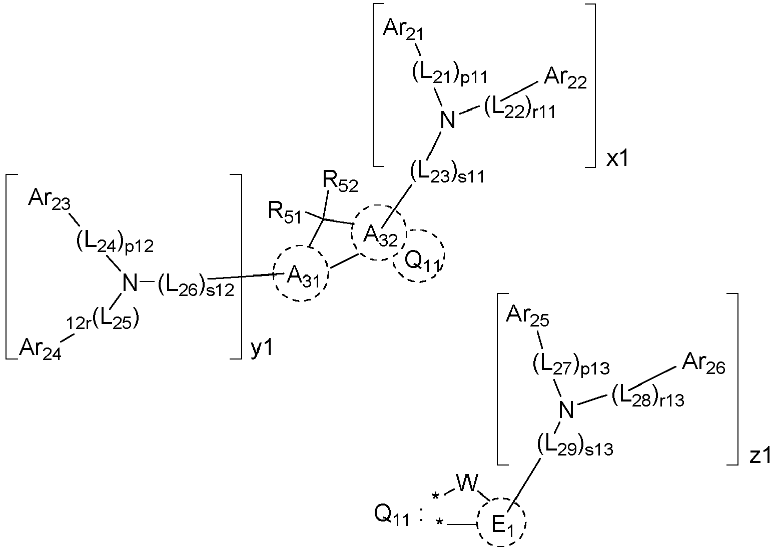

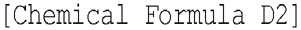

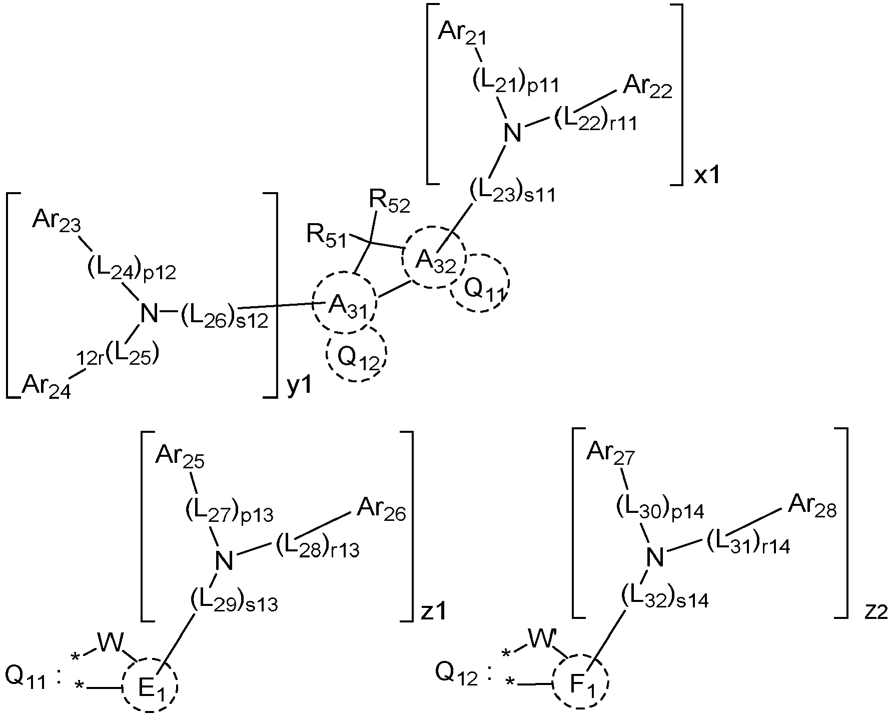

A₃₁, A₃₂, E₁, and F₁, which are same or different, are each independently a substituted or unsubstituted aromatic hydrocarbon ring of 6 to 50 carbon atoms, or a substituted or unsubstituted heteroaromatic ring of 2 to 40 carbon atoms,
wherein two adjacent carbon atoms of the aromatic ring A₃₁ and two adjacent carbon atoms of the aromatic ring A₃₂ form a 5-membered fused ring together with a carbon atom to which substituents R₅₁ and R₅₂ are bonded;
linkers L₂₁ to L₃₂, which are same or different, are each independently selected from among a single bond, a substituted or unsubstituted alkylene of 1 to 60 carbon atoms, a substituted or unsubstituted alkenylene of 2 to 60 carbon atoms, a substituted or unsubstituted alkynylene of 2 to 60 carbon atoms, a substituted or unsubstituted cycloalkylene of 3 to 60 carbon atoms, a substituted or unsubstituted heterocycloalkylene of 2 to 60 carbon atoms, a substituted or unsubstituted arylene of 6 to 60 carbon atoms, and a substituted or unsubstituted heteroarylene of 2 to 60 carbon atoms;
W and W', which are same or different, are each independently any one selected from among N-R₅₃, CR₅₄R₅₅, SiR₅₆R₅₇, GeR₅₈R₅₉, O, S, and Se;
R₅₁ to R₅₉, and Ar₂₁ to Ar₂₈, which are same or different, are each independently any one selected from among a hydrogen atom, a deuterium atom, a substituted or unsubstituted alkyl of 1 to 30 carbon atoms, a substituted or unsubstituted aryl of 6 to 50 carbon atoms, a substituted or unsubstituted alkenyl of 2 to 30 carbon atoms, a substituted or unsubstituted alkynyl of 2 to 20 carbon atoms, a substituted or unsubstituted cycloalkyl of 3 to 30 carbon atoms, a substituted or unsubstituted cycloalkenyl of 5 to 30 carbon atoms, a substituted or unsubstituted heteroaryl of 2 to 50 carbon atoms, a substituted or unsubstituted heterocycloalkyl of 2 to 30 carbon atoms, a substituted or unsubstituted alkoxy of 1 to 30 carbon atoms, a substituted or unsubstituted aryloxy of 6 to 30 carbon atoms, a substituted or unsubstituted alkylthioxy of 1 to 30 carbon atoms, a substituted or unsubstituted arylthioxy of 5 to 30 carbon atoms, a substituted or unsubstituted alkylamine of 1 to 30 carbon atoms, a substituted or unsubstituted arylamine of 5 to 30 carbon atoms, a substituted or unsubstituted alkylsilyl of 1 to 30 carbon atoms, a substituted or unsubstituted arylsilyl of 5 to 30 carbon atoms, a substituted or unsubstituted alkylgermyl of 1 to 30 carbon atoms, a substituted or unsubstituted arylgermyl of 1 to 30 carbon atoms, a cyano, a nitro, and a halogen, wherein R₅₁ and R₅₂ together may form a mono- or polycyclic aliphatic or aromatic ring that may be a heterocyclic ring bearing a heteroatom selected from among N, O, P, Si, S, Ge, Se, and Te as a ring member;
p11 to p14, r11 to r14, and s11 to s14 are each independently an integer of 1 to 3, wherein when any of them is 2 or greater, the corresponding linkers L₂₁ to L₃₂ may be same or different,
x1 is 1, and y1, z1, and z2, which are same or different, are each independently an integer of 0 to 1; and
Ar₂₁ may form a ring with Ar₂₂, Ar₂₃ may form a ring with Ar₂₄, Ar₂₅ may form a ring with Ar₂₆, and Ar₂₇ may form a ring with Ar₂₈,
two adjacent carbon atoms of the A₃₂ ring moiety of Chemical Formula D1 may occupy respective positions * of Structural Formula Q₁₁ to form a fused ring, and
two adjacent carbon atoms of the A₃₁ ring moiety of Chemical Formula D2 may occupy respective positions * of structural Formula Q₁₂ to form a fused ring, and two adjacent carbon atoms of the A₃₂ ring moiety of Chemical Formula D2 may occupy respective positions * of Structural Formula Q₁₁ to form a fused ring, 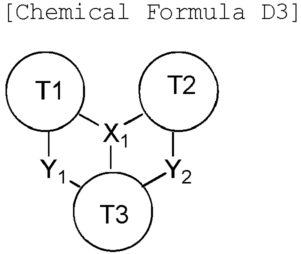
wherein,
X₁ is any one selected from among B, P, and P=O
T1 to T3, which are same or different, are each independently a substituted or unsubstituted aromatic hydrocarbon ring of 6 to 50 carbon atoms, or a substituted or unsubstituted heteroaromatic ring of 2 to 40 carbon atoms;
Y₁ is any one selected from among N-R₆₁, CR₆₂R₆₃, O, S, and SiR₆₄R₆₅;
Y₂ is any one selected from among N-R₆₆, CR₆₇R₆₈, O, S, and SiR₆₉R₇₀;
R₆₁ to R₇₀, which are same or different, are each independently any one selected from among a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted alkoxy of 1 to 30 carbon atoms, a substituted or unsubstituted aryloxy of 6 to 30 carbon atoms, a substituted or unsubstituted alkylthioxy of 1 to 30 carbon atoms, a substituted or unsubstituted arylthioxy of 5 to 30 carbon atoms, a substituted or unsubstituted alkylamine of 1 to 30 carbon atoms, a substituted or unsubstituted arylamine of 5 to 30 carbon atoms, a substituted or unsubstituted alkylsilyl of 1 to 30 carbon atoms, a substituted or unsubstituted arylsilyl of 5 to 30 carbon atoms, a cyano, and a halogen, and wherein at least one of R₆₁ to R₇₀ may be connected to at least one of T₁ to T₃ to form an additional mono- or polycyclic aliphatic or aromatic ring; 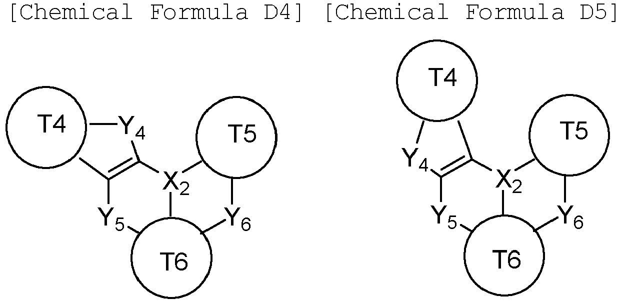
wherein,
X₂ is any one selected from among B, P, and P=O,
T4 to T6 are as defined for T1 to T3 in Chemical Formula D3,
Y₄ is any one selected from among N-R₆₁, CR₆₂R₆₃, O, S, and SiR₆₄R₆₅;
Y₅ is any one selected from among N-R₆₆, CR₆₇R₆₈, O, S, and SiR₆₉R₇₀;
Y₆ is any one selected from among N-R₇₁, CR₇₂R₇₃, O, S, and SiR₇₄R₇₅; and
R₆₁ to R₇₅ being as defined for R₆₁ to R₇₀ in Chemical Formula D3; 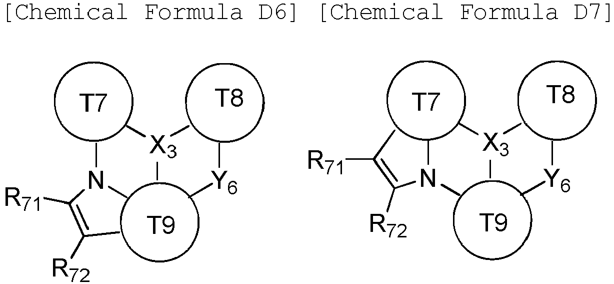
X₃ is any one selected from among B, P, and P=O,
T7 to T9 are defined as for T1 to T3 in Chemical Formula D3,
Y₆ is any one selected from among N-R₆₁, CR₆₂R₆₃, O, S, and SiR₆₄R₆₅;
R₆₁ to R₆₅ and R₇₁ to R₇₂ are each as defined for R₆₁ to R₇₀ in Chemical Formula D3,
   wherein R₇₁ and R₇₂ may be connected to each other to form an additional mono- or polycyclic aliphatic or aromatic ring, or may be connected to the T₇ ring moiety or T₉ ring moiety to form an additional mono- or polycyclic aliphatic or aromatic ring; 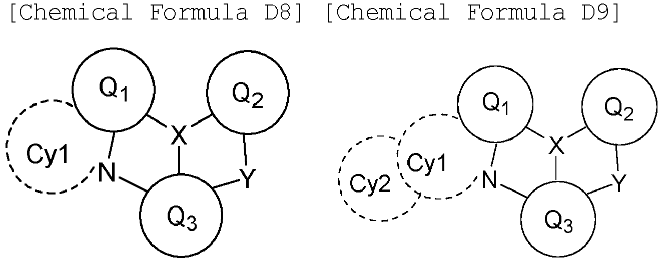 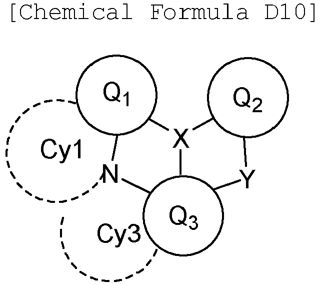
wherein,
X is any one selected from among B, P, and P=O,
Q₁ to Q₃ are each as defined for T1 to T3 in Chemical Formula D3,
Y is any one selected from among N-R₃, CR₄R₅, O, S, and Se,
R₃ to R₅ are each as defined for R₆₁ to R₇₀ in Chemical Formula D3,
R₃ to R₅ may each be connected to the Q₂ or Q₃ ring moiety to form an additional mono- or polycyclic aliphatic or aromatic ring,
R₄ and R₅ may be connected to each other to form an additional mono- or polycyclic aliphatic or aromatic ring,
the ring formed by Cy1 is a substituted or unsubstituted alkylene of 1 to 10 carbon atoms, except for the nitrogen (N) atom, the aromatic carbon atom of Q₁ to which the nitrogen (N) atom is connected, and the aromatic carbon atom of Q₁ to which Cy1 is to bond,
"Cy2" in Chemical Formula D9 forms a saturated hydrocarbon ring added to Cy1 wherein the ring formed by Cy2 is a substituted or unsubstituted alkylene of 1 to 10 carbon atoms, except for the carbon atoms included in Cy1, and
the ring formed by Cy3 in Chemical Formula D10 is a substituted or unsubstituted alkylene of 1 to 10 carbon atoms, except for the aromatic carbon atom of Q₃ to which Cy3 is to bond, the aromatic carbon atom of Q₃ to which the nitrogen (N) atom is connected, the nitrogen (N) atom, and the carbon atom of Cy1 to which the nitrogen (N) atom is connected,
wherein the term "substituted" in the expression "substituted or unsubstituted" used for compounds of Chemical Formulas D1 to D10 means having at least one substituent selected from the group consisting of a deuterium atom, a cyano, a halogen, a hydroxy, a nitro, an alkyl of 1 to 24 carbon atoms, a hydrogenated alkyl of 1 to 24 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 2 to 24 carbon atoms, an alkoxy of 1 to 24 carbon atoms, an alkylamino of 1 to 24 carbon atoms, a diarylamino of 12 to 24 carbon atoms, a diheteroarylamino of 2 to 24 carbon atoms, an aryl(heteroaryl)amino of 7 to 24 carbon atoms, an alkylsilyl of 1 to 24 carbon atoms, an arylsilyl of 6 to 24 carbon atoms, an aryloxy of 6 to 24 carbon atoms, and an arylthionyl of 6 to 24 carbon atoms, and more particularly, having at least one substituent selected from the group consisting of a deuterium atom, a cyano, a halogen, a hydroxy, a nitro, an alkyl of 1 to 12 carbon atoms, a halogenated alkyl of 1 to 12 carbon atoms, an alkenyl of 2 to 12 carbon atoms, an alkynyl of 2 to 12 carbon atoms, a cycloalkyl of 3 to 12 carbon atoms, a heteroalkyl of 1 to 12 carbon atoms, an aryl of 6 to 18 carbon atoms, an arylalkyl of 7 to 20 carbon atoms, an alkylaryl of 7 to 20 carbon atoms, a heteroaryl of 2 to 18 carbon atoms, a heteroarylalkyl of 2 to 18 carbon atoms, an alkoxy of 1 to 12 carbon atoms, an alkylamino of 1 to 12 carbon atoms, a diarylamino of 12 to 18 carbon atoms, a diheteroarylamino of 2 to 18 carbon atoms, an aryl(heteroaryl)amino of 7 to 18 carbon atoms, an alkylsilyl of 1 to 12 carbon atoms, an arylsilyl of 6 to 18 carbon atoms, an aryloxy of 6 to 18 carbon atoms, and an arylthionyl of 6 to 18 carbon atoms.

Among the dopant compounds according to the present disclosure, the boron compounds represented by Chemical Formulas D3 to D10 may have, on the aromatic hydrocarbon rings or heteroaromatic rings of T1 to T9 or on the aromatic hydrocarbon rings or heteroaromatic rings of Q₁ to Q₃, a substituent selected from a deuterium atom, an alkyl of 1 to 24 carbon atoms, an aryl of 6 to 24 carbon atoms, an alkylamino of 1 to 24 carbon atoms, and an arylamino of 6 to 24 carbon atoms, wherein the alkyl radicals or the aryl radicals in the alkylamino of 1 to 24 carbon atoms and the arylamino of 6 to 24 carbon atoms on the rings may be linked to each other, and particularly a substituent selected from an alkyl of 1 to 12 carbon atoms, an aryl of 6 to 18 carbon atoms, an alkylamino of 1 to 12 carbon atoms, and an arylamino of 6 to 18 carbon atoms wherein the alkyl radicals or aryl radicals in the alkylamino of 1 to 12 carbon atoms and the arylamino of 6 to 18 carbon atoms on the rings may be linked to each other.

Concrete examples of the dopant compounds of Chemical Formulas D1 and D2 used in the light-emitting layer include the compounds of the following Chemical Formulas <d 1> to <d 239>:

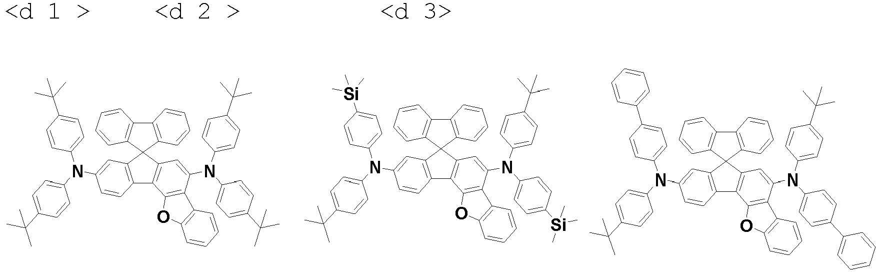

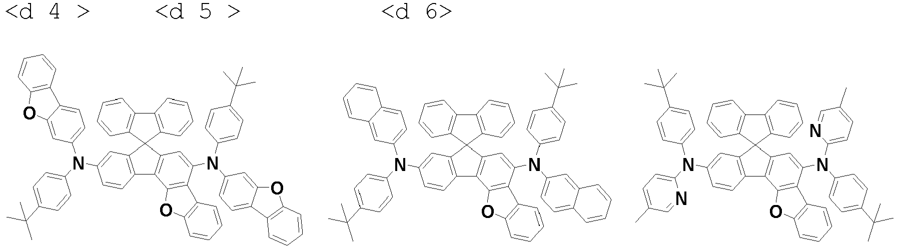

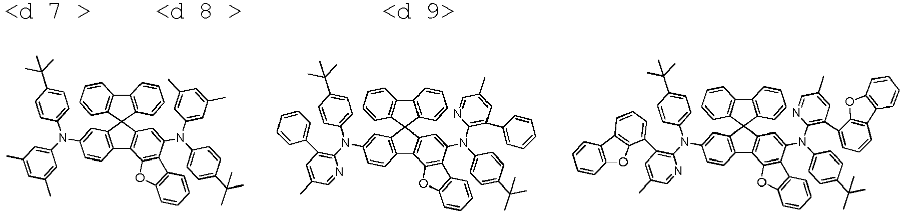

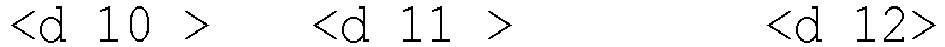

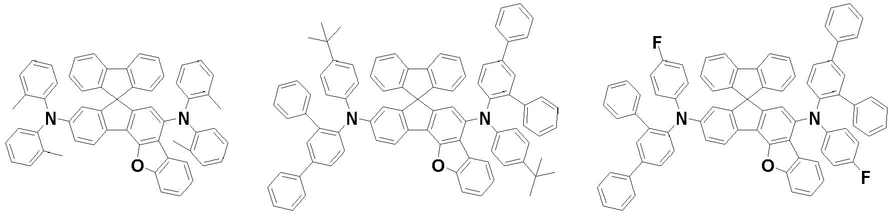

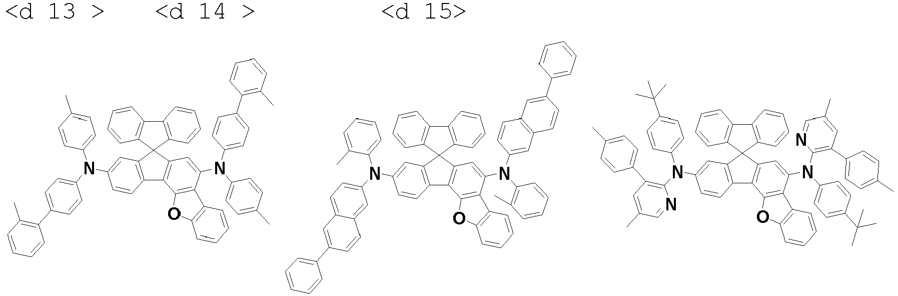

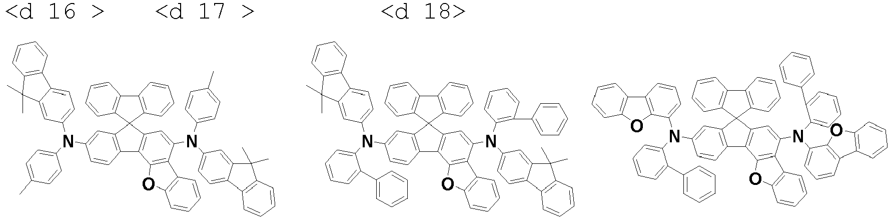

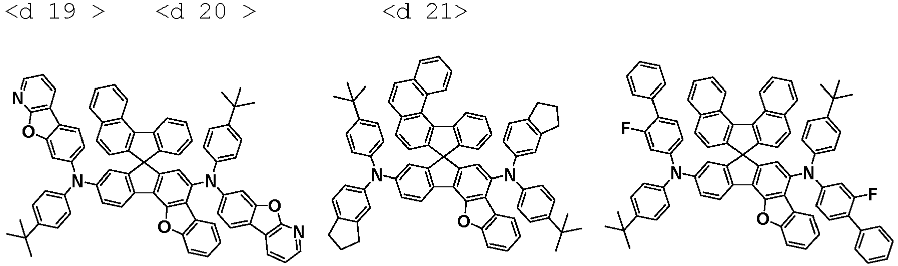

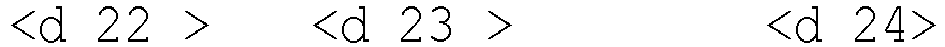

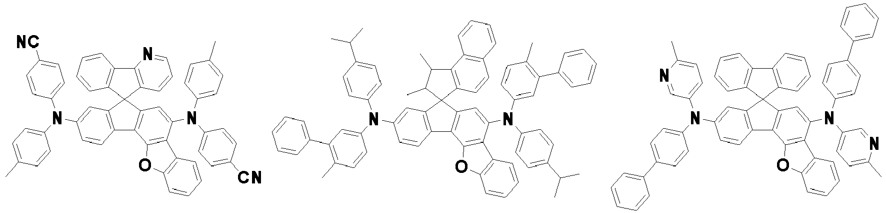

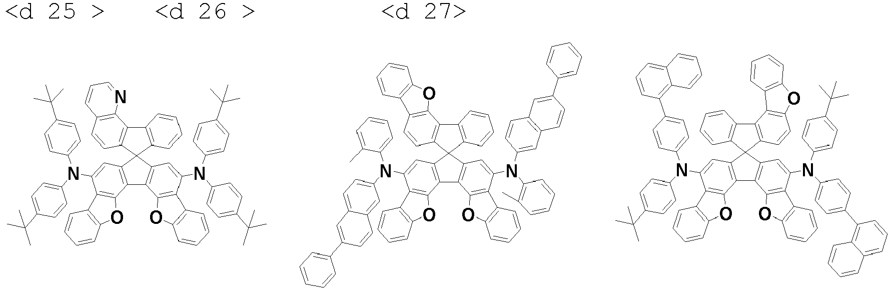

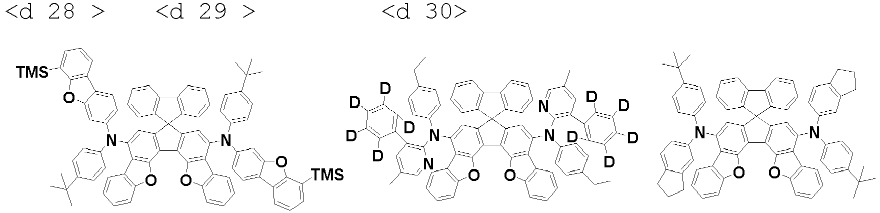

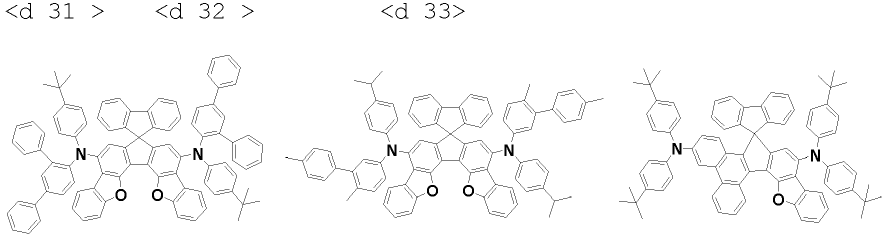

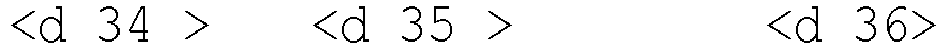

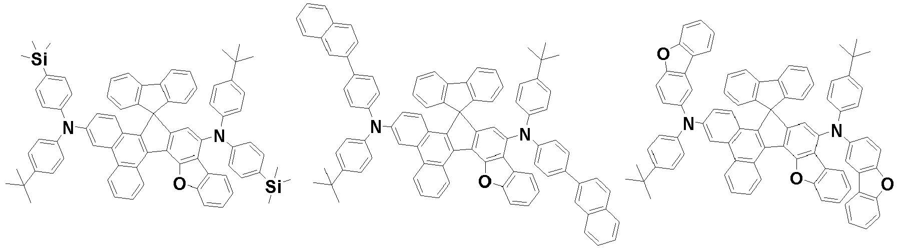

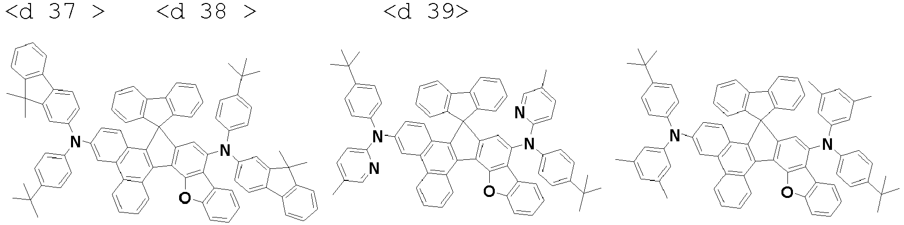

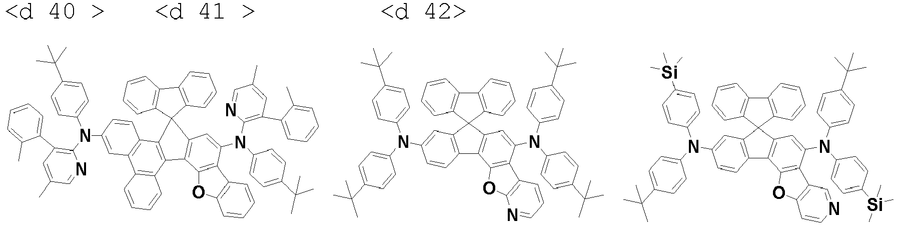

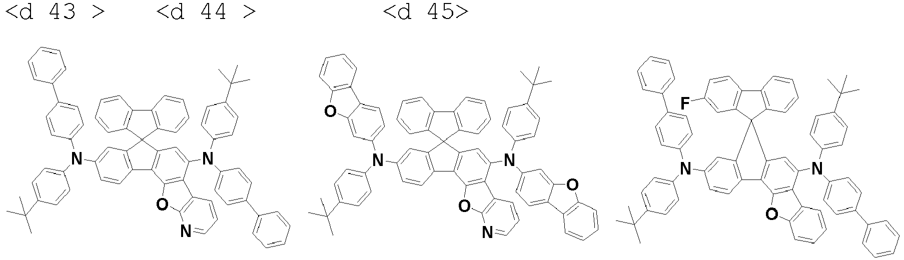

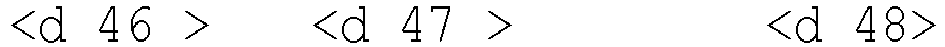

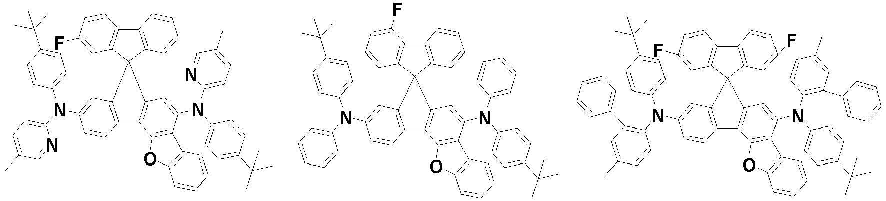

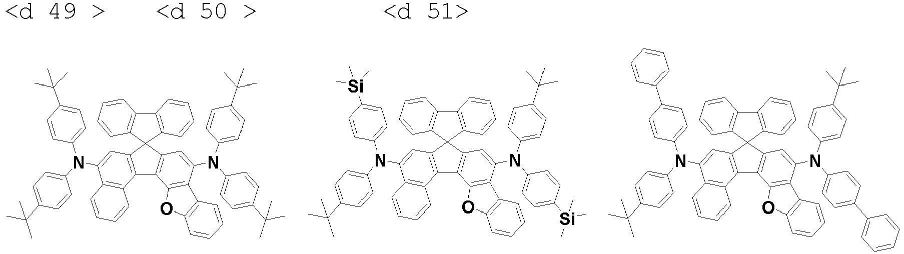

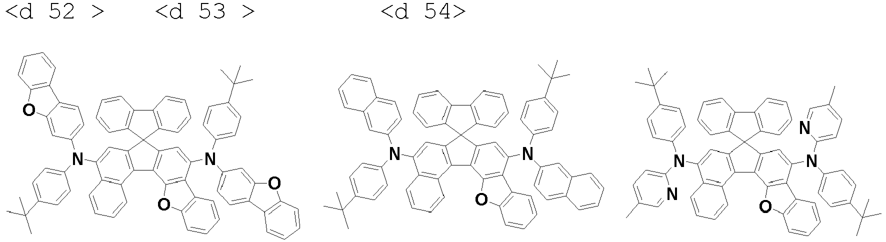

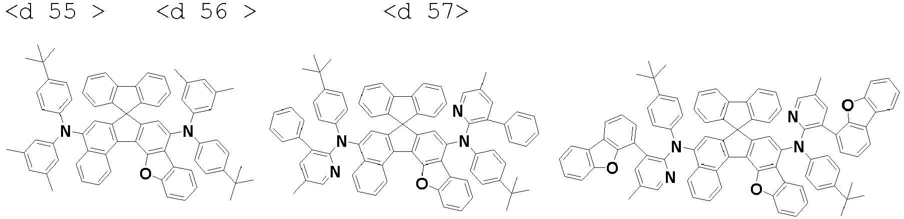

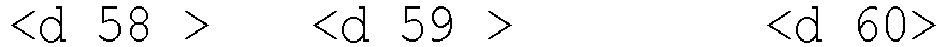

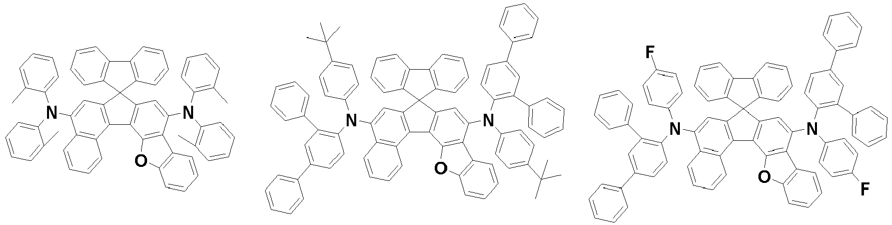

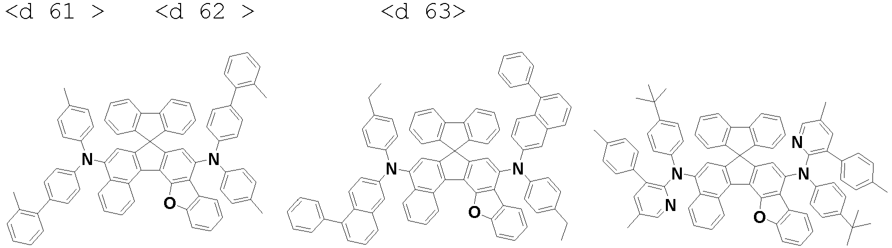

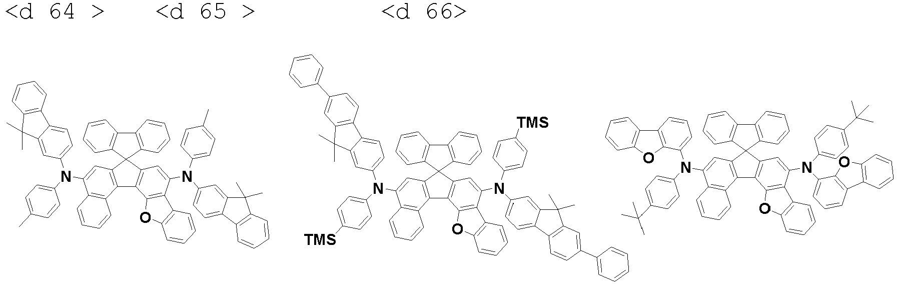

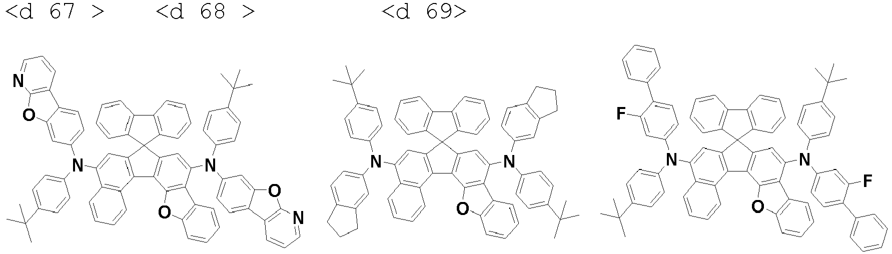

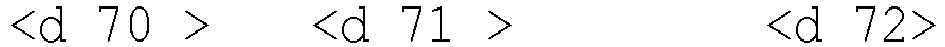

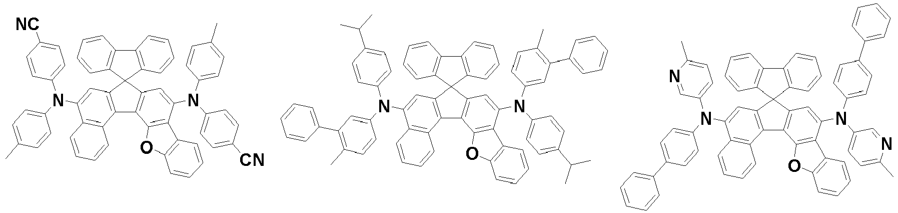

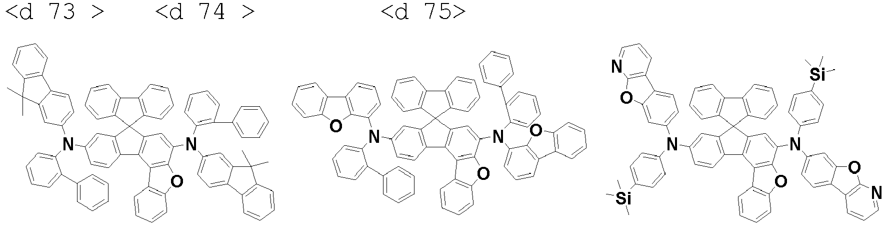

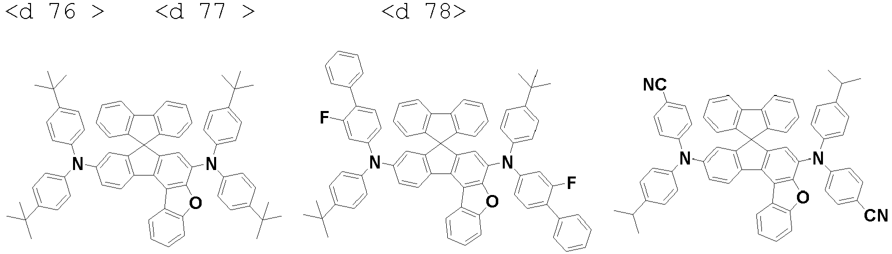

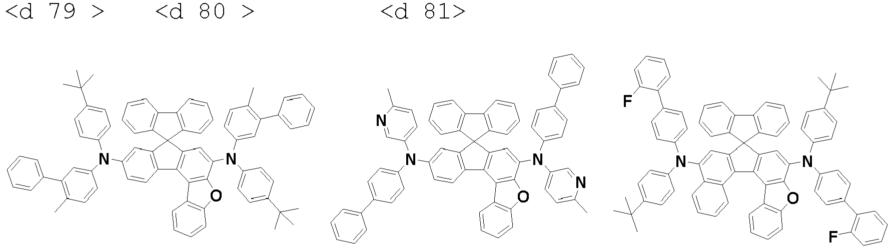

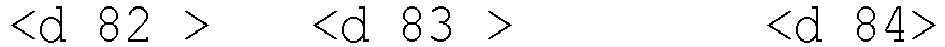

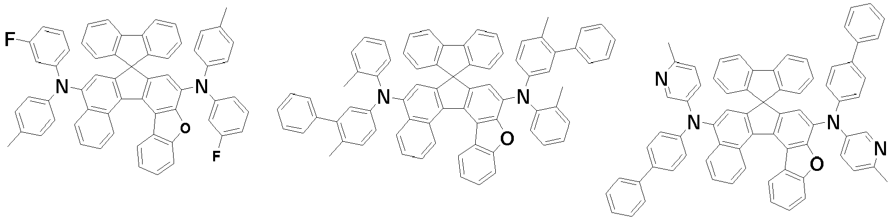

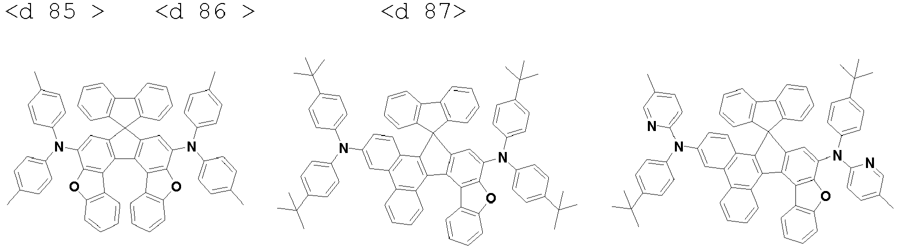

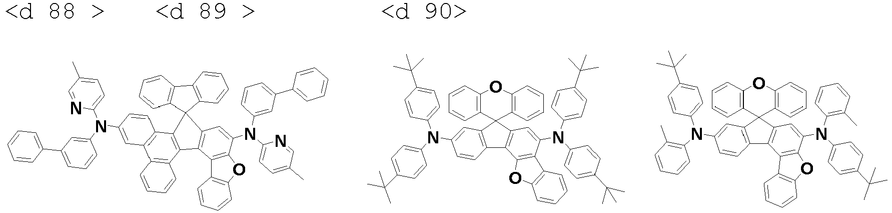

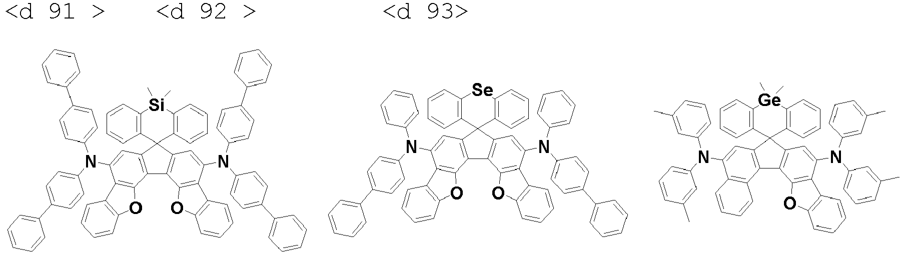

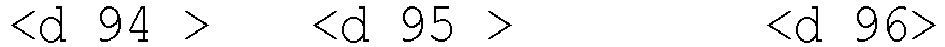

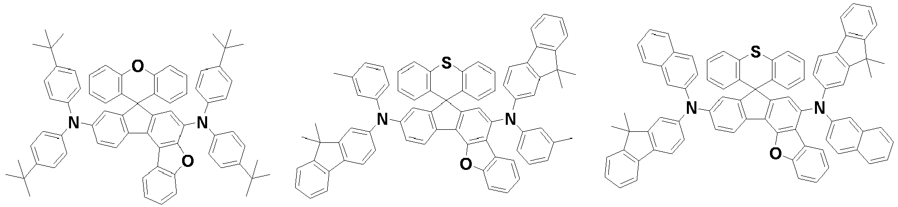

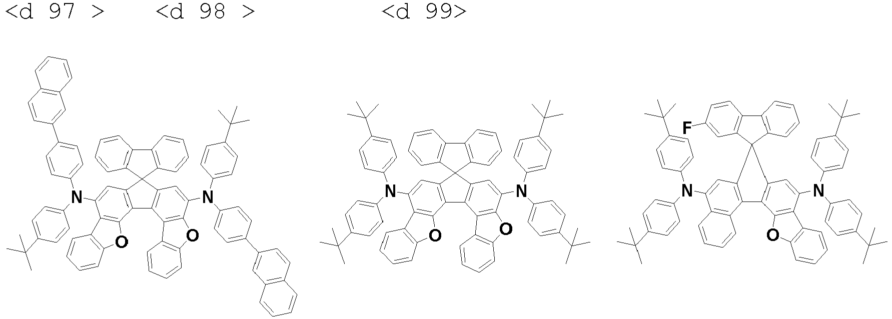

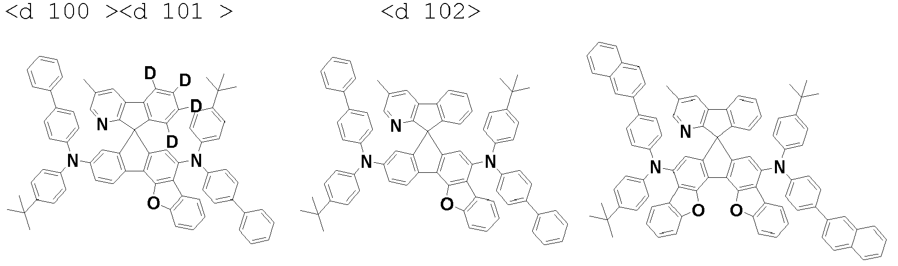

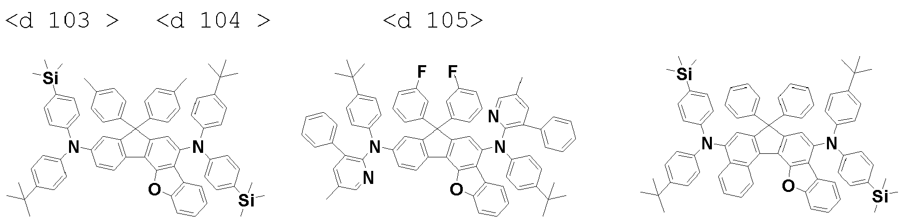

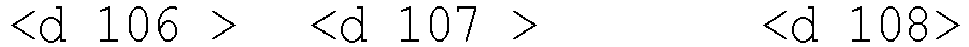

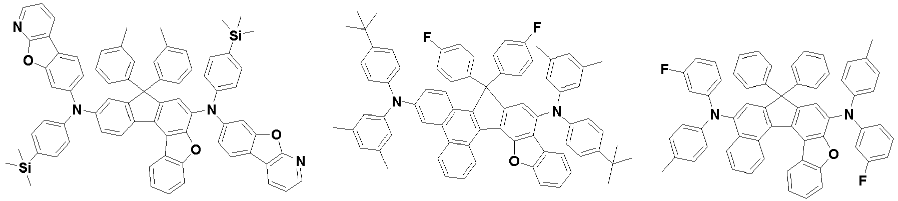

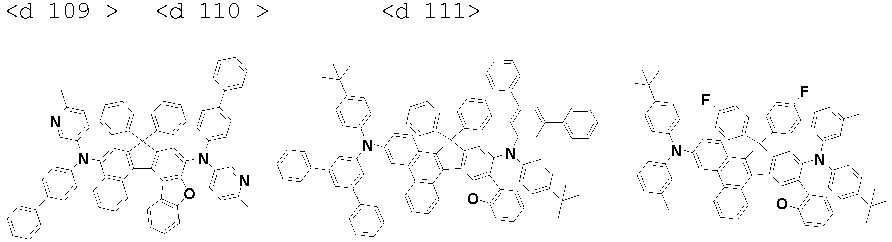

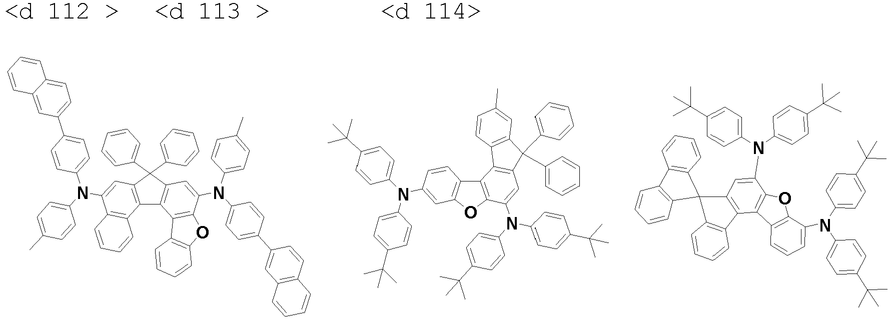

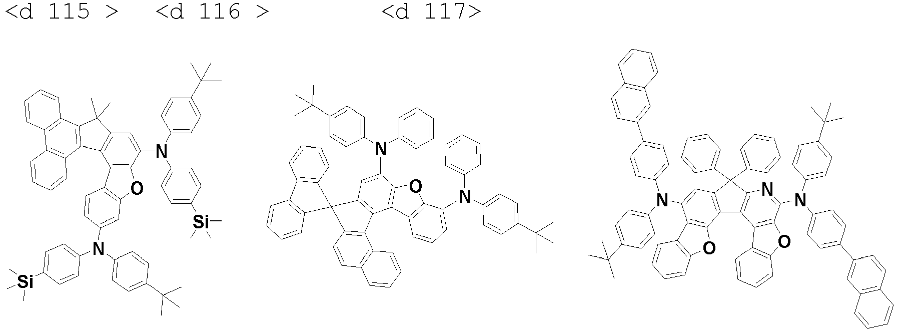

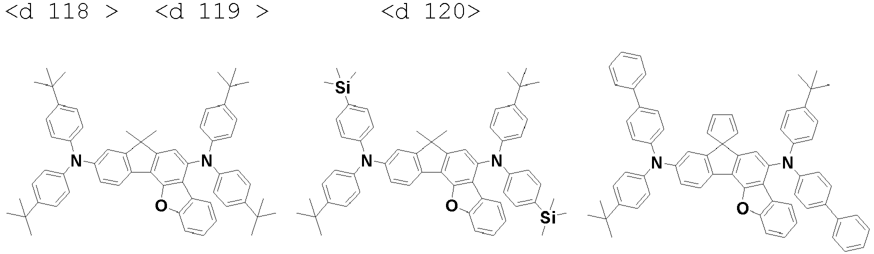

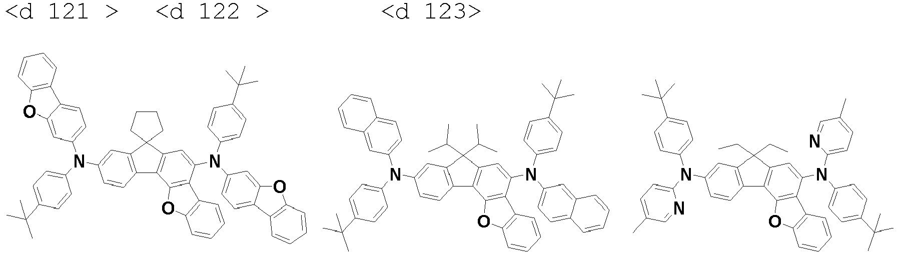

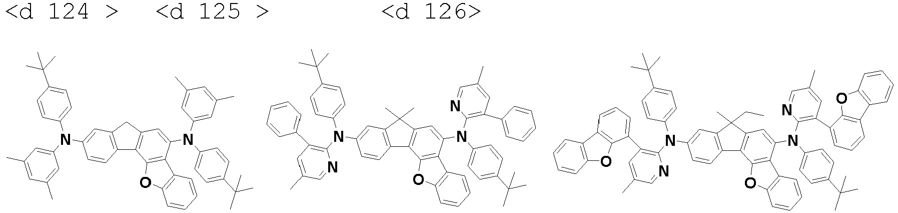

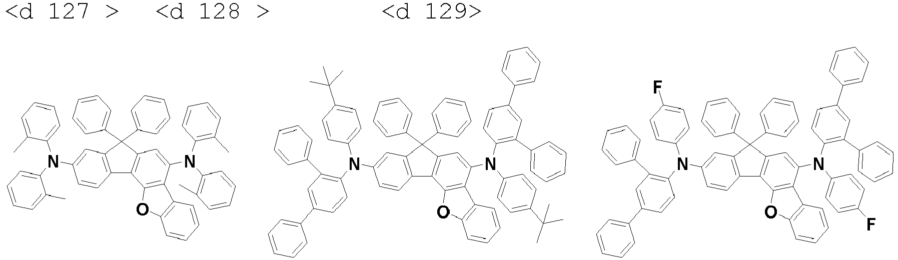

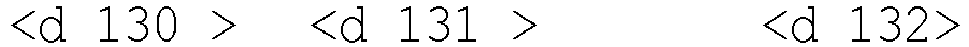

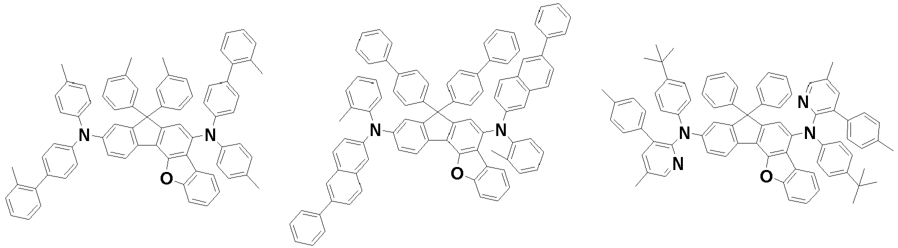

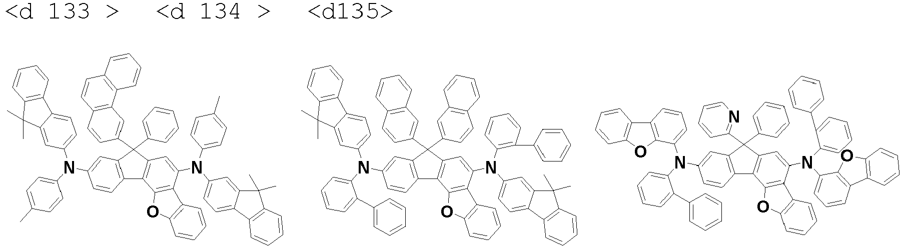

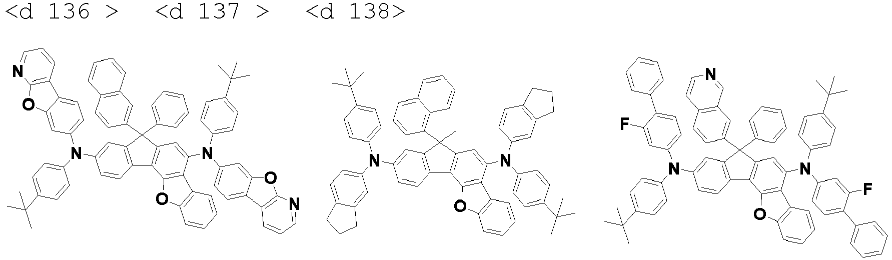

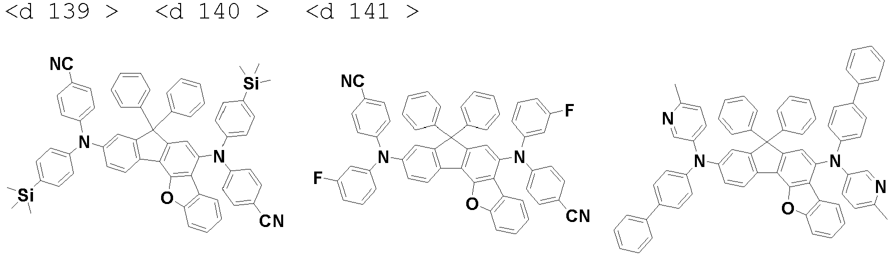

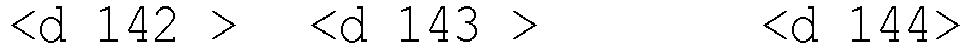

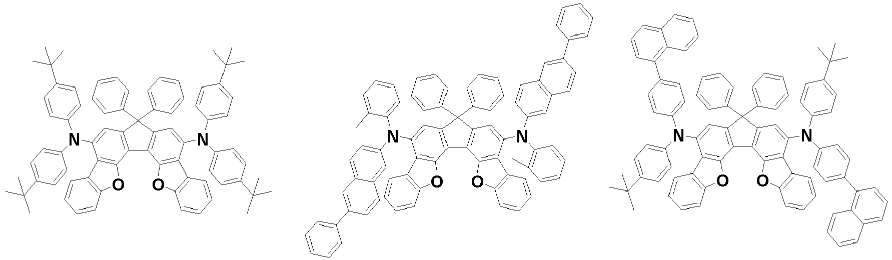

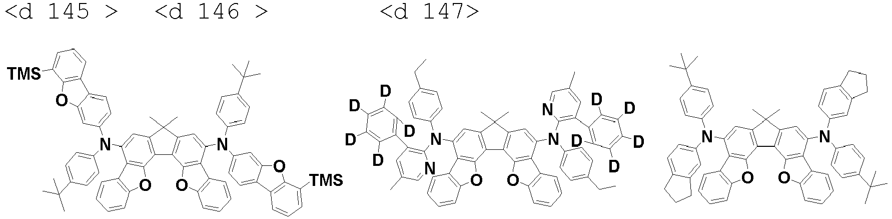

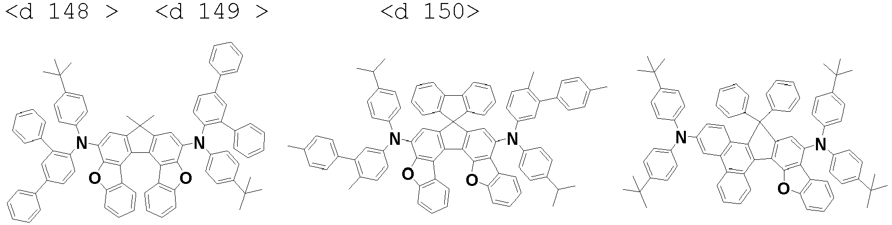

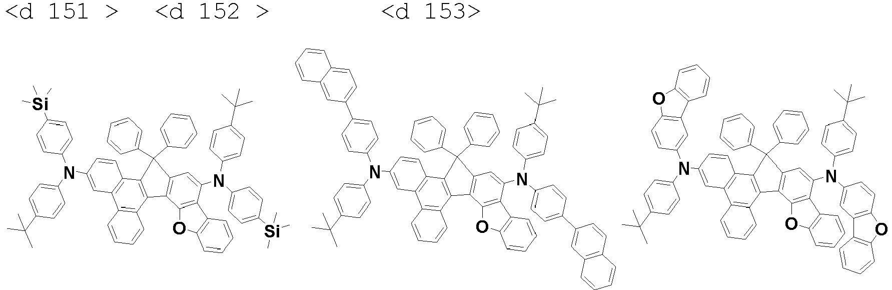

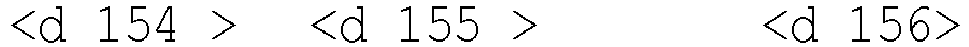

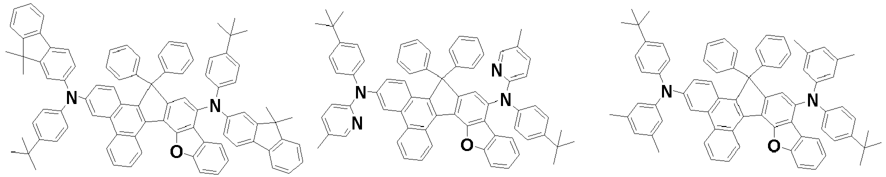

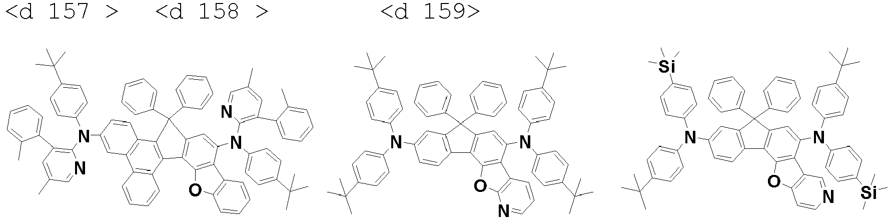

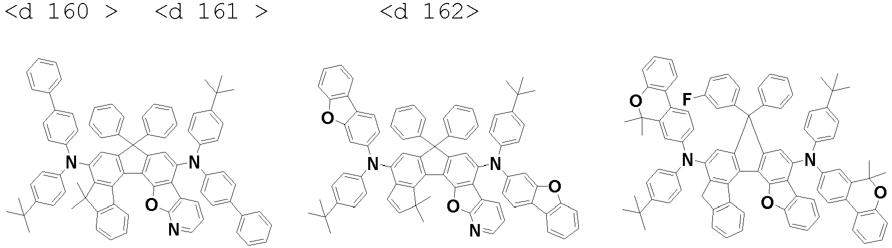

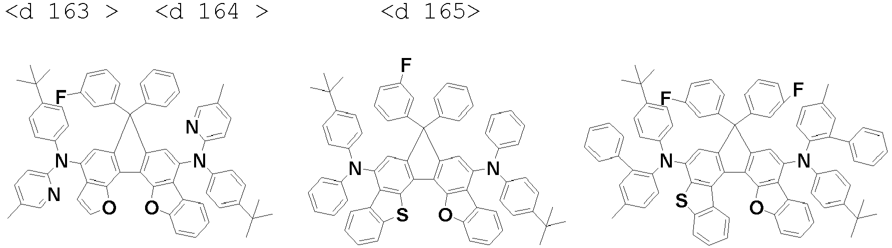

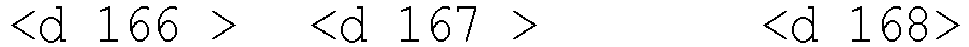

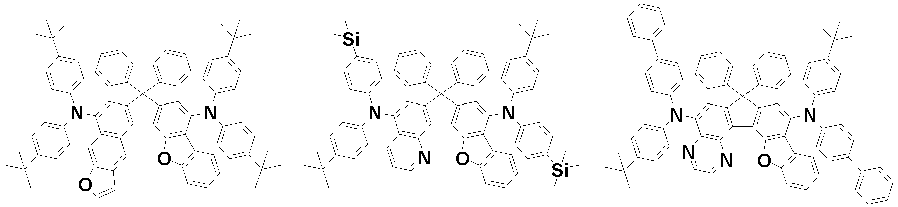

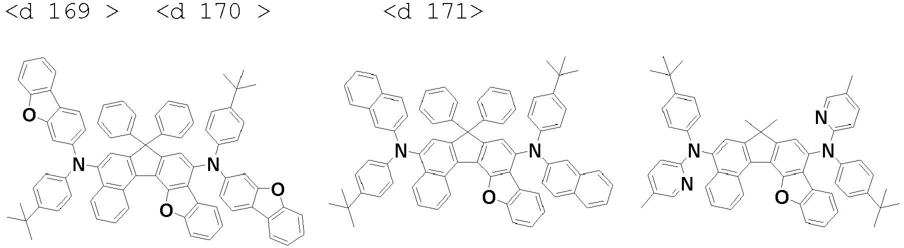

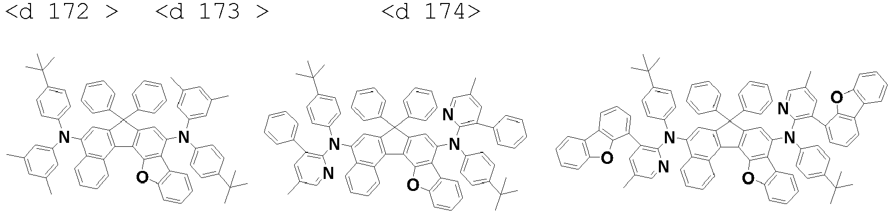

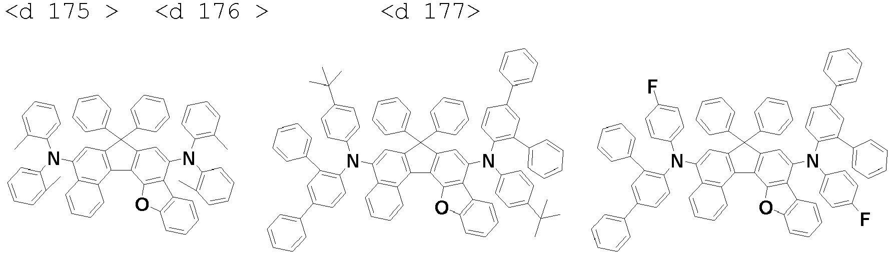

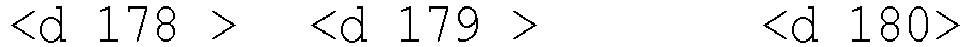

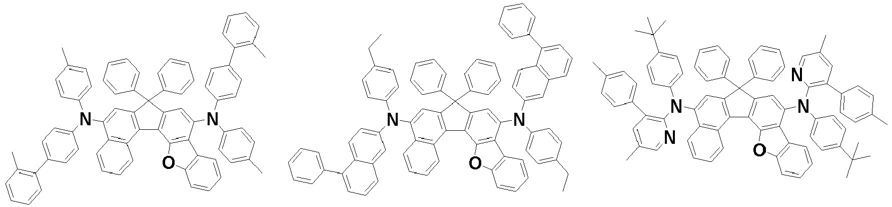

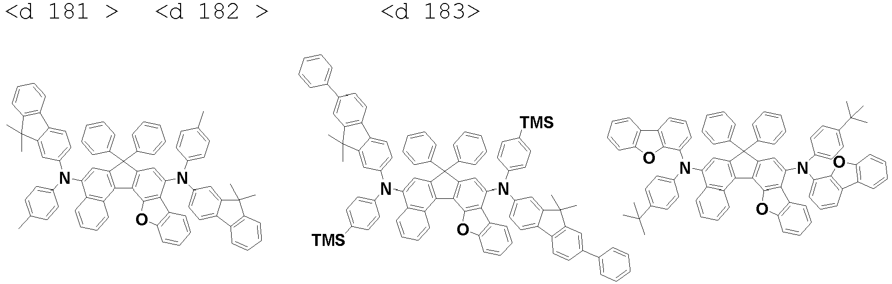

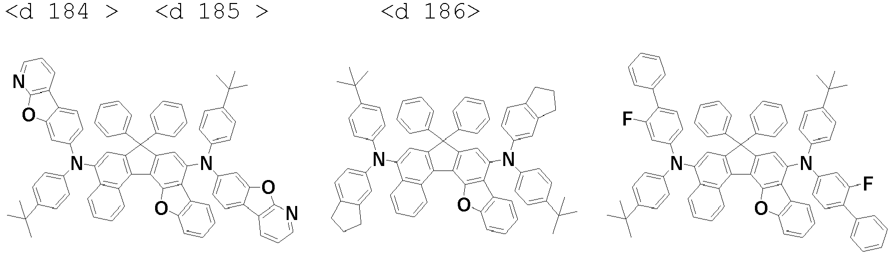

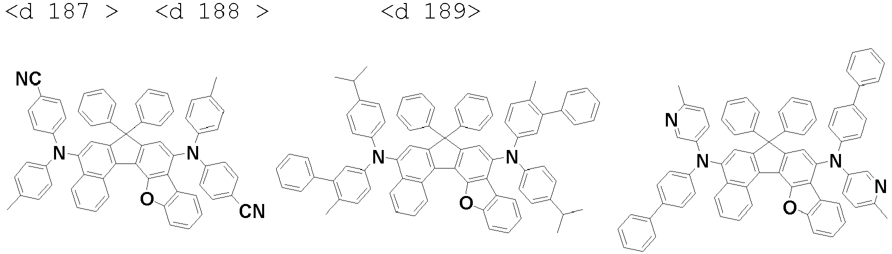

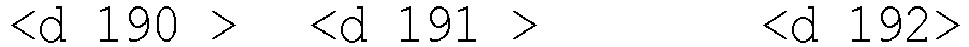

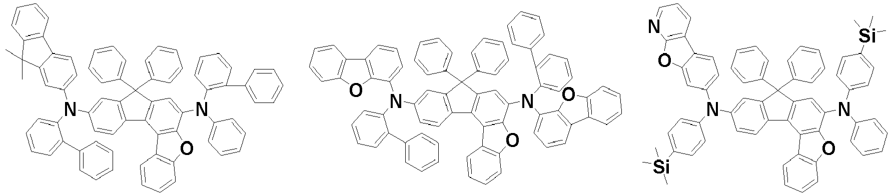

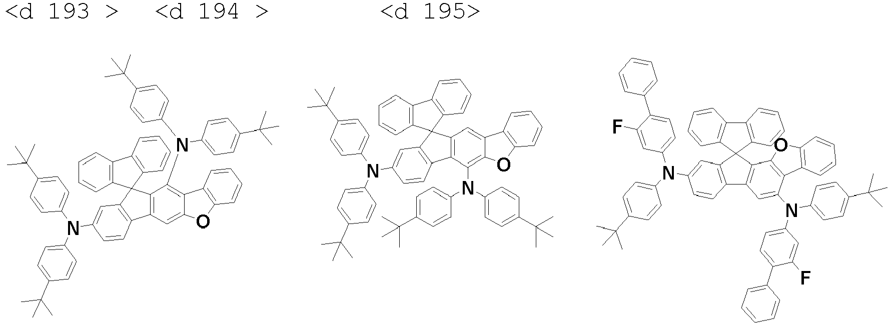

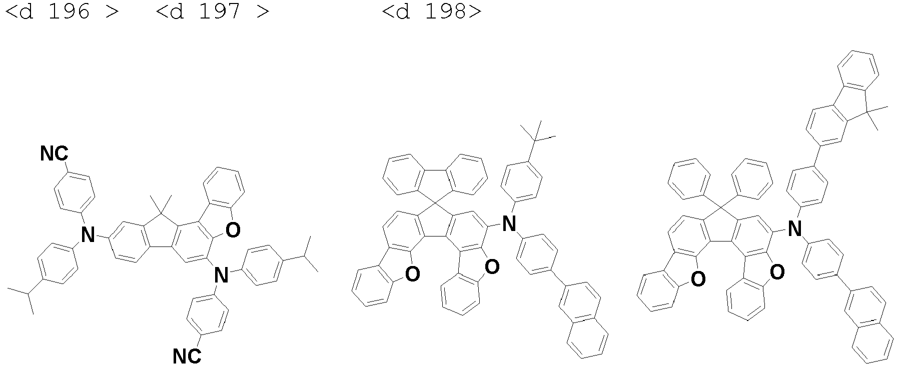

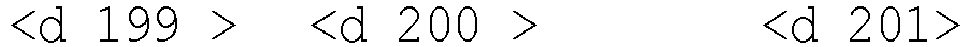

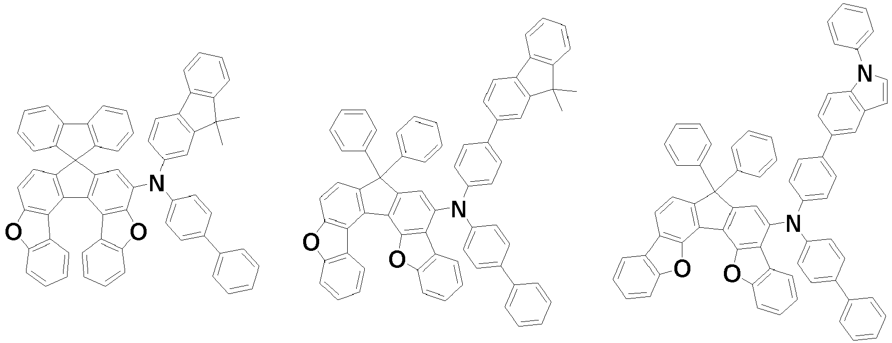

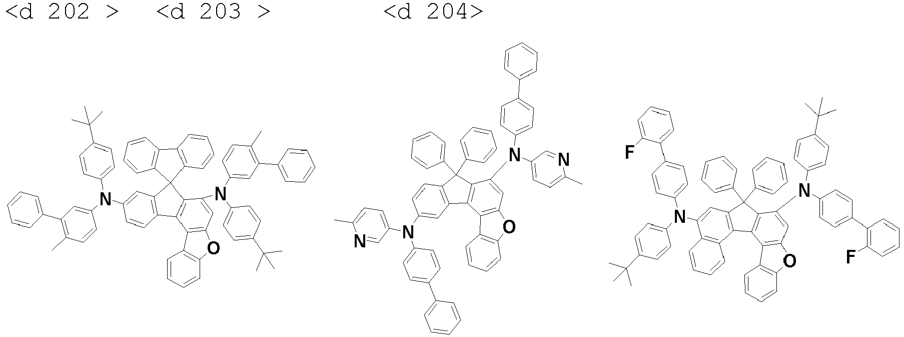

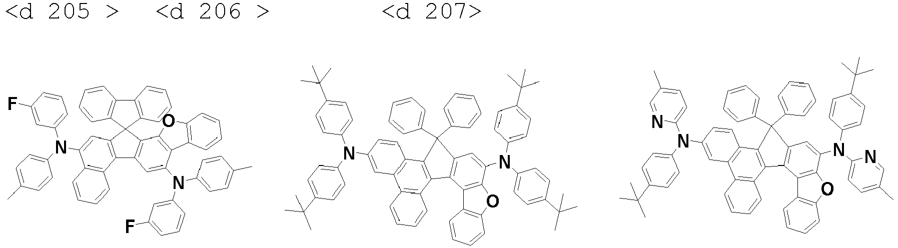

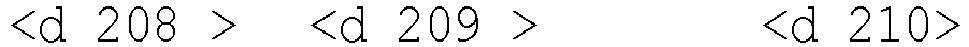

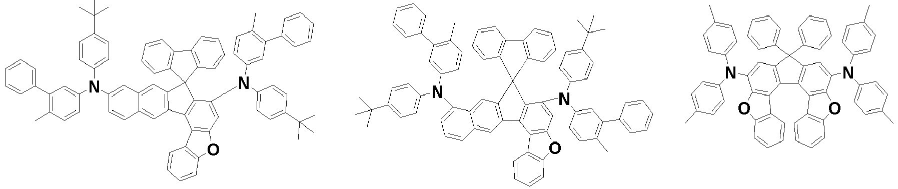

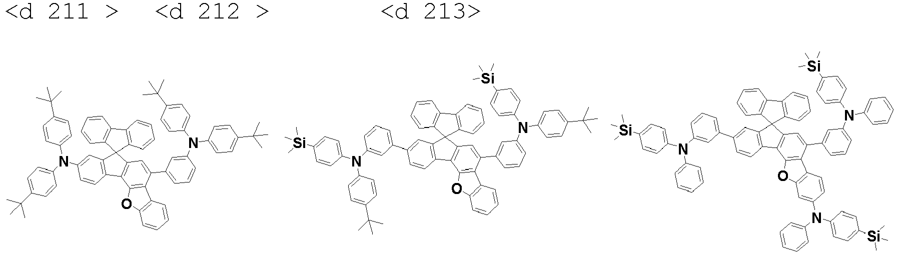

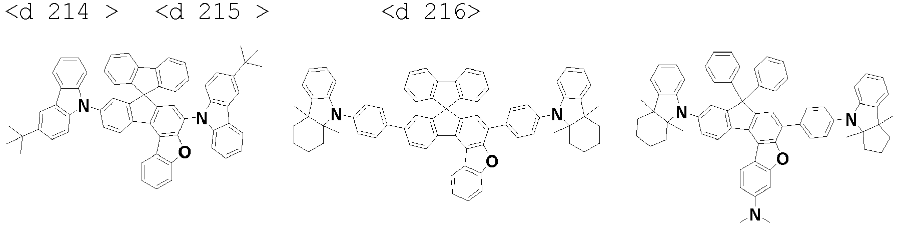

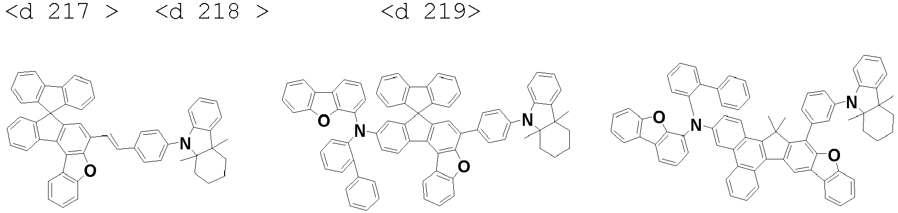

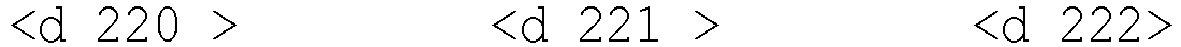

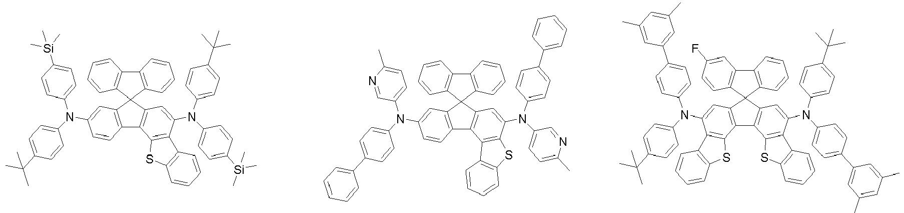

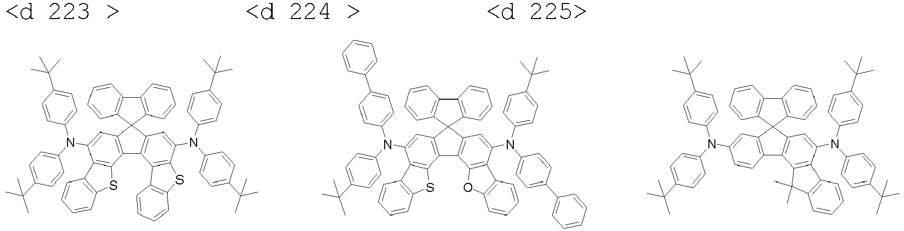

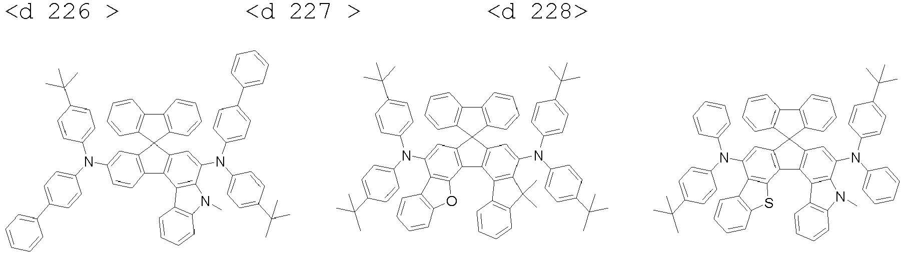

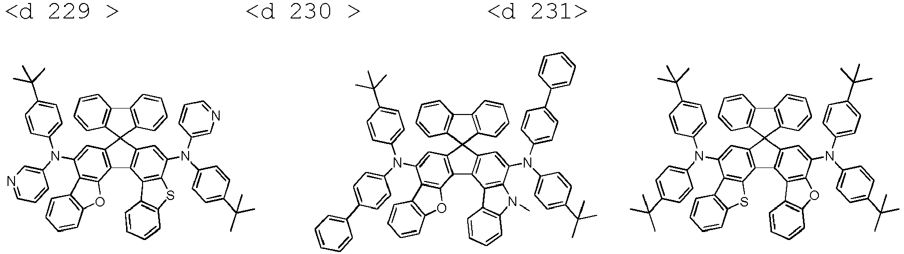

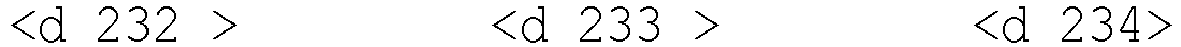

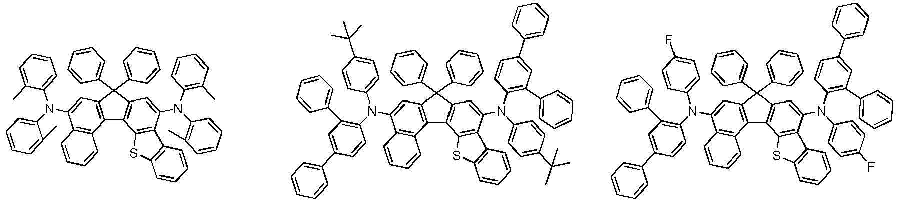

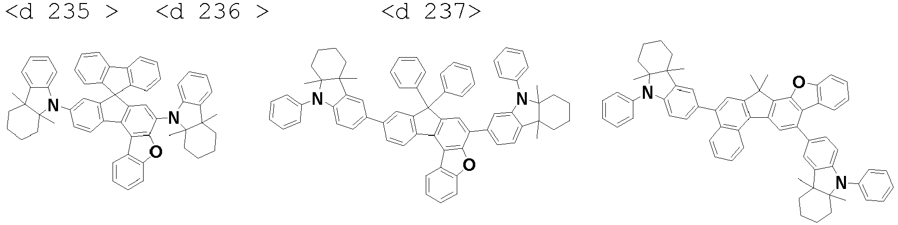

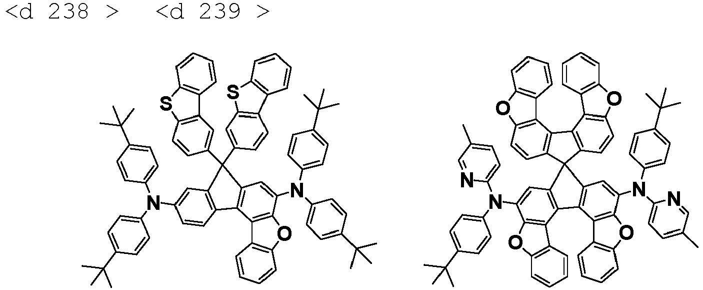

Among the dopant compounds used in the light-emitting layer, the compound represented by Chemical Formula D3 may be any one of the following <D 101> to <D 130>:

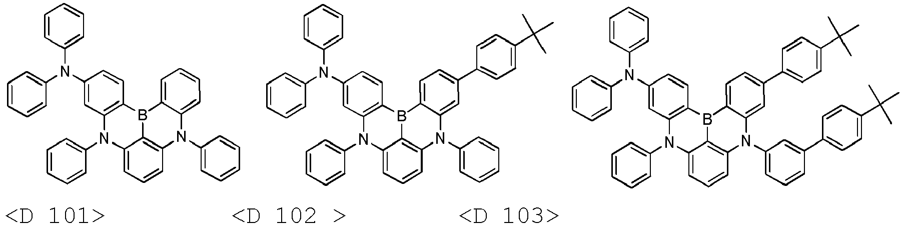

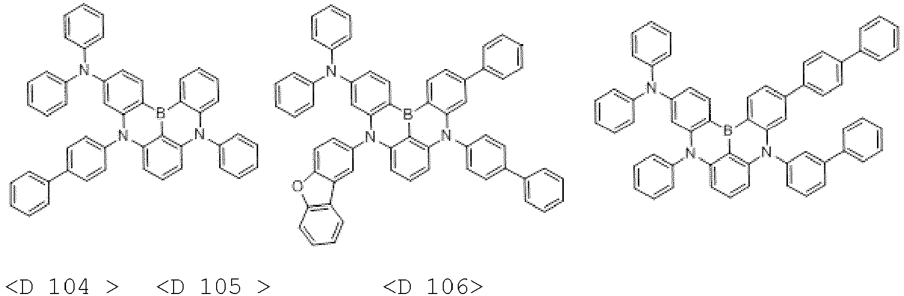

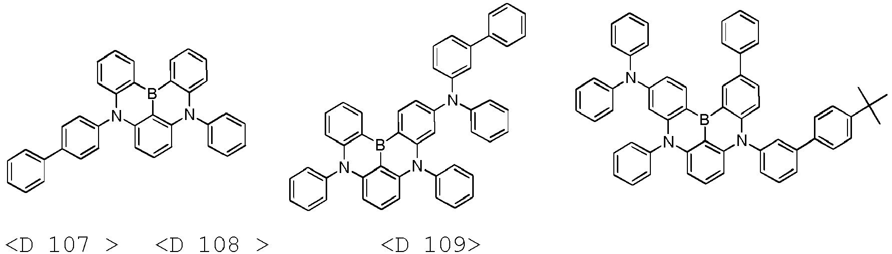

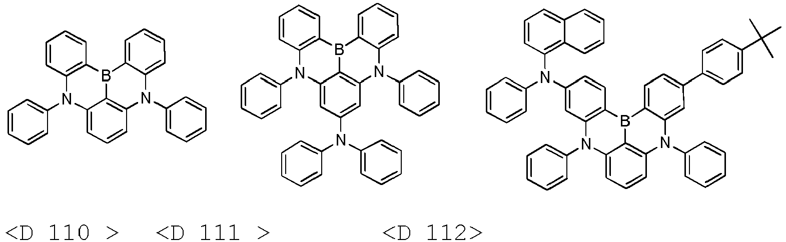

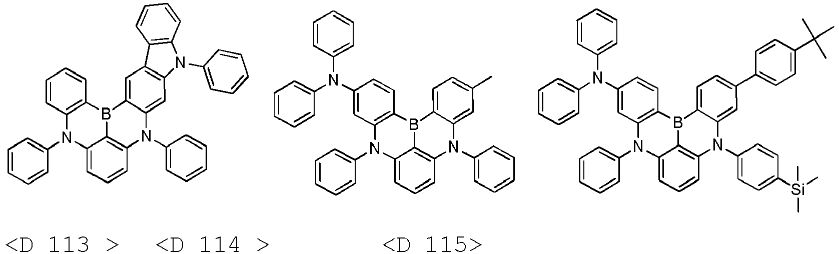

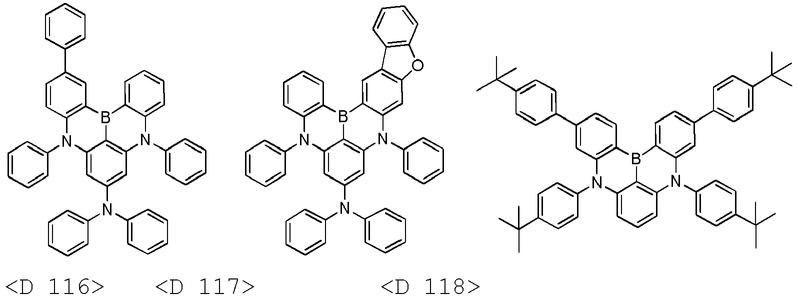

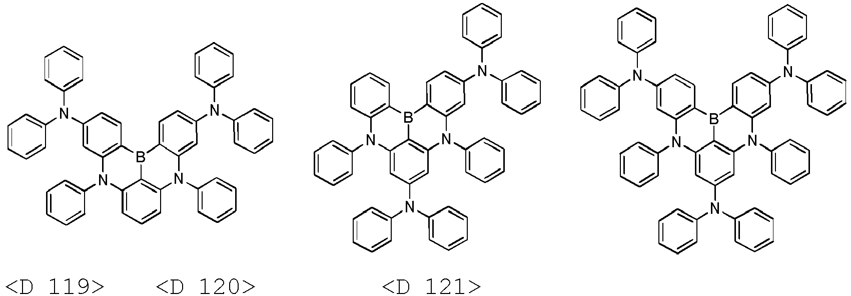

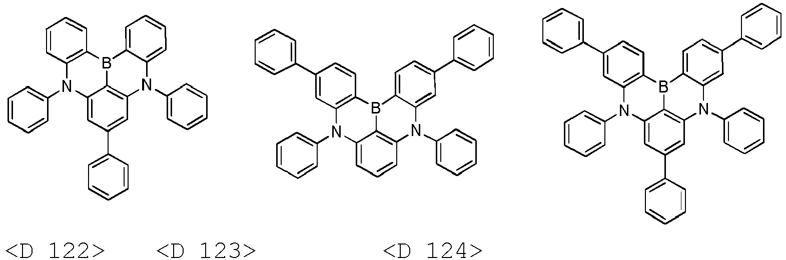

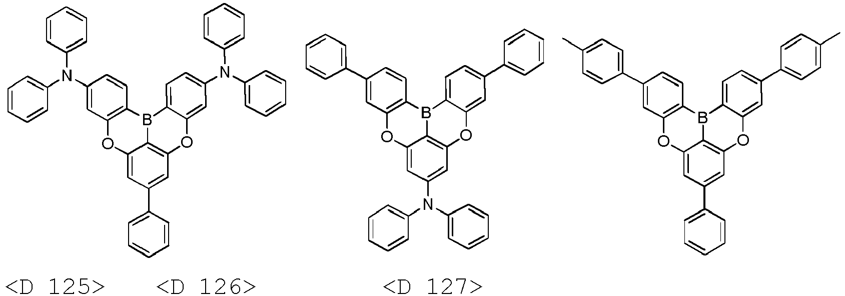

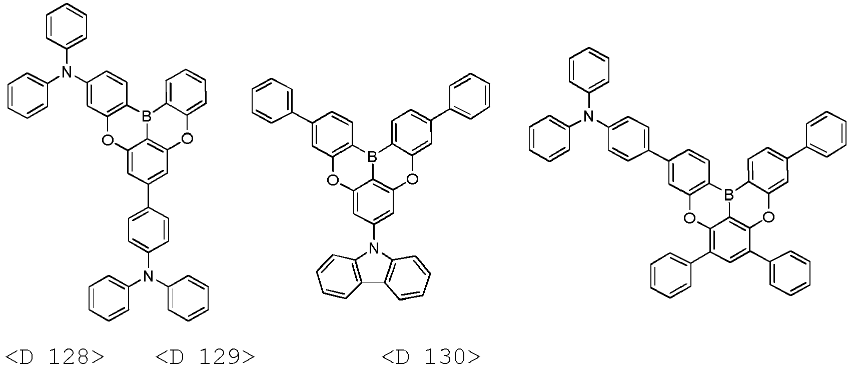

Examples of the compound represented by any one of [Chemical Formula D4], [Chemical Formula D5], and [Chemical Formula D8] to [Chemical Formula D10] include the compounds of the following [D 201] to [D 476]:

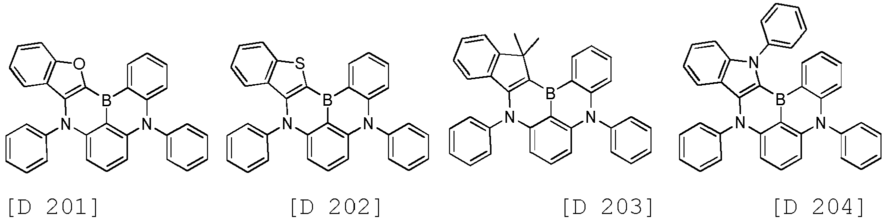

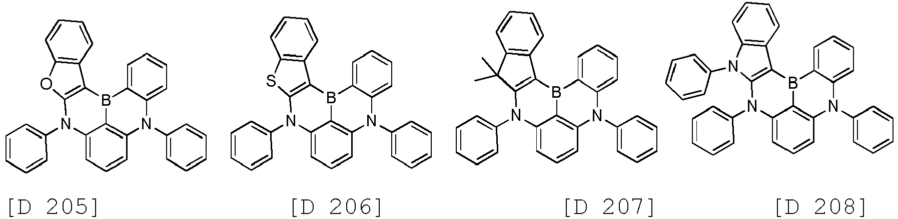

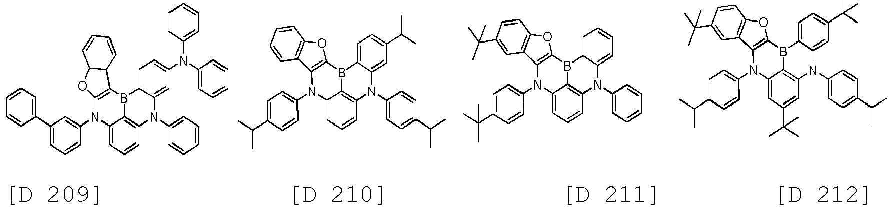

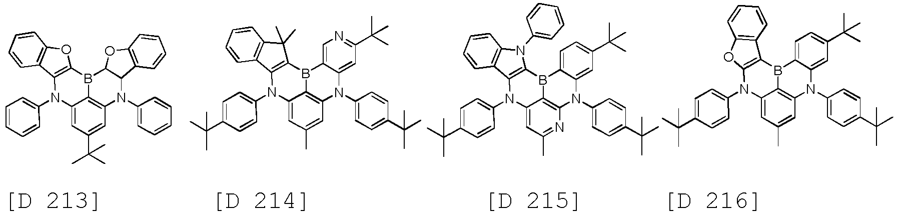

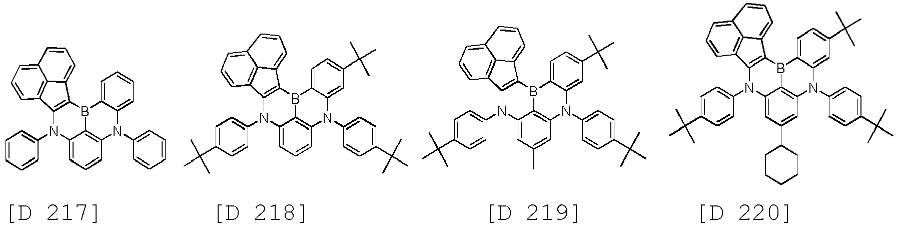

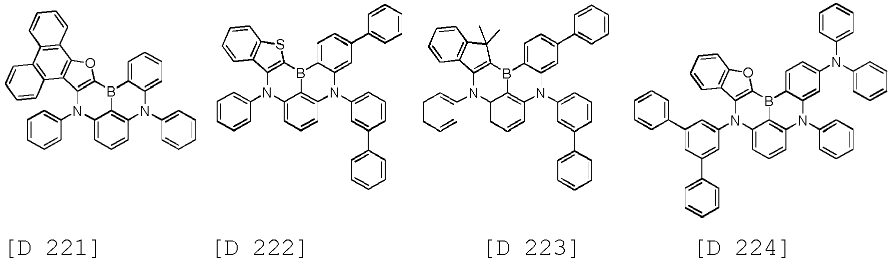

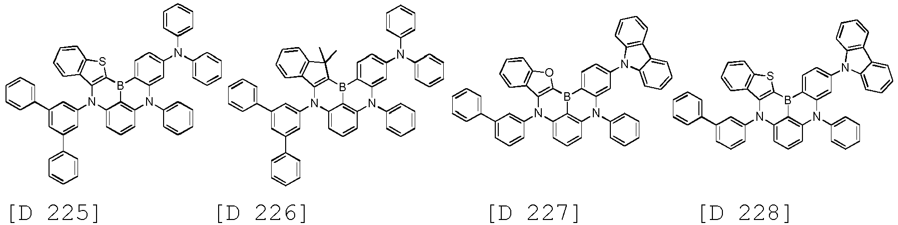

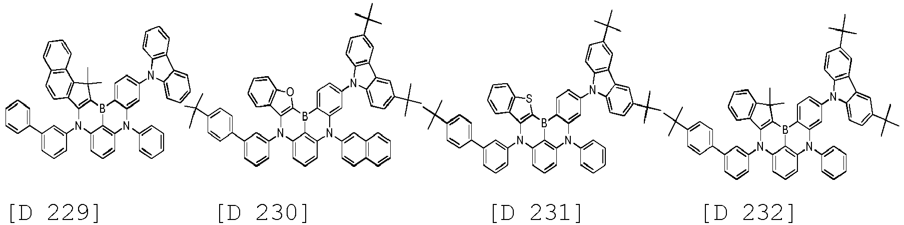

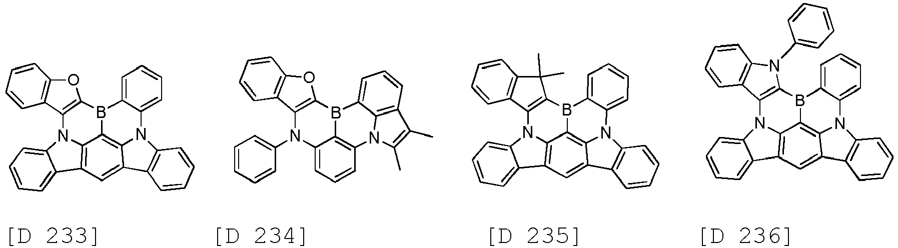

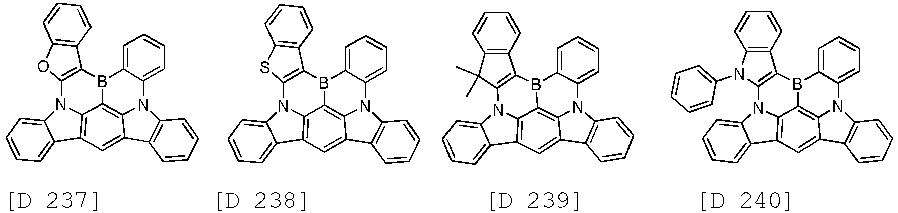

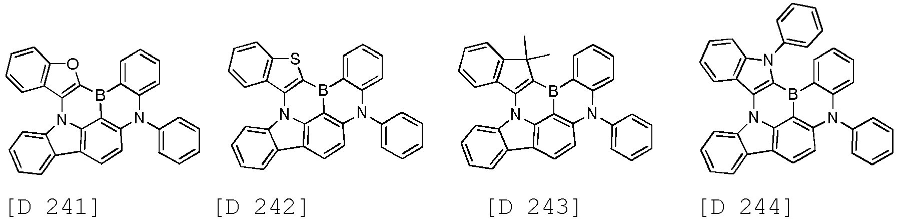

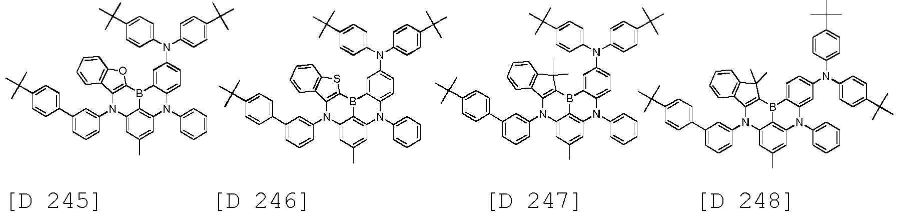

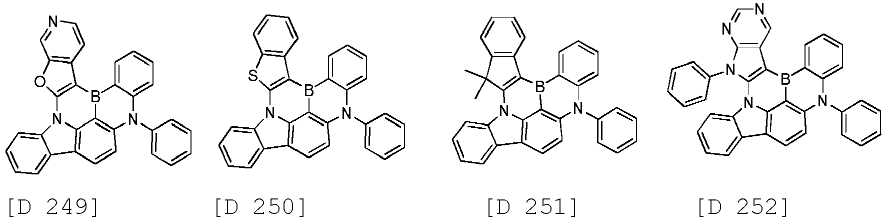

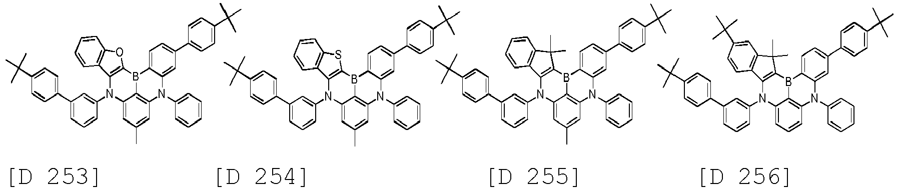

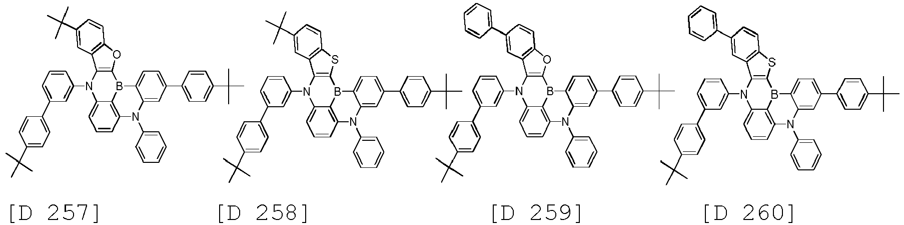

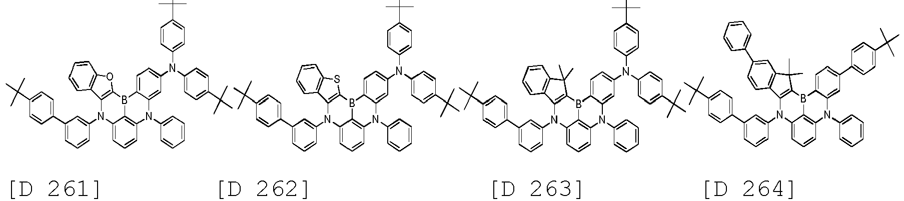

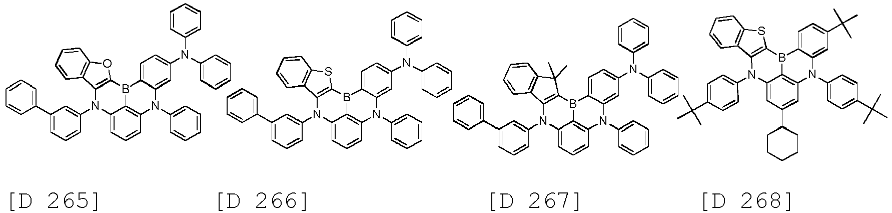

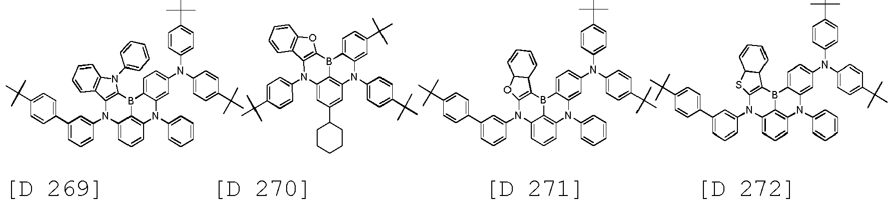

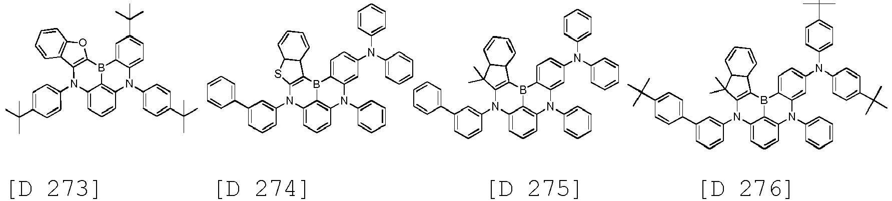

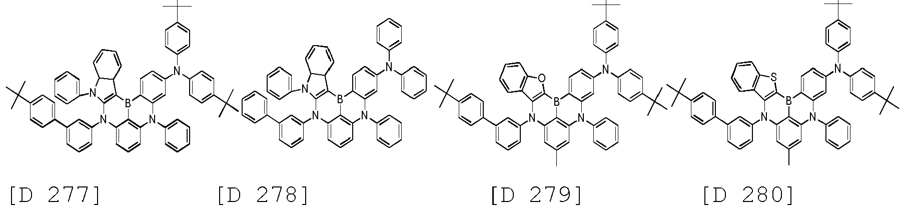

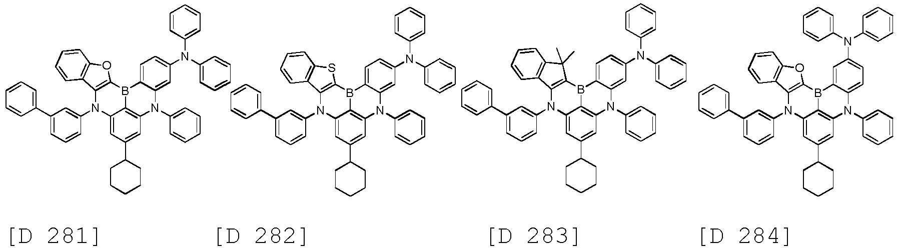

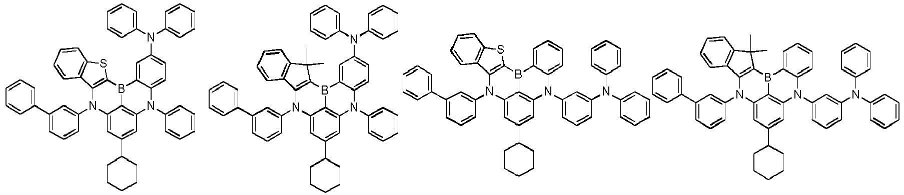

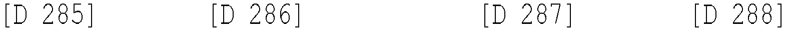

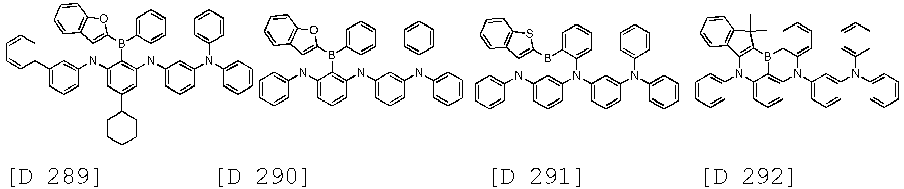

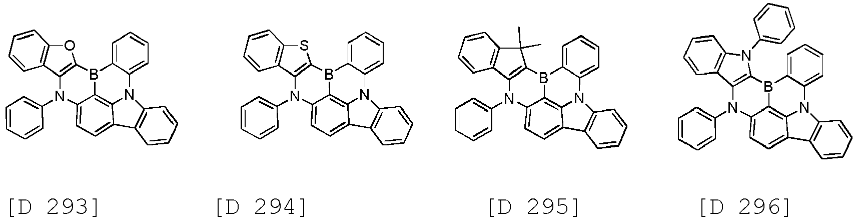

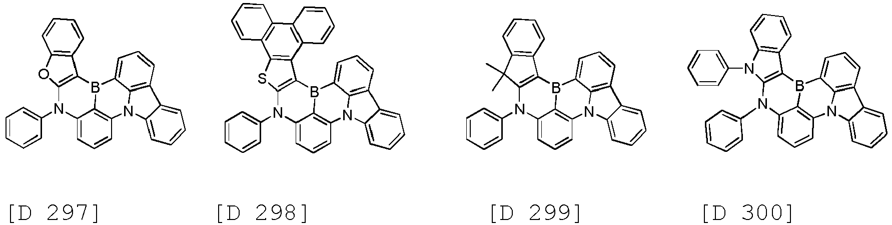

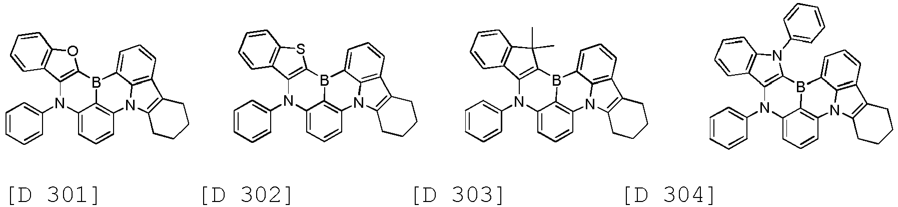

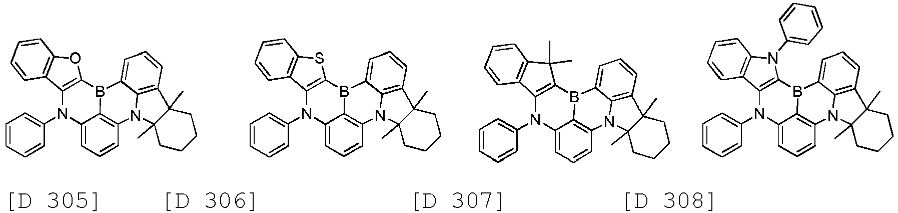

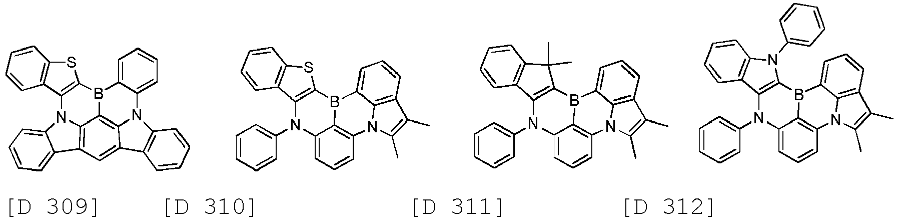

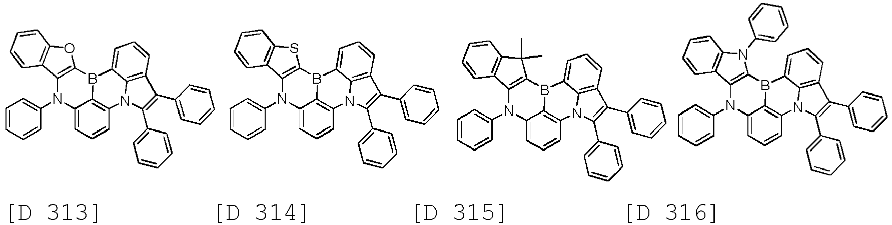

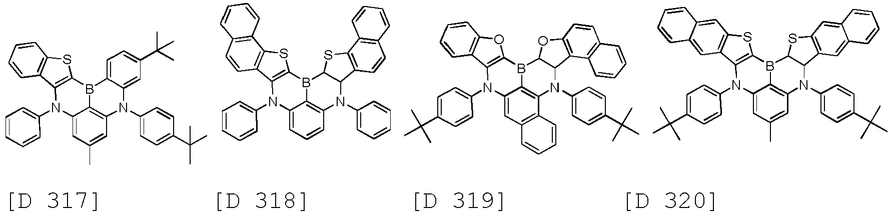

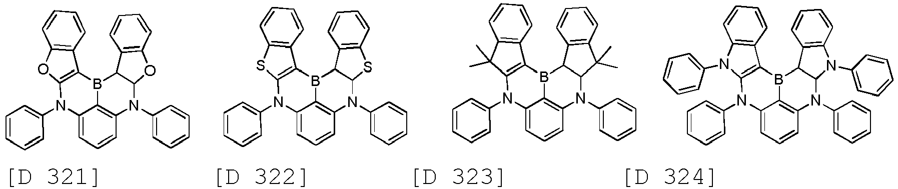

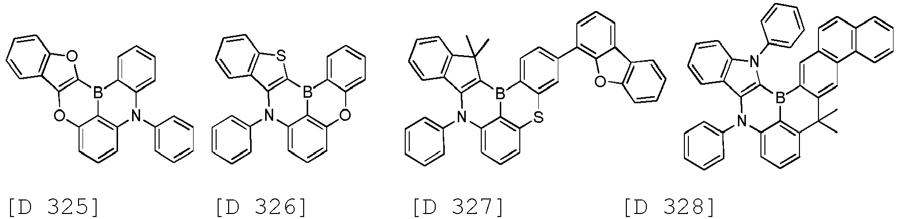

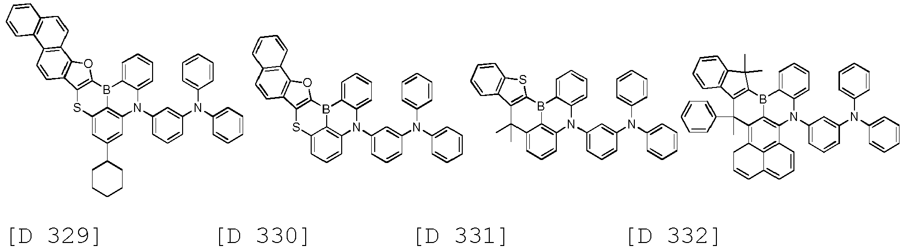

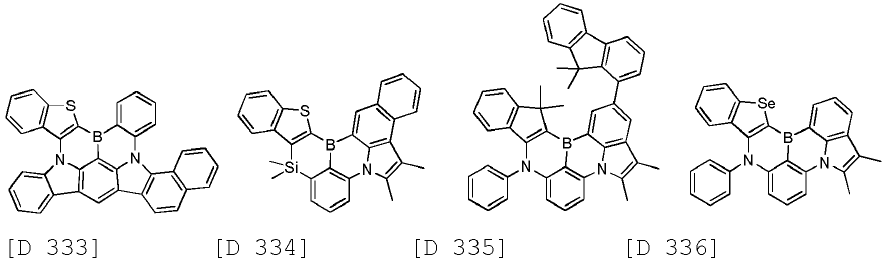

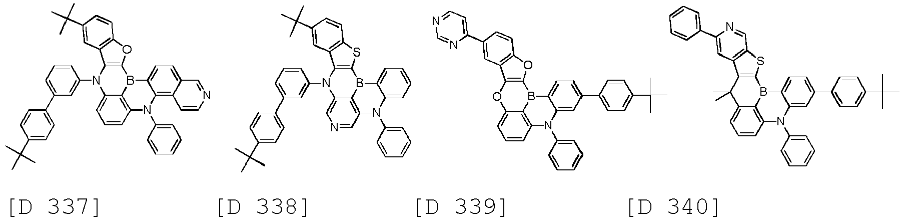

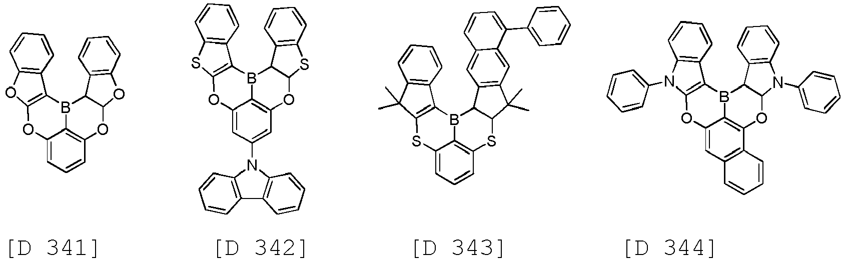

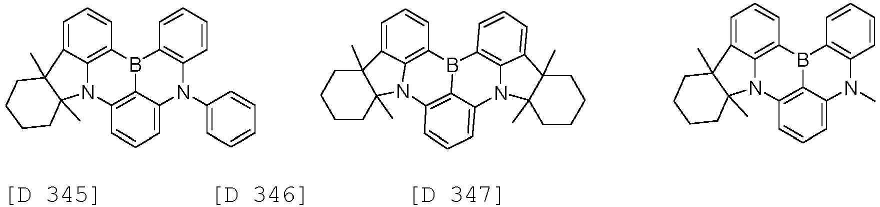

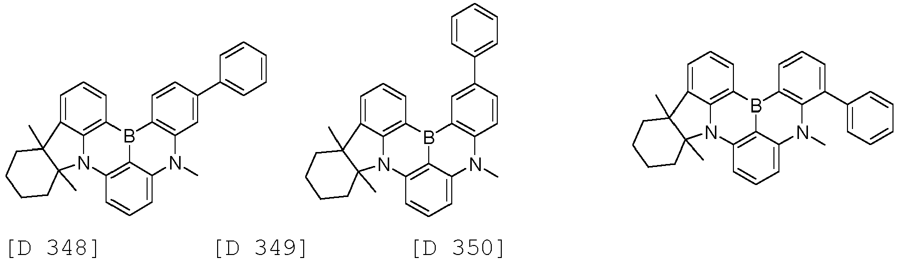

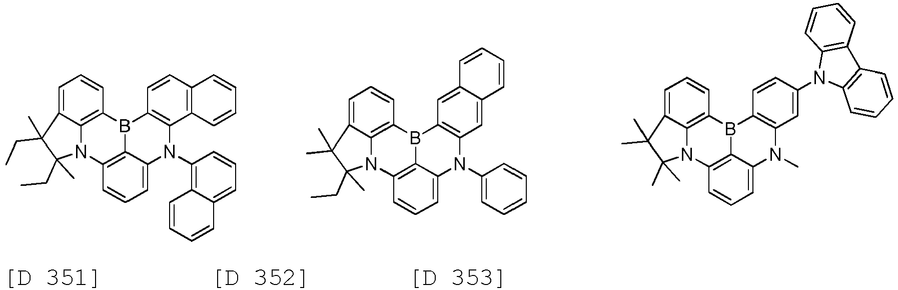

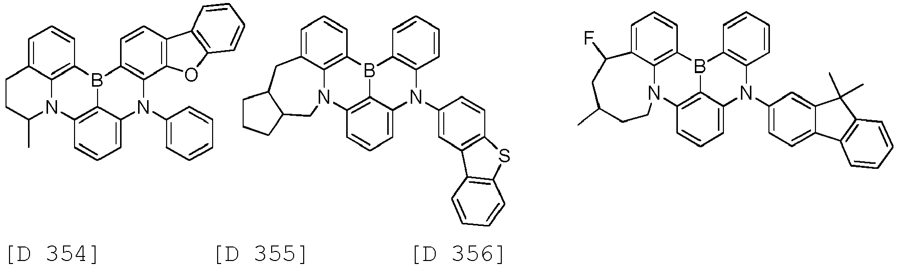

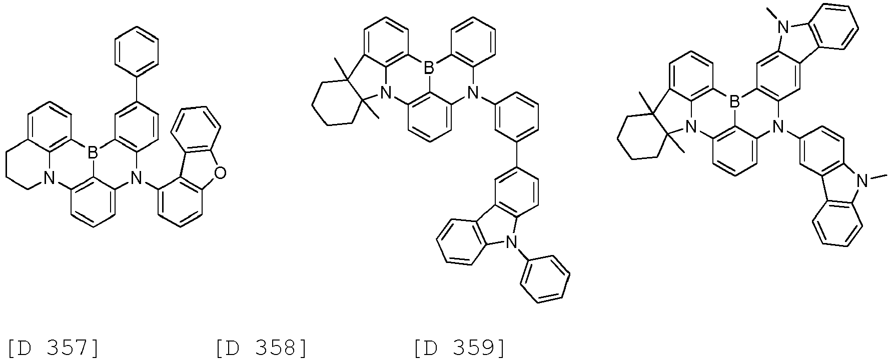

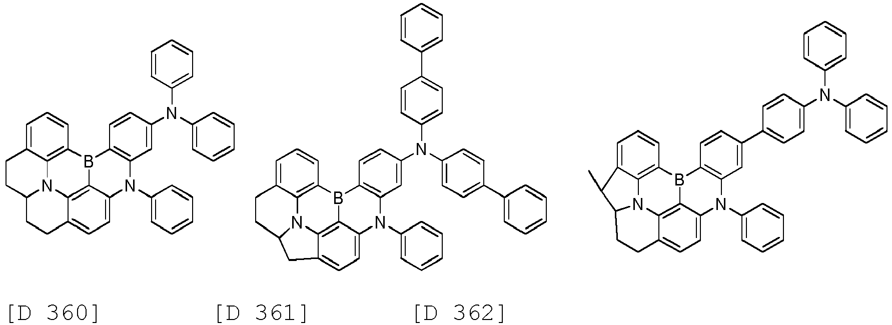

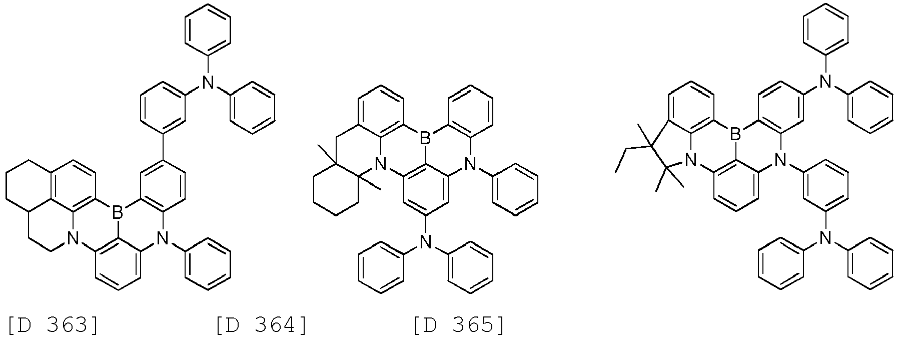

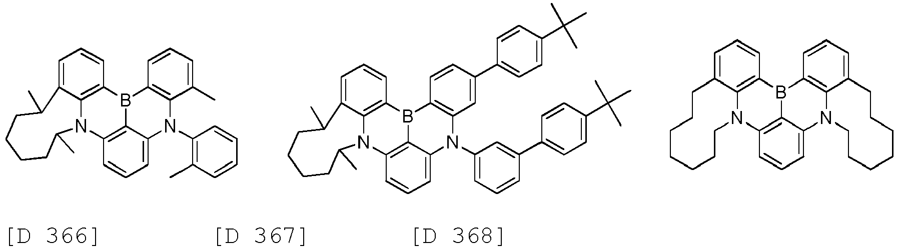

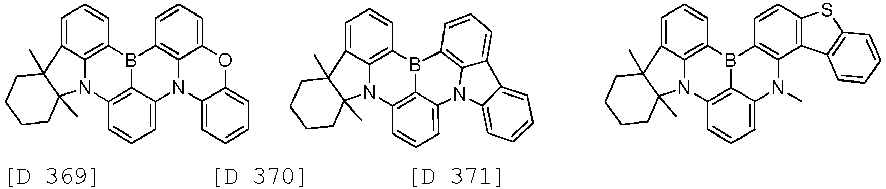

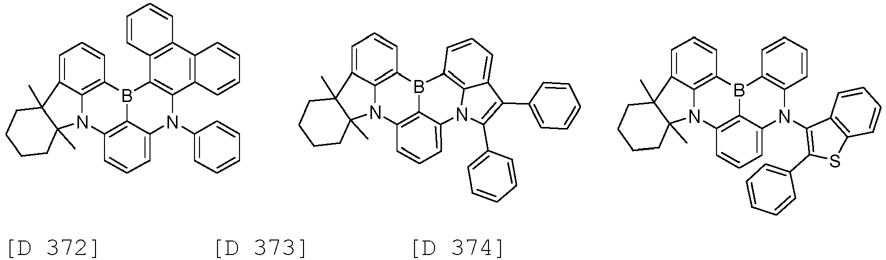

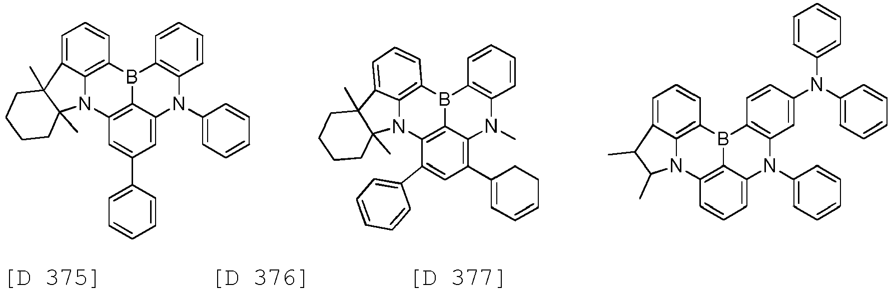

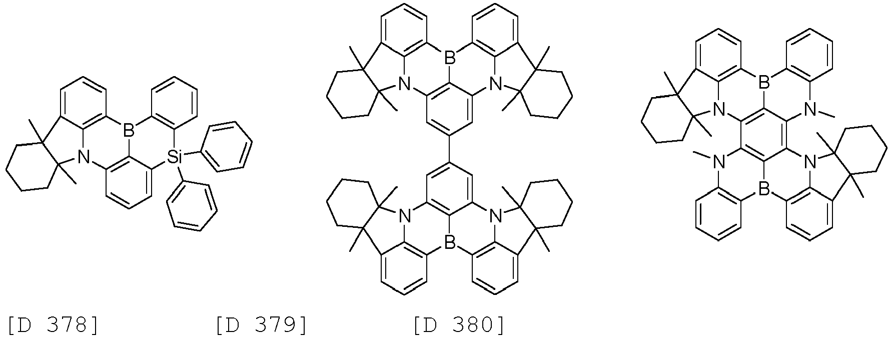

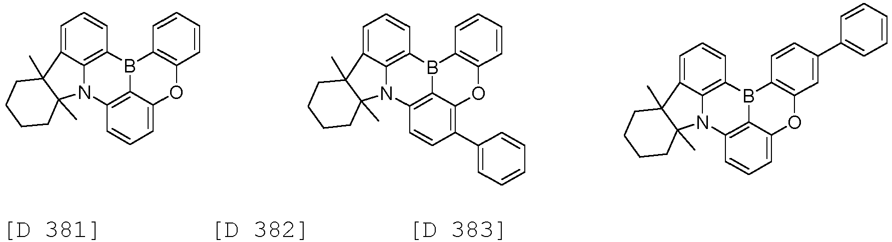

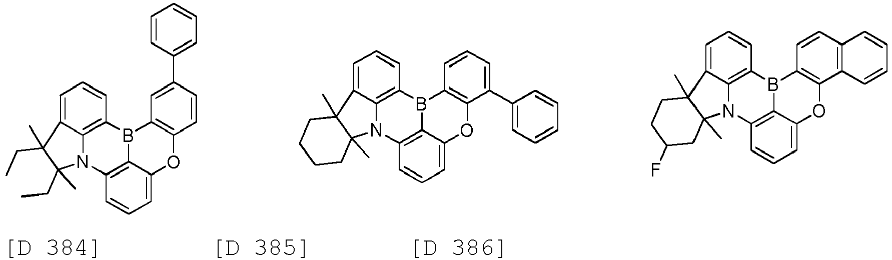

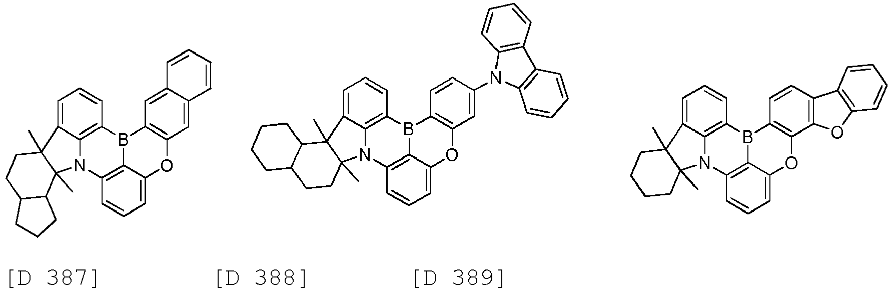

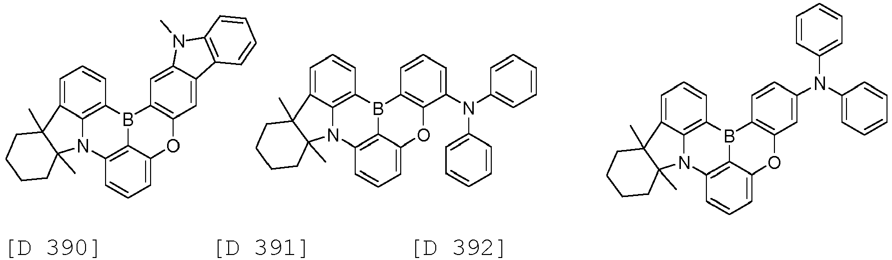

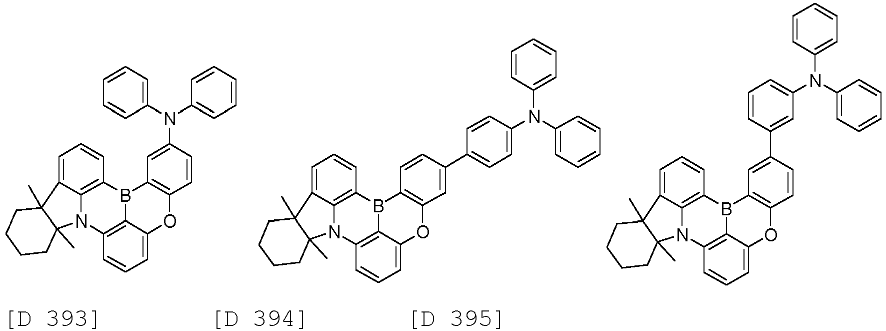

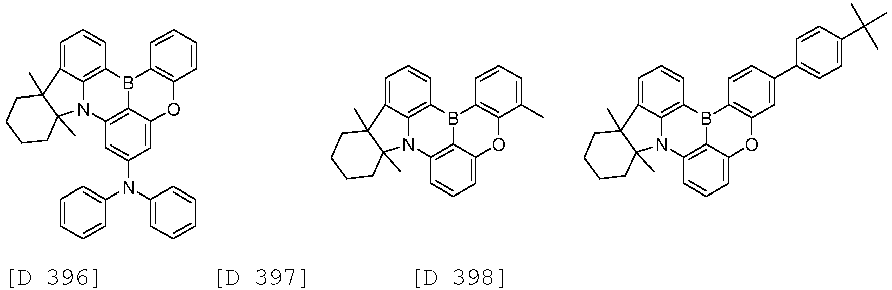

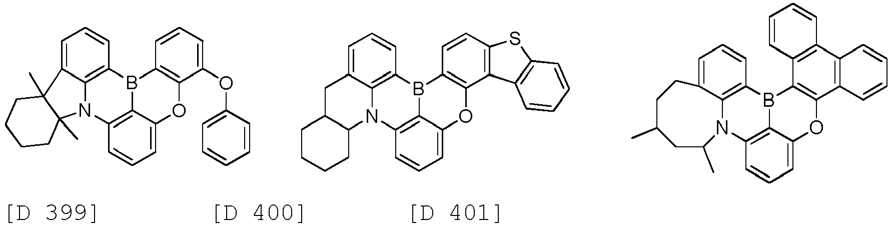

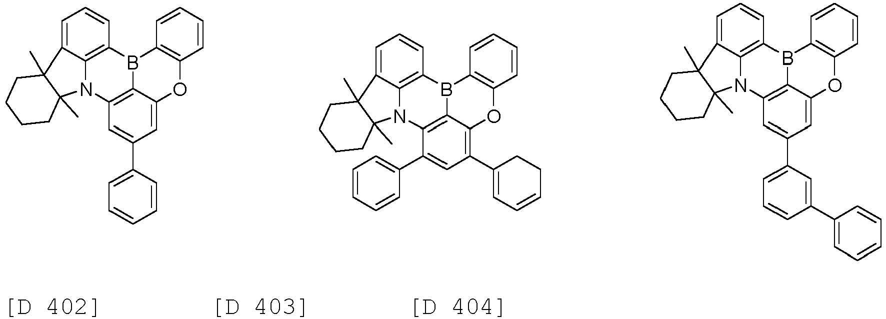

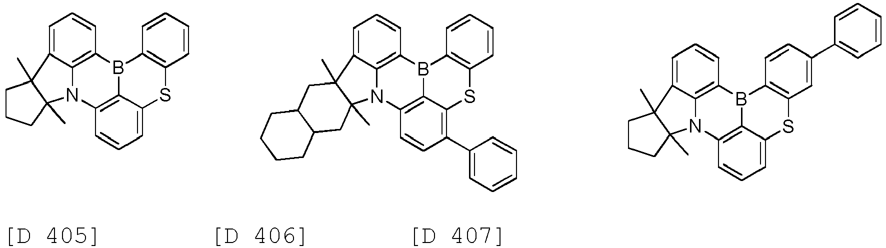

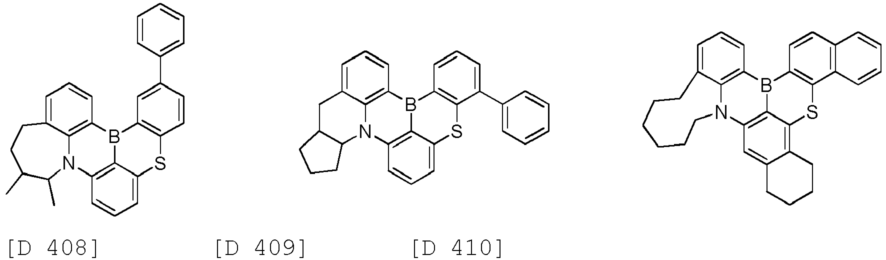

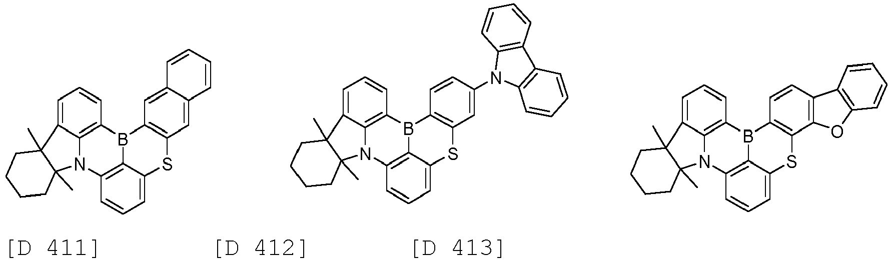

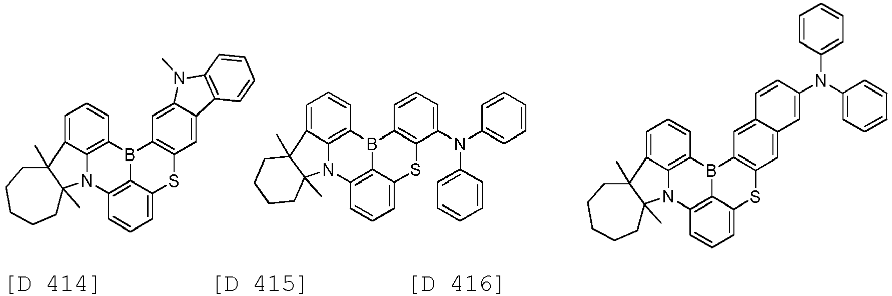

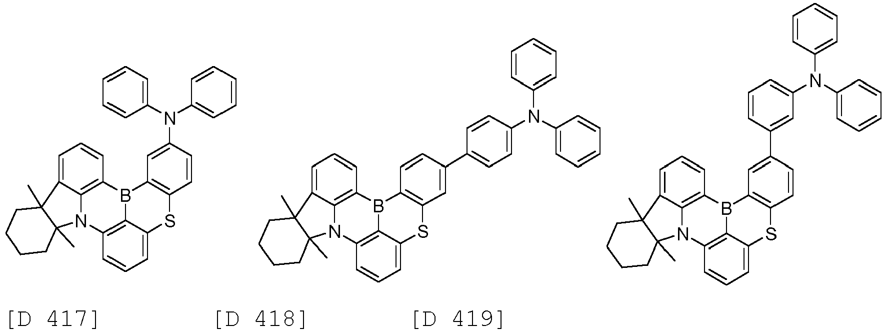

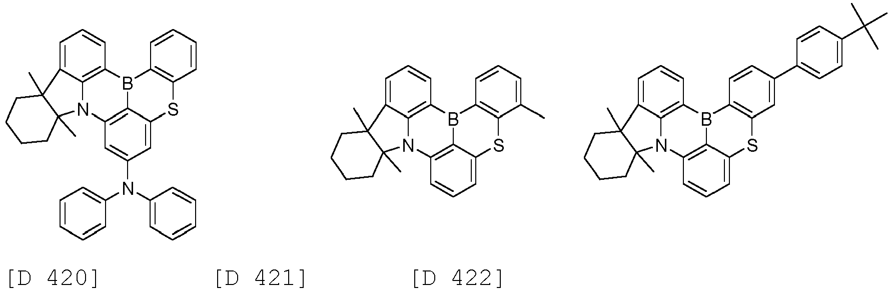

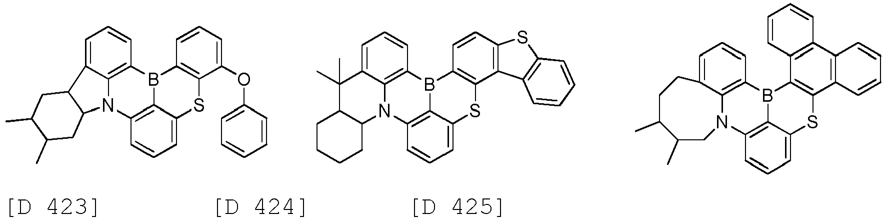

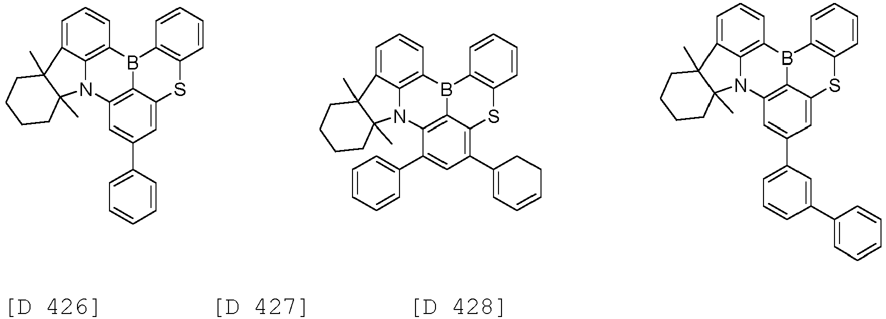

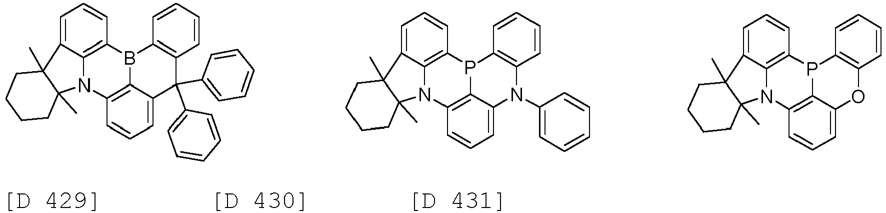

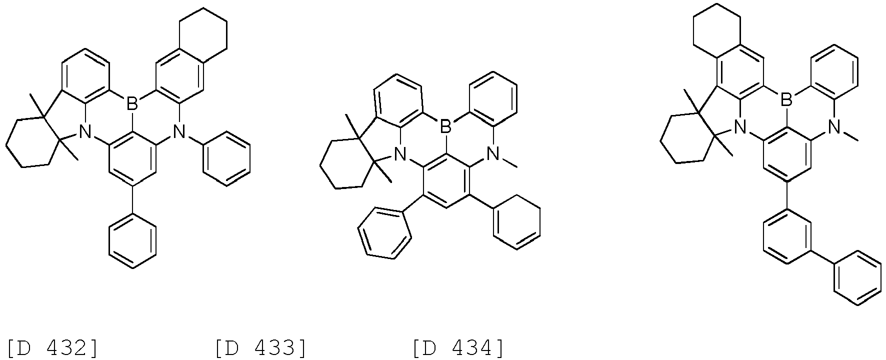

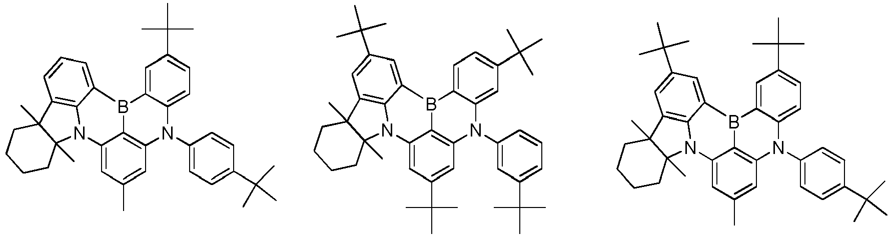

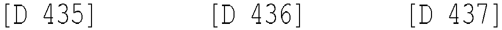

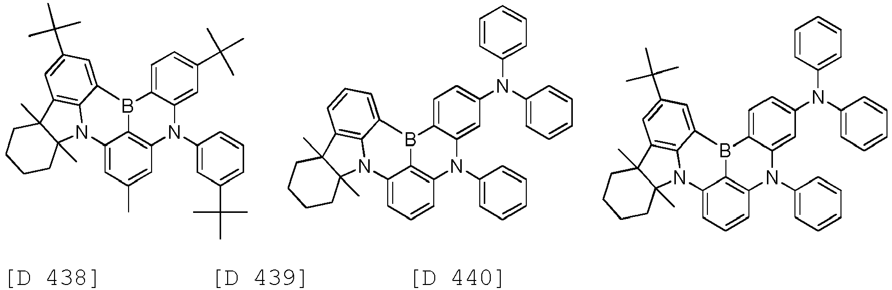

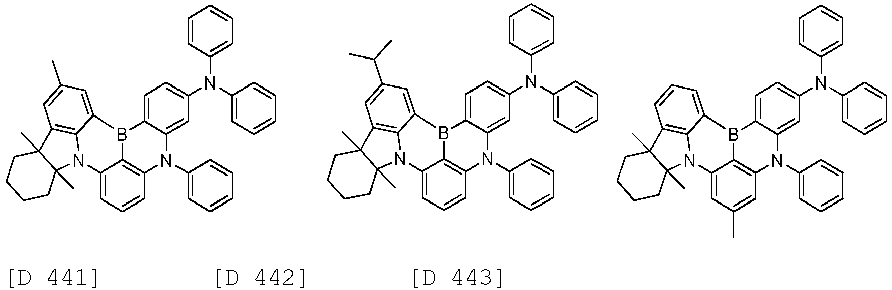

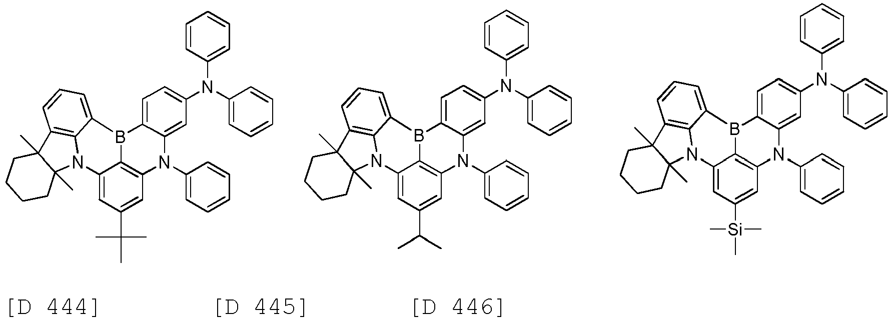

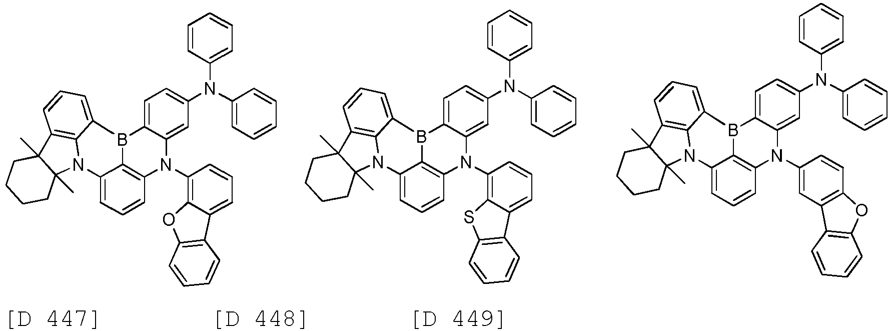

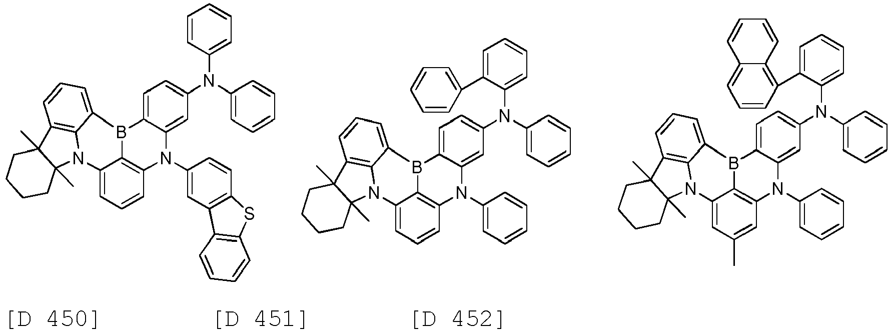

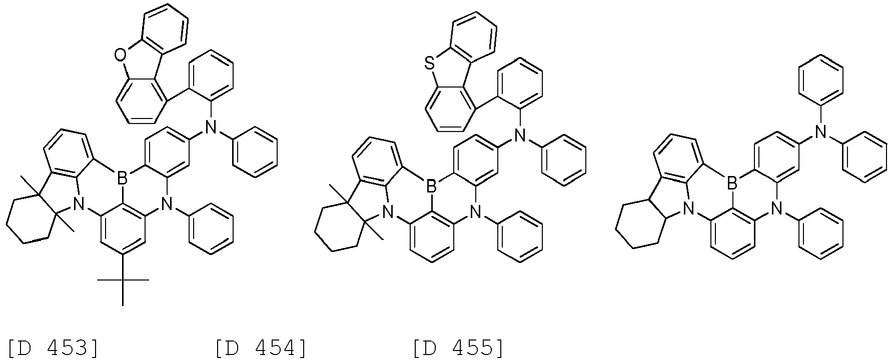

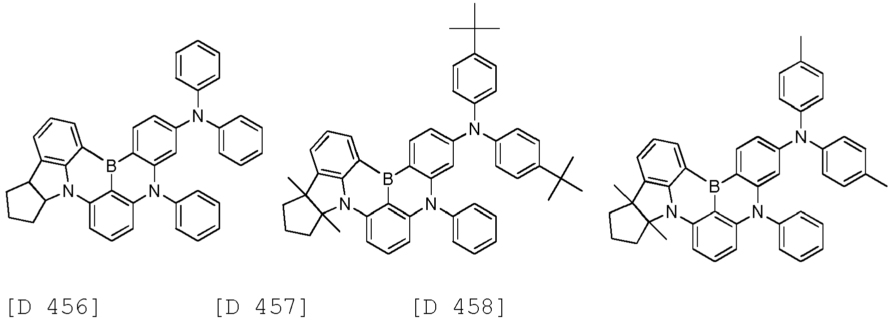

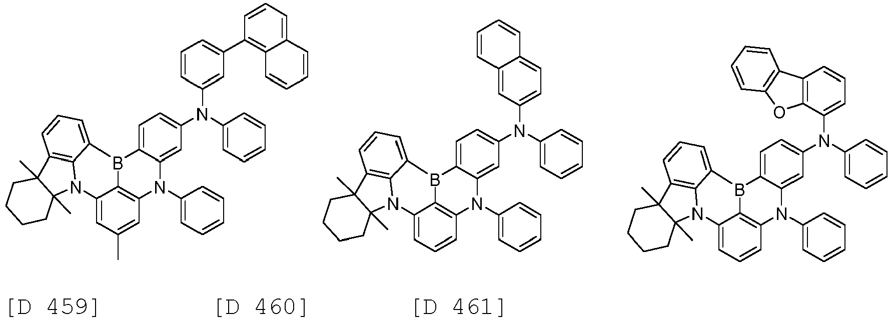

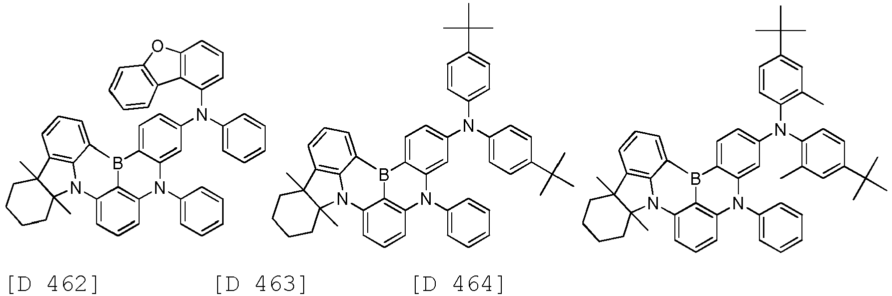

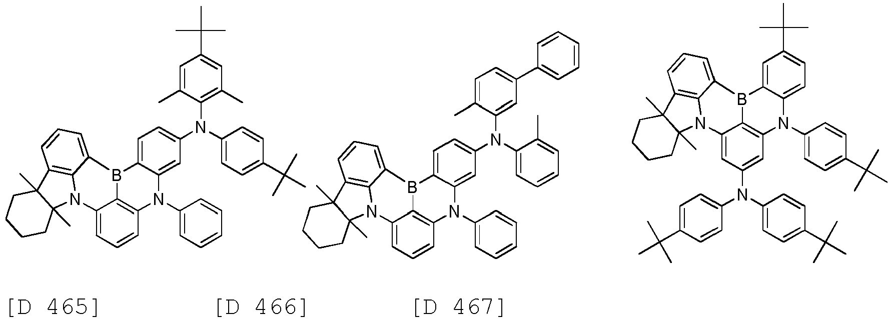

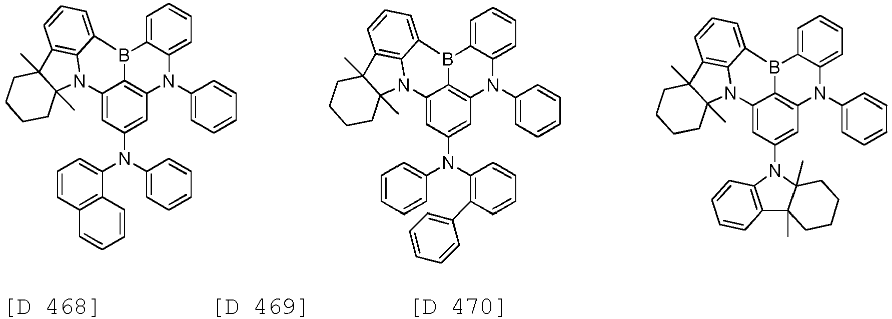

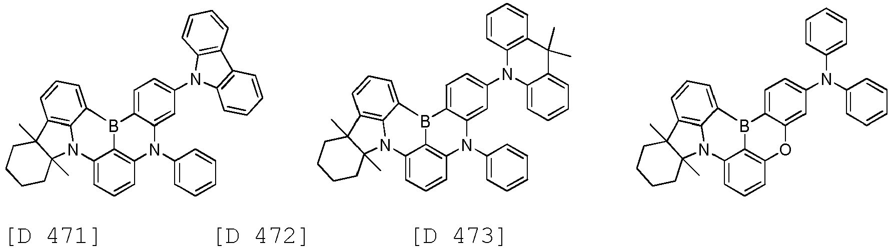

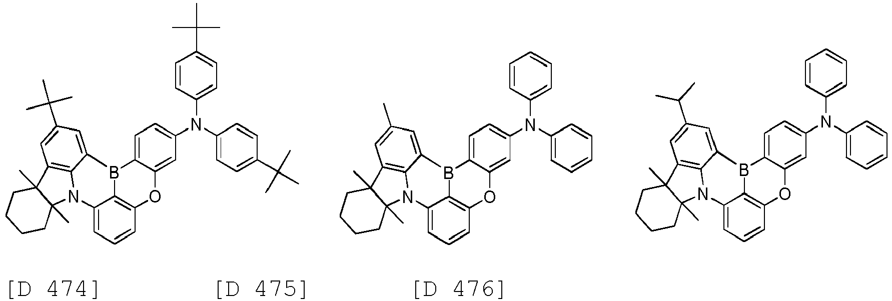

Among the dopant compounds useful in the light-emitting layer according to the present disclosure, examples of the compound represented by Chemical Formula D6 or D7 include the following <D 501> to <D 587>:

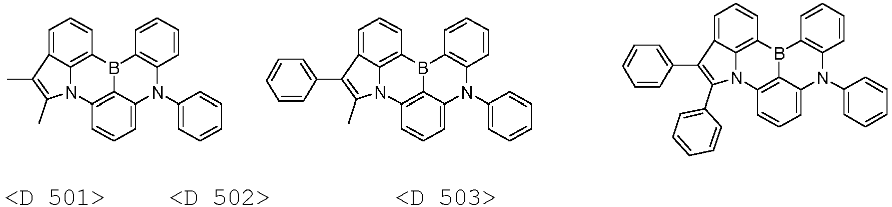

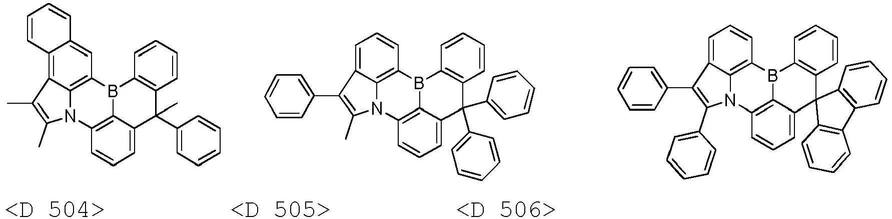

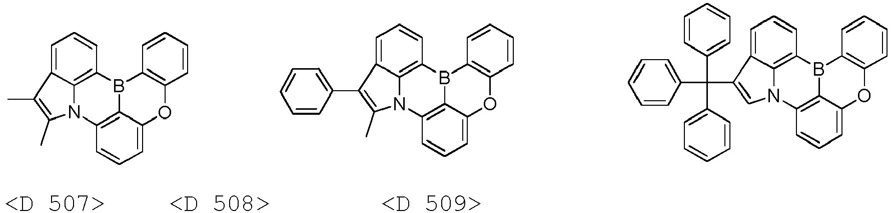

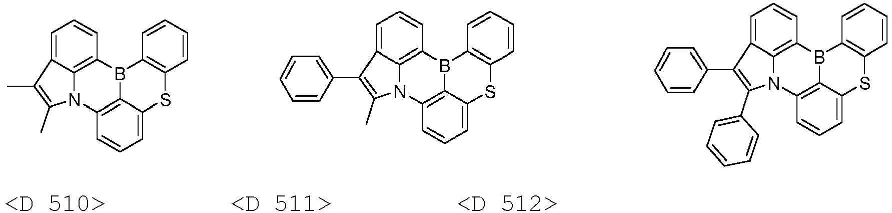

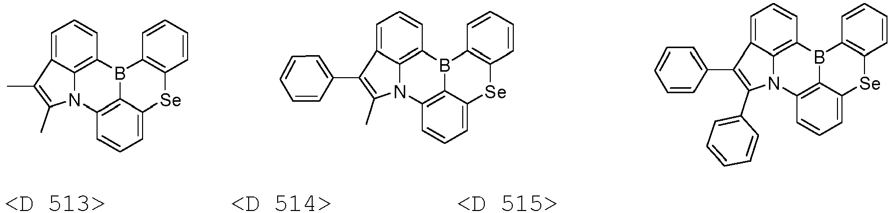

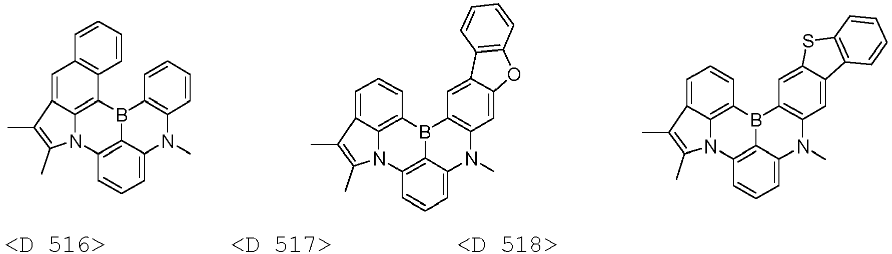

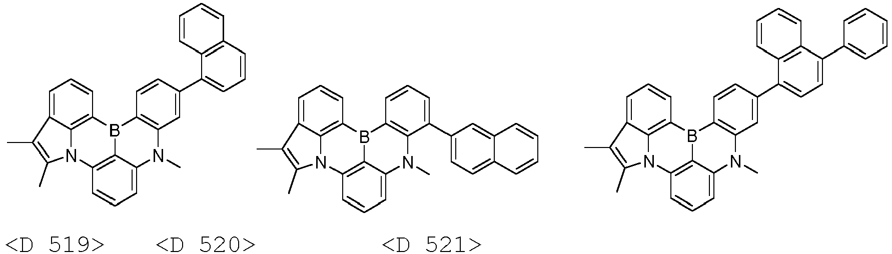

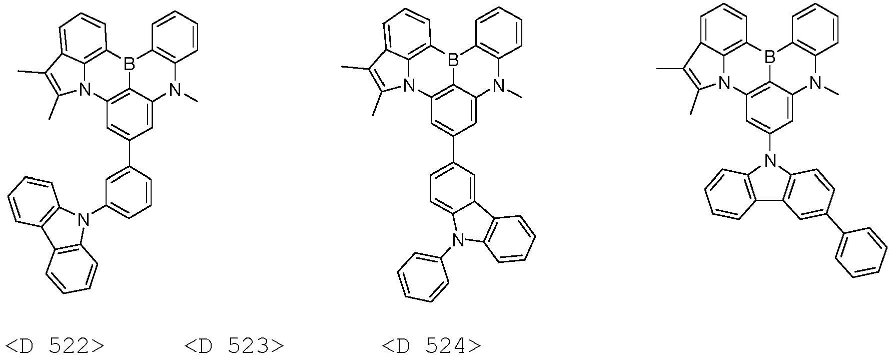

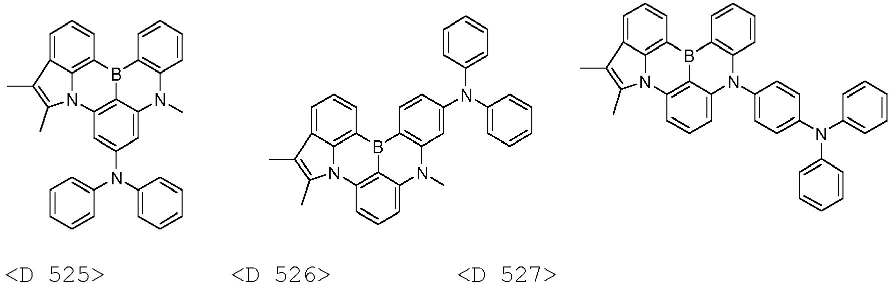

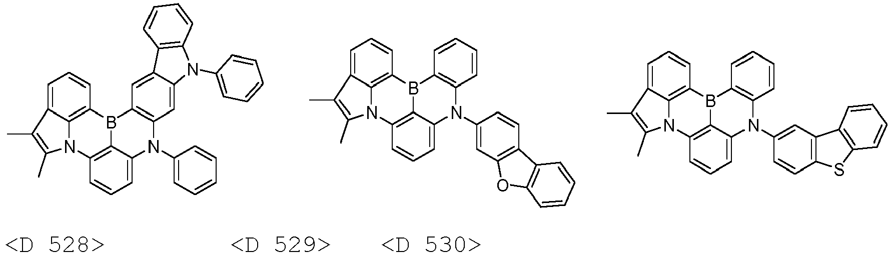

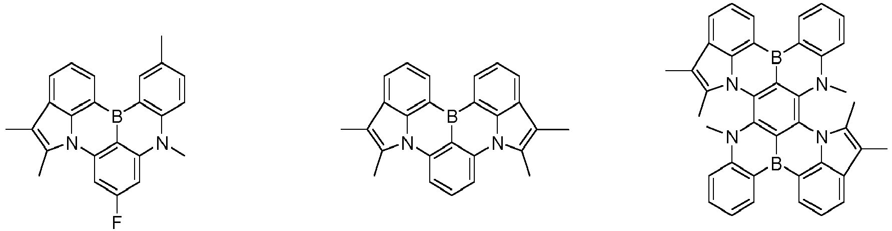

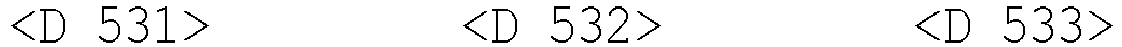

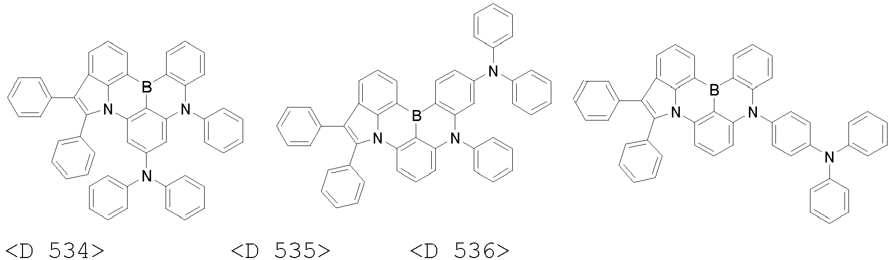

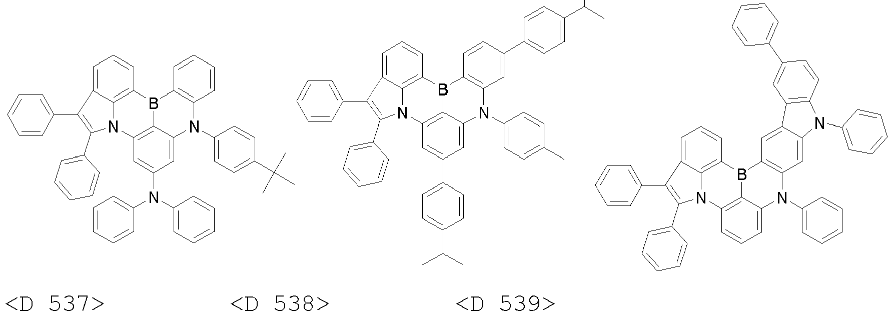

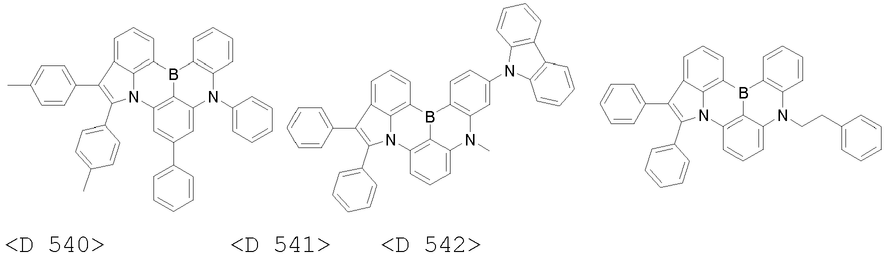

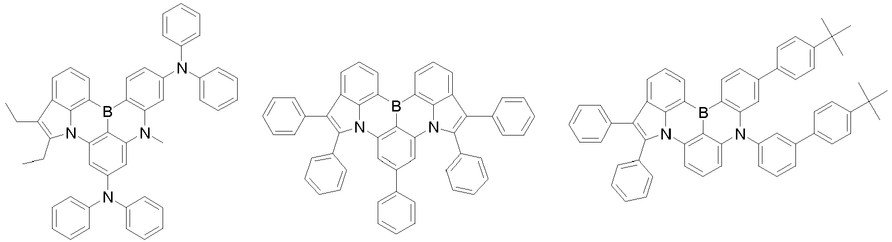

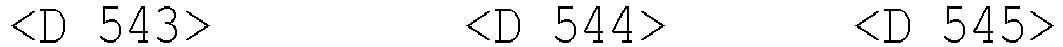

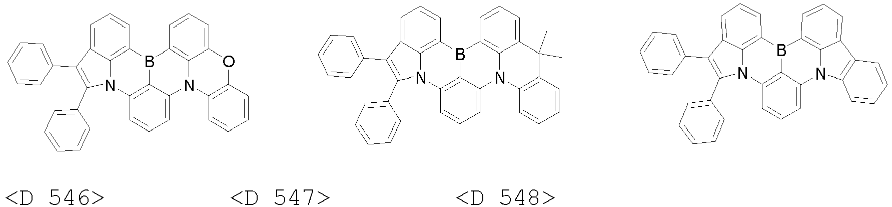

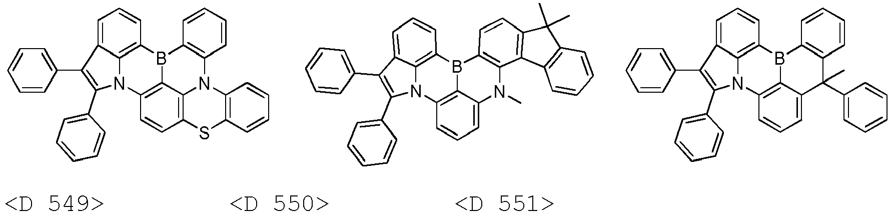

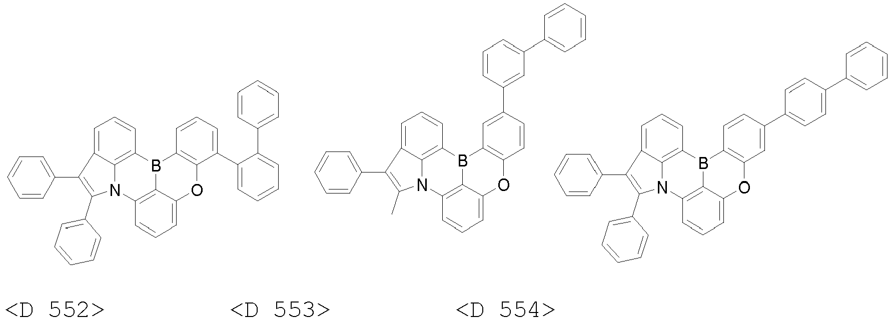

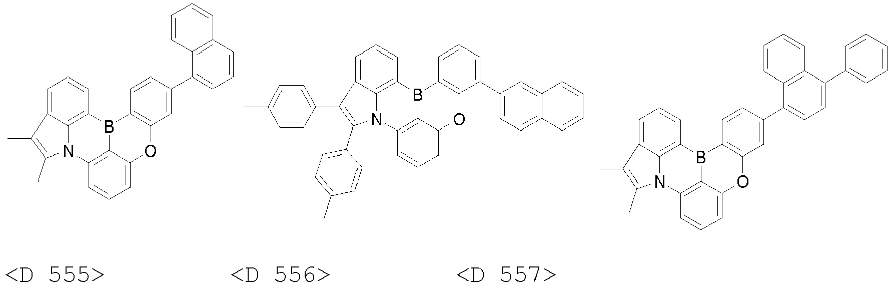

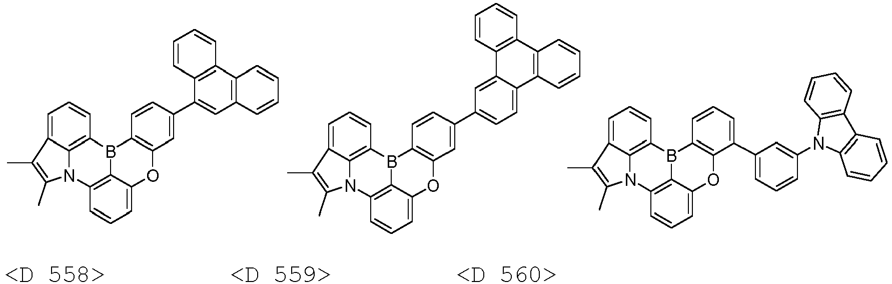

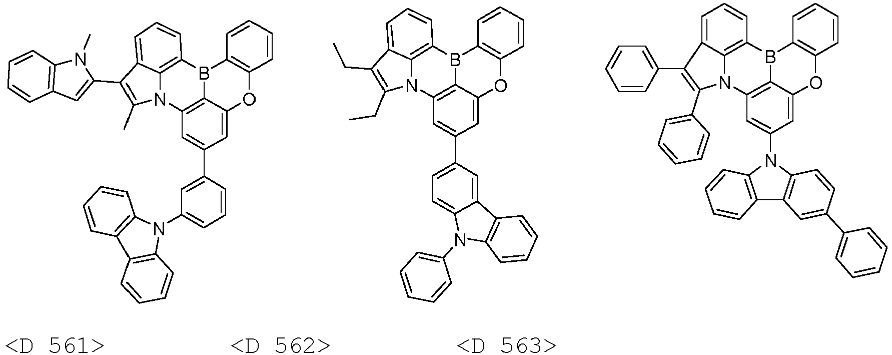

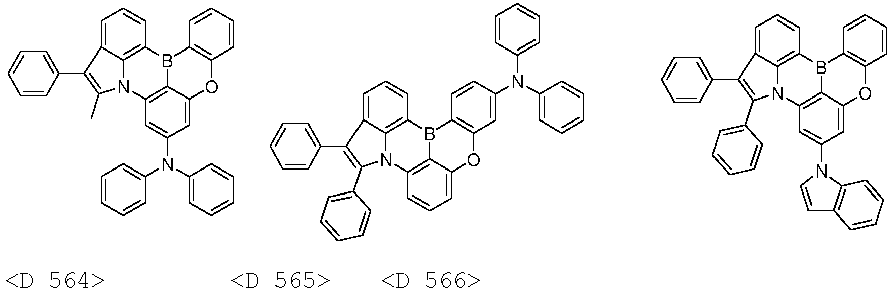

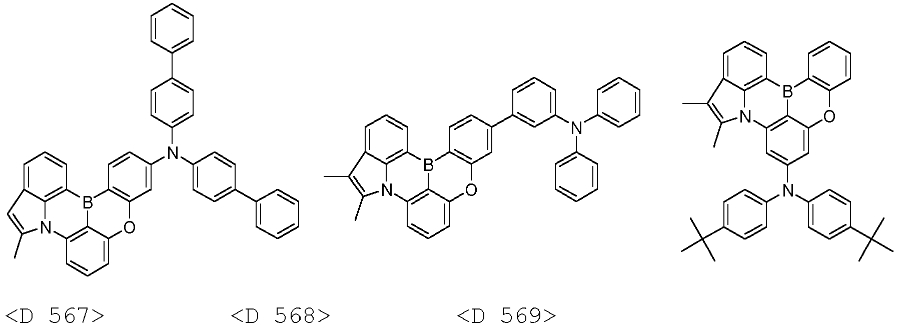

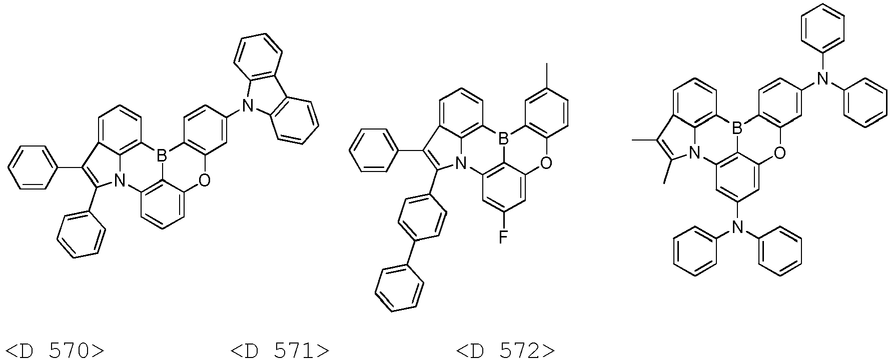

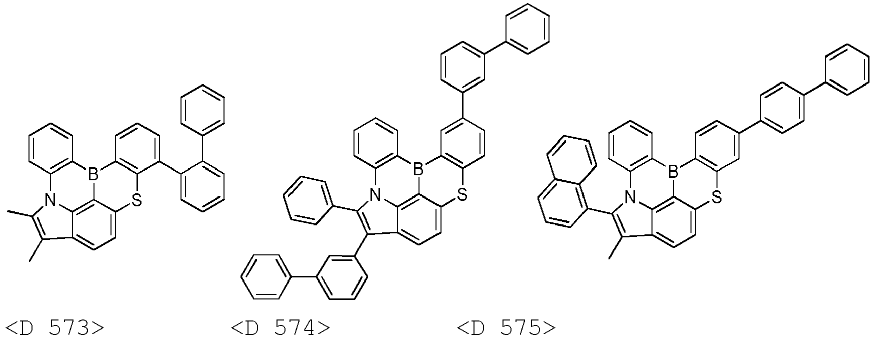

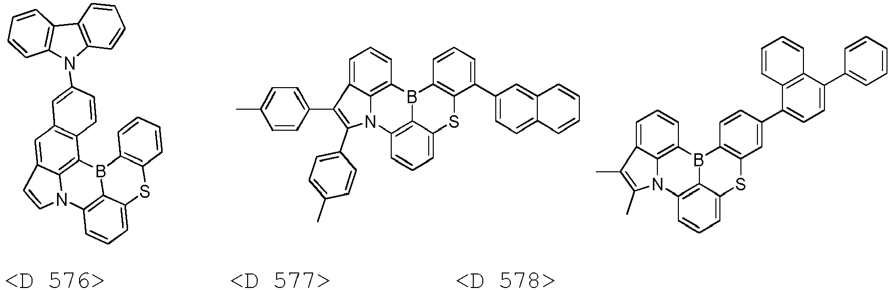

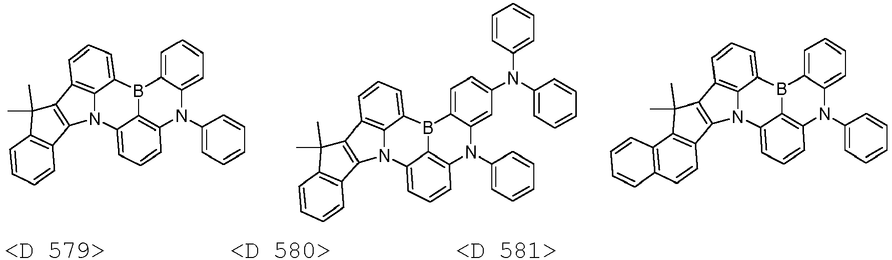

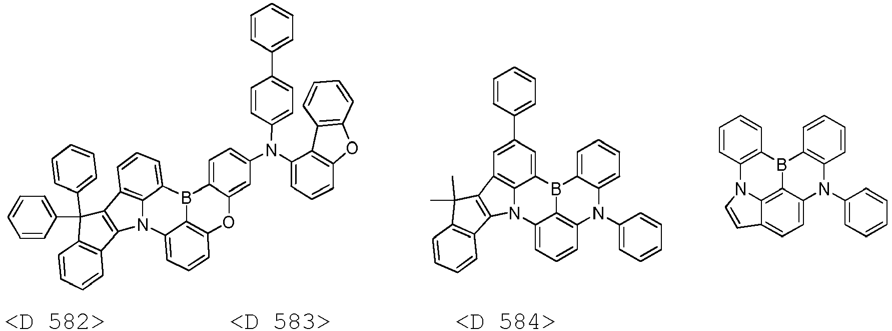

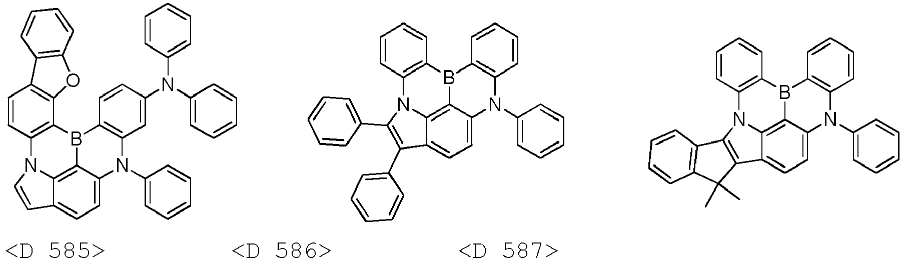

The content of the dopant in the light-emitting layer may range from about 0.01 to 20 parts by weight, based on 100 parts by weight of the host, but is not limited thereto.

In addition to the above-mentioned dopants and hosts, the light-emitting layer may further include various hosts and dopant materials.

Below, the organic light-emitting diode of the present disclosure will be explained with reference to the drawing.

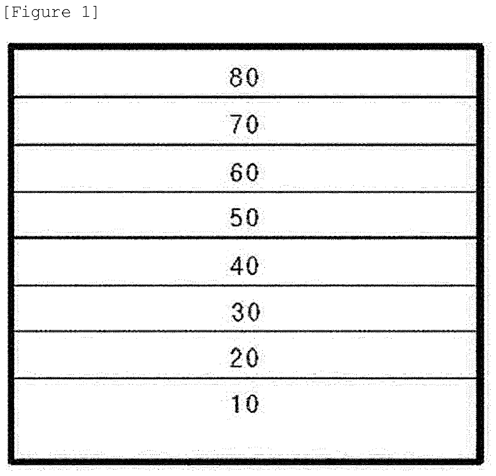

FIG. 1 is a schematic cross-sectional view of the structure of an organic light-emitting diode according to an embodiment of the present disclosure.

As shown in FIG. 1, the organic light-emitting diode according to an embodiment of the present disclosure comprises: an anode (20); a first emission part including a hole transport layer (40), a light-emitting layer (50) containing a host and a dopant, an electron transport layer (60), and a cathode (80) in that order, wherein the anode and the cathode serve as a first electrode and a second electrode, respectively, with the interposition of the hole transport layer between the anode and the light-emitting layer, and the electron transport layer between the light-emitting layer and the cathode.

Furthermore, the organic light-emitting diode according to an embodiment of the present disclosure may comprise a hole injection layer (30) between the anode (20) and the hole transport layer (40), and an electron injection layer (70) between the electron transport layer (60) and the cathode (80) .

Reference is made to Figure with regard to the organic light emitting diode of the present disclosure and the fabrication method therefor.

First, a substrate (10) is coated with an anode electrode material to form an anode (20). So long as it is used in a typical organic electroluminescence (EL) device, any substrate may be used as the substrate (10). Preferable is an organic substrate or transparent plastic substrate that exhibits excellent transparency, surface smoothness, ease of handling, and waterproofness. As the anode electrode material, indium tin oxide (ITO), indium zinc oxide (IZO), tin oxide (SnO₂), or zinc oxide (ZnO), which are transparent and superior in terms of conductivity, may be used.

A hole injection layer material is applied on the anode (20) by thermal deposition in a vacuum or by spin coating to form a hole injection layer (30). Subsequently, thermal deposition in a vacuum or by spin coating may also be conducted to form a hole transport layer (40) with a hole transport layer material on the hole injection layer (30).

So long as it is typically used in the art, any material may be selected for the hole injection layer without particular limitations thereto. Examples include, but are not limited to, 2-TNATA [4,4',4"-tris(2-naphthylphenyl-phenylamino)-triphenylamine], NPD [N,N'-di(1-naphthyl)-N,N'-diphenylbenzidine)], TPD [N,N'-diphenyl-N,N'-bis(3-methylphenyl)-1,1'-biphenyl-4,4'-diamine], and DNTPD [N,N'-diphenyl-N,N'-bis-[4-(phenyl-m-tolyl-amino)-phenyl]-biphenyl-4,4'-diamine].

Any material that is typically used in the art may be selected for the hole transport layer without particular limitations thereto. Examples include, but are not limited to, N,N'-bis(3-methylphenyl)-N,N'-diphenyl-[1,1-biphenyl]-4,4'-diamine (TPD) and N,N'-di(naphthalen-1-yl)-N,N'-diphenylbenzidine (a-NPD).

In an embodiment of the present disclosure, an electron blocking layer may be additionally disposed on the hole transport layer. Functioning to prevent the electrons injected from the electron injection layer from entering the hole transport layer from the light-emitting layer, the electron blocking layer is adapted to increase the life span and luminous efficiency of the diode. The electron blocking layer may be formed at a suitable position between the light emitting layer and the hole injection layer. Particularly, the electron blocking layer may be formed between the light emitting layer and the hole transport layer.

Next, the light-emitting layer (50) may be deposited on the hole transport layer (40) or the electron blocking layer by deposition in a vacuum or by spin coating.

Herein, the light-emitting layer may contain a host and a dopant and the materials are as described above.

In some embodiments of the present disclosure, the light-emitting layer particularly ranges in thickness from 50 to 2,000 Å.

Meanwhile, the electron transport layer (60) is applied on the light-emitting layer by deposition in a vacuum and spin coating.

A material for use in the electron transport layer functions to stably carry the electrons injected from the electron injection electrode (cathode), and may be an electron transport material known in the art. Examples of the electron transport material known in the art include quinoline derivatives, particularly, tris(8-quinolinolate)aluminum (Alq₃), Liq, TAZ, BAlq, beryllium bis(benzoquinolin-10-olate) (Bebq₂), Compound 201, Compound 202, BCP, and oxadiazole derivatives such as PBD, BMD, and BND, but are not limited thereto:

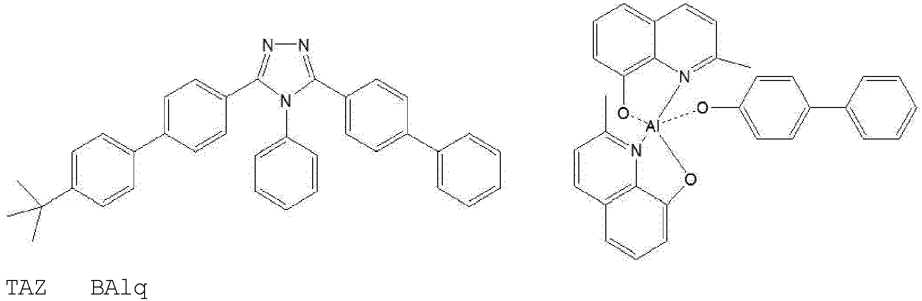

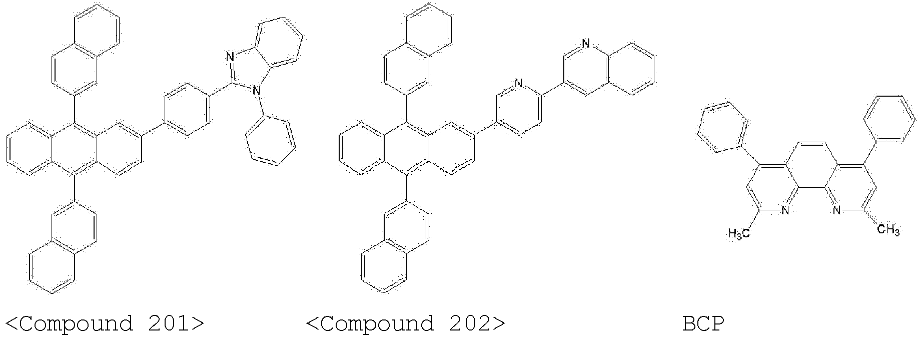

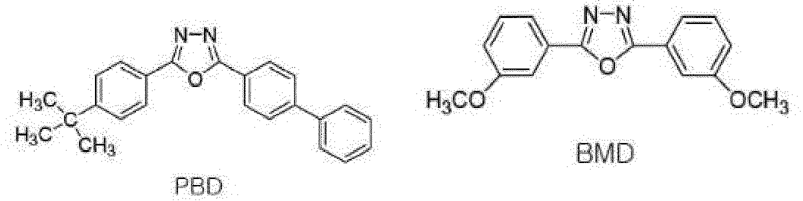

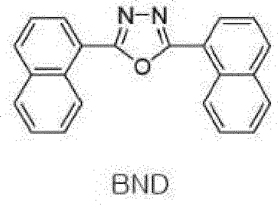

In the organic light emitting diode of the present disclosure, an electron injection layer (EIL) that functions to facilitate electron injection from the cathode may be deposited on the electron transport layer. The material for the EIL is not particularly limited.

Any material that is conventionally used in the art can be available for the electron injection layer without particular limitations. Examples include CsF, NaF, LiF, Li₂O, and BaO. Deposition conditions for the electron injection layer may vary, depending on compounds used, but may be generally selected from condition scopes that are almost the same as for the formation of hole injection layers.

The electron injection layer may range in thickness from about 1 Å to about 100 Å, and particularly from about 3 Å to about 90 Å. Given the thickness range for the electron injection layer, the diode can exhibit satisfactory electron injection properties without actually elevating a driving voltage.

In order to facilitate electron injection, the cathode may be made of a material having a small work function, such as metal or metal alloy such as lithium (Li), magnesium (Mg), calcium (Ca), an alloy aluminum (Al) thereof, aluminum-lithium (Al-Li), magnesium-indium (Mg-In), and magnesium-silver (Mg-Ag). Alternatively, ITO or IZO may be employed to form a transparent cathode for an organic light-emitting diode.

Moreover, the organic light-emitting diode of the present disclosure may further comprise a light-emitting layer containing a blue, green, or red luminescent material that emits radiations in the wavelength range of 380 nm to 800 nm. That is, the light-emitting layer in the present disclosure has a multi-layer structure wherein the blue, green, or red luminescent material may be a fluorescent material or a phosphorescent material.

Furthermore, at least one selected from among the layers may be deposited using a single-molecule deposition process or a solution process.

Here, the deposition process is a process by which a material is vaporized in a vacuum or at a low pressure and deposited to form a layer, and the solution process is a method in which a material is dissolved in a solvent and applied for the formation of a thin film by means of inkjet printing, roll-to-roll coating, screen printing, spray coating, dip coating, spin coating, etc.

Also, the organic light-emitting diode of the present disclosure may be applied to a device selected from among flat display devices, flexible display devices, monochrome or grayscale flat illumination devices, and monochrome or grayscale flexible illumination devices.

A better understanding of the present disclosure may be obtained through the following examples which are set forth to illustrate, but are not to be construed as limiting the present disclosure.

### (EXAMPLES)

### SYNTHESIS EXAMPLE 1. Synthesis of Chemical Formula 19

### Synthesis Example 1-1. Synthesis of <1-a>

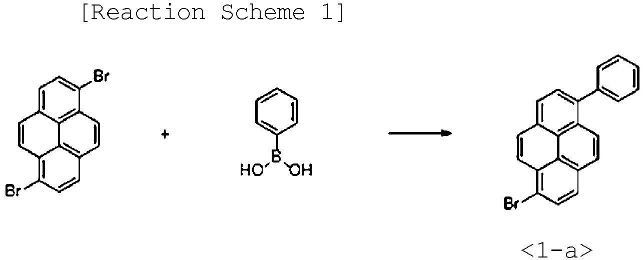

In a 3000-ml round-bottom flask purged with nitrogen, 1,6-dibromopyrene (100 g, 0.278 mol), phenylboronic acid (33.9 g, 0.278 mol), tetrakis(triphenylphosphine)palladium (Pd[PPh₃]₄) (6.4 g, 0.006 mol), sodium carbonate (88.3 g, 0.833 mol), toluene (1400 ml), and water (420 ml) were refluxed together for 9 hours. After completion of the reaction, the reaction mixture was cooled to room temperature and the solid thus formed was filtered out. The remaining solution was subjected to extraction with ethyl acetate and water. The organic layer thus formed was dehydrated. After concentration in a vacuum, column chromatography isolated <1-a> (45.4 g, yield 45.7%)

### Synthesis Example 1-2. Synthesis of <1-b>

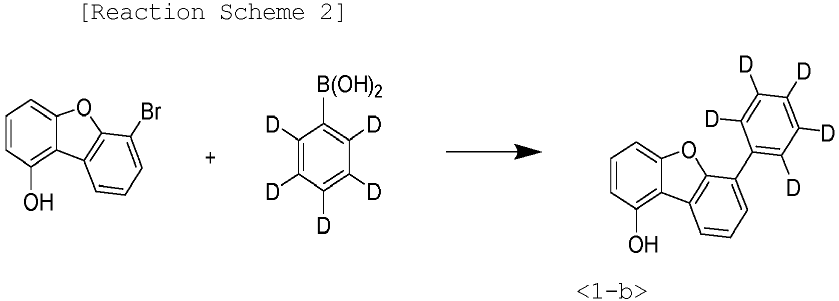

In a 500-ml round-bottom flask purged with nitrogen, 6-bromo-1-dibenzofuranol (20 g, 0.076 mol), phenylboronic acid (D5) (11.6 g, 0.091 mol), tetrakis(triphenylphosphine) palladium (Pd[PPh₃]₄) (1.8 g, 0.002 mol), potassium carbonate (17.9 g, 0.129 mol), toluene (140 ml), ethanol (35 ml), and water (65 ml) were refluxed together for 5 hours. After completion of the reaction, the reaction mixture was cooled to the room temperature and subjected to extraction with ethyl acetate and water. The organic layer thus obtained were dehydrated. After concentration in a vacuum, recrystallization in ethyl acetate and heptane afforded <1-b> (15.2 g, yield 75.4%) .

### Synthesis Example 1-3. Synthesis of <1-c>

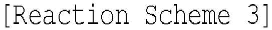

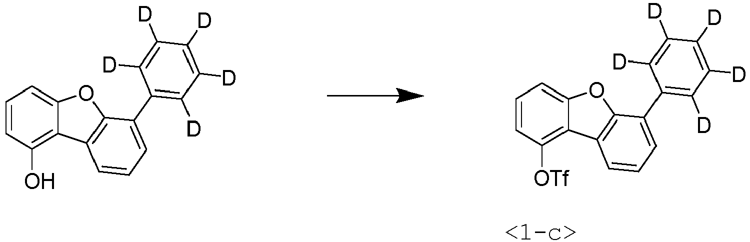

In a 500-ml round-bottom flask purged with nitrogen, <1-b> (15.2 g, 0.058 mol), pyridine (6 g, 0.076 mol), and dichloromethane (150 ml) were cooled together to 0°C or less. Then, drops of trifluoromethane sulfonic anhydride (18.1 g, 0.064 mol) were slowly added. After dropwise addition, the mixture was warmed to room temperature and stirred to conduct the reaction. After completion of the reaction, the reaction mixture was subjected to extraction with dichloromethane and water. The organic layer was dehydrated and distilled in a vacuum, followed by column chromatography to afford <1-c> (20 g, yield 87.3%) .

### Synthesis Example 1-4. Synthesis of <1-d>

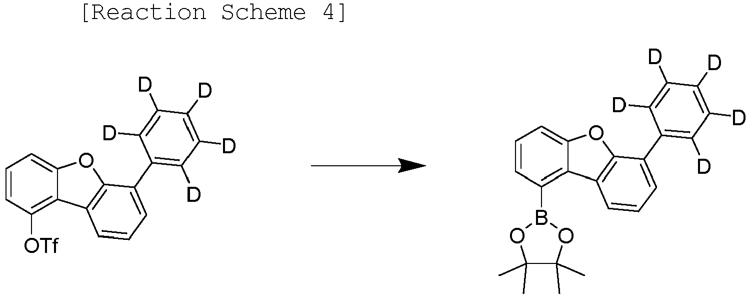

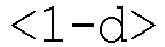

In a 300 ml round-bottom flask purged with nitrogen, <1-c> (20 g, 0.050 mol), bis(pinacolato)diboron (16.6 g, 0.065 mol), bis(diphenylphosphino)ferrocene dichloropalladium (0.8 g, 0.001 mol), calcium acetate (9.9 g, 0.101 mol), and 1,4-dioxane (200 ml) were refluxed for 12 hours. After completion of the reaction, the reaction mixture was cooled to room temperature and filtered through a silica pad topped with celite. The filtrate was concentrated and purified by column chromatography to afford <1-d> (14.8 g, yield 78.4%).

### Synthesis Example 1-5. Synthesis of [Chemical Formula 19]

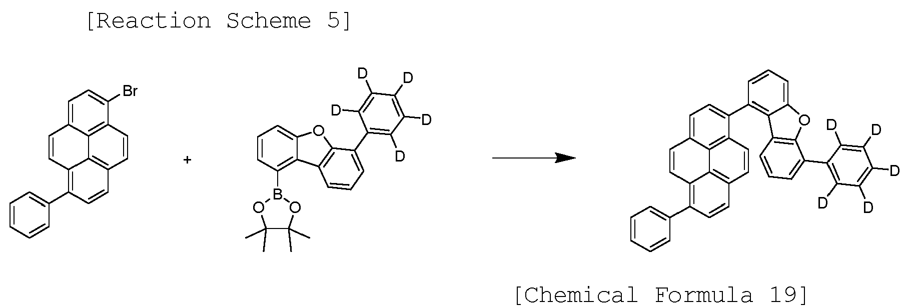

In a 300-ml round-bottom flask purged with nitrogen, <1-a> (10.7 g, 0.030 mol), <1-d> (13.7 g, 0.036 mol), tetrakis(triphenylphosphine)palladium (0.7 g, 0.001 mol), potassium carbonate (7.4 g, 0.053 mol), toluene (80 ml), ethanol (20 ml), and water (26 ml) were refluxed together for 4 hours. After completion of the reaction, the reaction mixture was cooled to room temperature and subjected to extraction with ethyl acetate and water. The organic layer was dehydrated and concentrated, followed by purification through column chromatography to afford [Chemical Formula 19] (8.4 g, yield 53.4%) .

MS(MALDI-TOF) : m/z 525.21[M]⁺

### SYNTHESIS EXAMPLE 2. Synthesis of [Chemical Formula 34]

### Synthesis Example 2-1. Synthesis of <2-a>

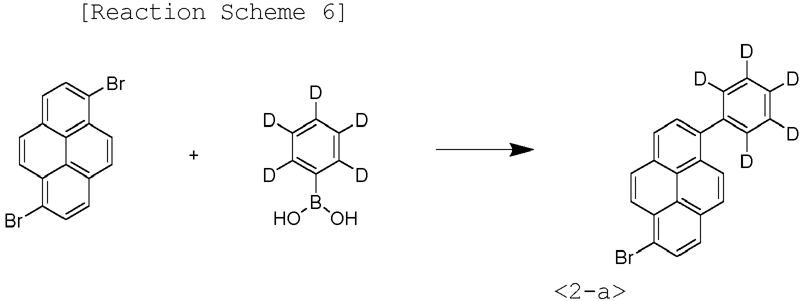

The same procedure as in Synthesis Example 1-1 was carried out, with the exception of using phenylboronic acid (D5) instead of phenylboronic acid, to afford <2-a> (yield 79.3%) .

### Synthesis Example 2-2. Synthesis of <2-b>

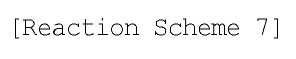

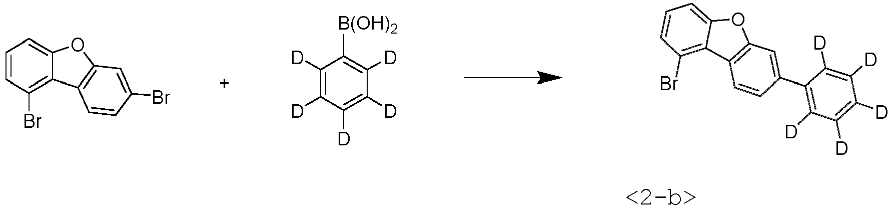

The same procedure as in Synthesis Example 1-2 was carried out, with the exception of using 1,7-dibromodibenzofuran instead of 6-bromo-1-dibenzofuranol, to afford <2-b> (yield 54%).

### Synthesis Example 2-3. Synthesis of <2-c>

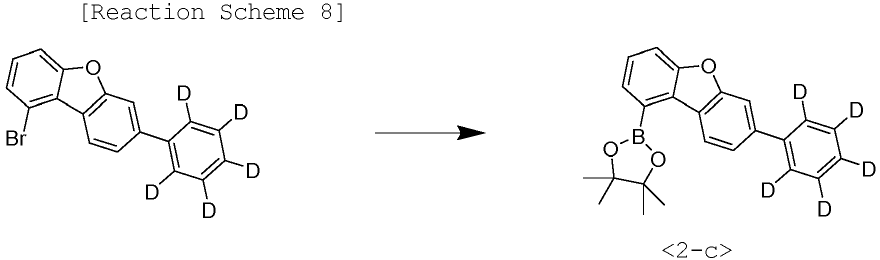

The same procedure as in Synthesis Example 1-4 was carried out, with the exception of using <2-b> instead of <1-c>, to afford <2-c> (yield 72.8%).

### Synthesis Example 2-4. Synthesis of [Chemical Formula 34]

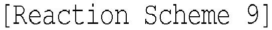

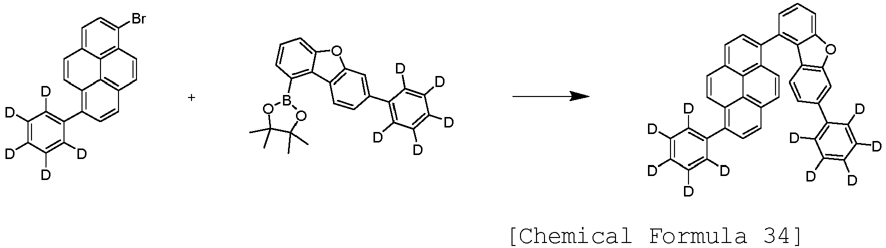

The same procedure as in Synthesis Example 1-5 was carried out, with the exception of using <2-a> and <2-c> instead of <1-a> and <1-d>, respectively, to afford [Chemical Formula 34] (yield 63.7%).

MS(MALDI-TOF) : m/z 530.25[M]⁺

### SYNTHESIS EXAMPLE 3. Synthesis of Chemical Formula 52

### Synthesis Example 3-1. Synthesis of <3-a>

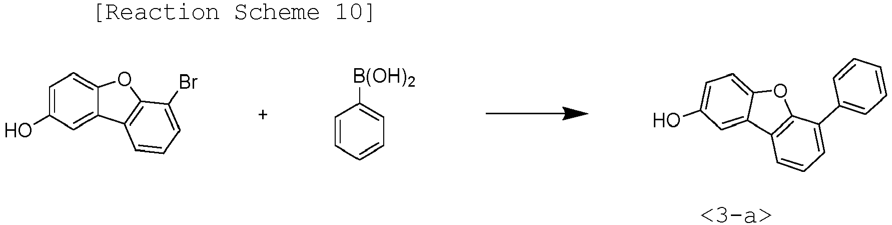

The same procedure as in Synthesis Example 1-2 was carried out, with the exception of using 6-bromo-2-dibenzofuranol and phenylboronic acid instead of 6-bromo-1-dibenzofuranol and phenylboronic acid(D5), respectively, to afford <3-a> (yield 72.0%).

### Synthesis Example 3-2. Synthesis of <3-b>

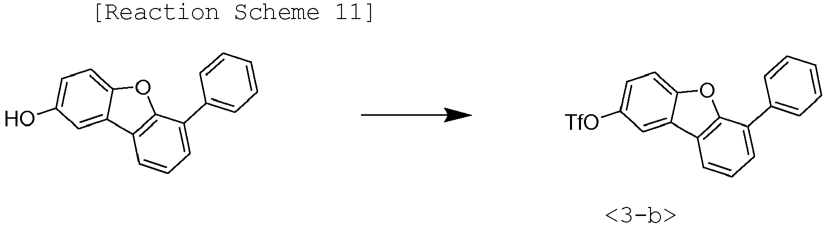

The same procedure as in Synthesis Example 1-3 was carried out, with the exception of using <3-a> instead of <1-b>, to afford <3-b> (yield 85.2%).

### Synthesis Example 3-3. Synthesis of <3-c>

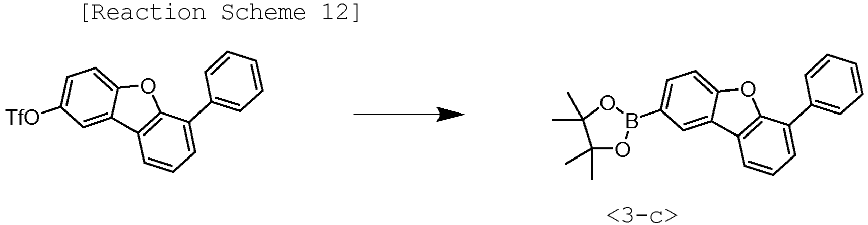

The same procedure as in Synthesis Example 1-4 was carried out, with the exception of using <3-b> instead of <1-c>, to afford <3-c> (yield 76.8%).

### Synthesis Example 3-4. Synthesis of [Chemical Formula 52]

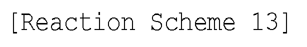

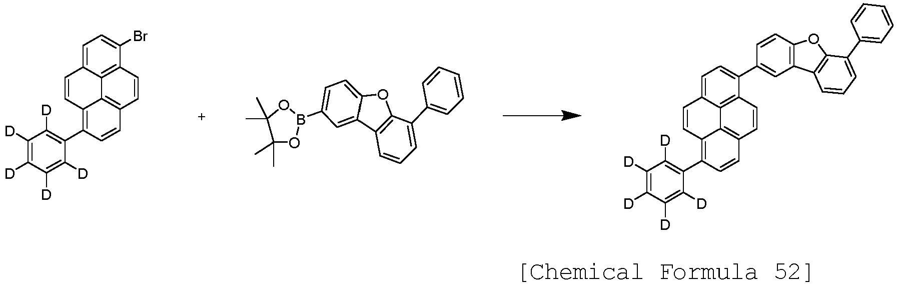

The same procedure as in Synthesis Example 2-4 was carried out, with the exception of using <3-c> instead of <2-c>, to afford [Chemical Formula 52] (yield 58.0%).

MS(MALDI-TOF) : m/z 525.21[M]⁺

### SYNTHESIS EXAMPLE 4. Synthesis of Chemical Formula 131

### Synthesis Example 4-1. Synthesis of [Chemical Formula 131]

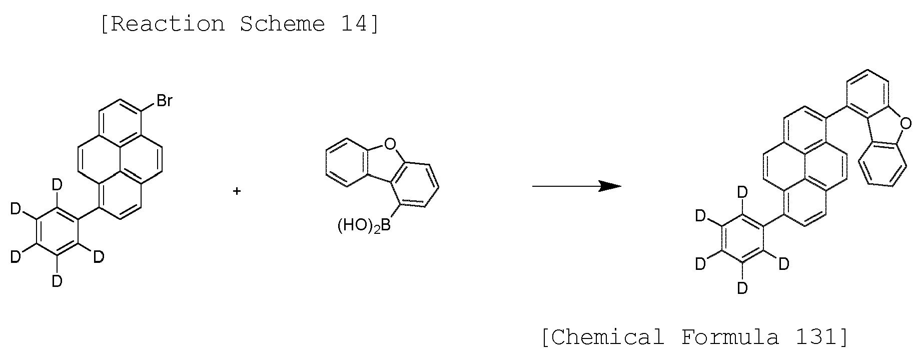

The same procedure as in Synthesis Example 2-4 was carried out, with the exception of using 1-dibenzofuran boronic acid instead of <2-c>, to afford [Chemical Formula 131] (yield 61.4%).

MS(MALDI-TOF) : m/z 449.18[M]⁺

### SYNTHESIS EXAMPLE 5. Synthesis of Chemical Formula 136

### Synthesis Example 5-1. Synthesis of <5-a>

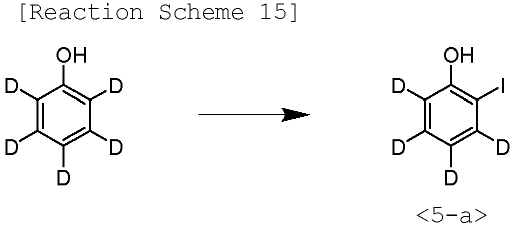

In a 1000 ml round-bottom flask purged with nitrogen, a 30wt% aqueous solution of hydrogen peroxide (50 ml, 0.477 mol), phenol (D5) (45 g, 0.454 mol), iodine (57.6 g, 0.227 mol), and water (450 ml) were stirred together at 50°C for 24 hours. After completion of the reaction, an aqueous sodium thiosulfate solution was added. The reaction mixture was subjected to extraction with ethyl acetate and water. The organic layer was dehydrated and concentrated in a vacuum. Purification by column chromatography afforded <5-a> (45 g, yield 44.3%) .

### Synthesis Example 5-2. Synthesis of <5-b>

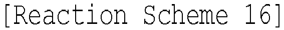

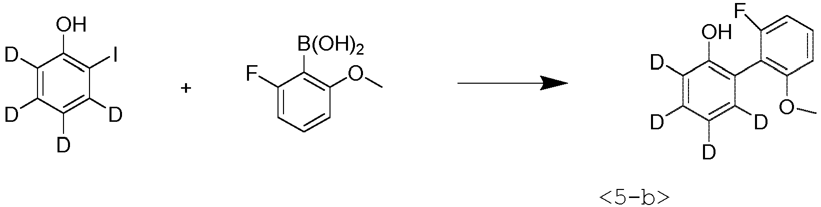

In a 1000-ml round-bottom flask purged with nitrogen, <5-a> (45 g, 0.201 mol), 2-fluoro-6-methoxyphenylboronic acid (41 g, 0.241 mol), tetrakis(triphenylphosphine)palladium (7 g, 0.006 mol), potassium carbonate (47.2 g, 0.341 mol), toluene (315 ml), ethanol (80 ml), and water (170 ml) were refluxed for 8 hours. After completion of the reaction, the reaction mixture was cooled to room temperature and subjected to extraction with ethyl acetate and water. The organic layer thus formed was dehydrated and concentrated in a vacuum, followed by purification through column chromatography to afford <5-b> (28.6 g, yield 64.1%).

### Synthesis Example 5-3. Synthesis of <5-c>

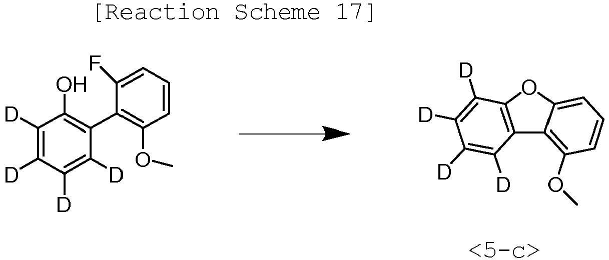

In a 500-ml round-bottom flask purged with nitrogen, <5-b> (28.6 g, 0.129 mol), potassium carbonate (44.5 g, 0.322 mol), and 1-methyl-2-pyrrolidine (143 ml) were refluxed together for 12 hours. After completion of the reaction, the reaction mixture was cooled to room temperature and slowly added with 2 N HCl (200 ml) while being sufficiently stirred. After extraction with ethyl acetate and water, the organic layer was concentrated and purified by column chromatography to afford <5-c> (21 g, yield 80.7%).

### Synthesis Example 5-4. Synthesis of <5-d>

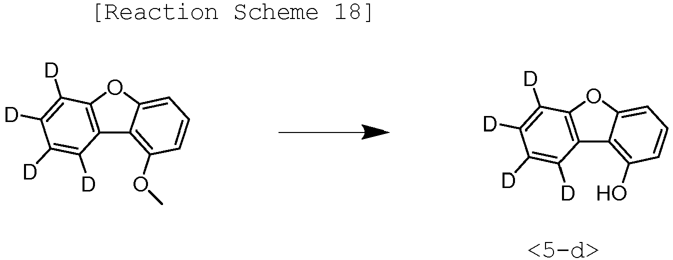

In a 500-ml round-bottom flask purged with nitrogen, <5-c> (21 g, 0.104 mol) and dichloromethane (120 ml) were cooled together to 0°C or less. Drops of boron tribromide (52 g, 0.208 mol) were slowly added, with attention paid to the temperature change. The mixture was heated to room temperature and stirred to conduct the reaction. After completion of the reaction, water (100 ml) was dropwise added to the reaction mixture and sufficiently stirred. The reaction mixture was subjected to extraction with dichloromethane and water, and the organic layer thus formed was dehydrated and concentrated in a vacuum, followed by purification through column chromatography to afford <5-d> (15 g, yield 76.8%).

### Synthesis Example 5-5. Synthesis of <5-e>

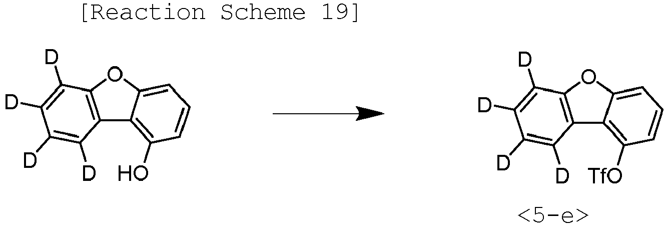

In a 500-ml round-bottom flask purged with nitrogen, <5-d> (15.0 g, 0.080 mol), pyridine (8.2 g, 0.104 mol), and dichloromethane (150 ml) were cooled together to 0°C or less. Thereafter, trifluoromethane sulfonic anhydride (24.7 g, 0.088 mol) was slowly added in a dropwise manner. The mixture was heated to room temperature and stirred to conduct the reaction. After completion of the reaction, the reaction mixture was subjected to extraction with dichloromethane and water. The organic layer thus formed was dehydrated and concentrated in a vacuum, followed by purification through column chromatography to afford <5-e> (20 g, yield 78.4%).

### Synthesis Example 5-6. Synthesis of <5-f>

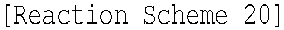

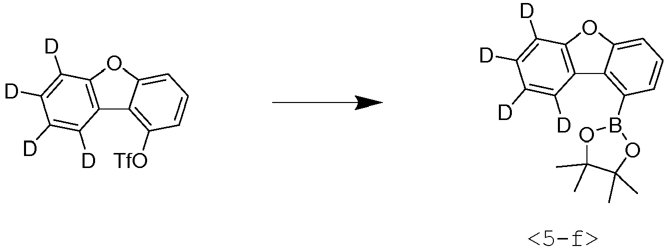

In a 500-ml round-bottom flask purged with nitrogen, <5-e> (20 g, 0.062 mol), bis(pinacolato)diboron (23.8 g, 0.094 mol), bis(diphenylphosphino)ferrocene dichloropalladium (2.5 g, 0.003 mol), calcium acetate (9.5 g, 0.125 mol), and 1,4-dioxane (200 ml) were refluxed for 12 hours. After completion of the reaction, the reaction mixture was cooled to room temperature and filtered through a silica pad topped with celite. The filtrate was concentrated and purified by column chromatography to afford <5-f> (15 g, yield 80.6%).

### Synthesis Example 5-7. Synthesis of [Chemical Formula 136]

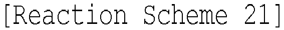

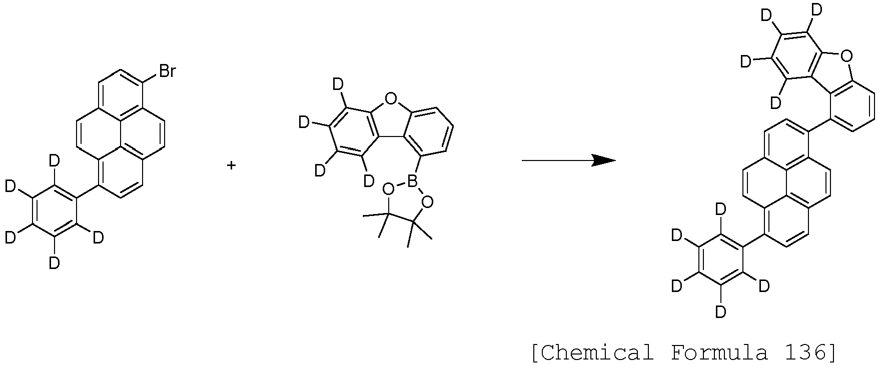

The same procedure as in Synthesis Example 2-4 was carried out, with the exception of using <5-f> instead of <2-c>, to afford [Chemical Formula 136] (yield 60.3%).

MS(MALDI-TOF) : m/z 453.21[M]⁺

### SYNTHESIS EXAMPLE 6. Synthesis of Chemical Formula 1

### Synthesis Example 6-1. Synthesis of <6-a>

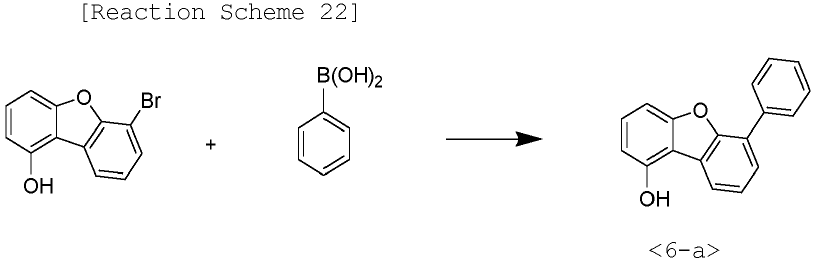

The same procedure as in Synthesis Example 1-2 was carried out, with the exception of using phenylboronic acid instead of phenylboronic acid (D5), to afford <6-a> (yield57%).

### Synthesis Example 6-2. Synthesis of <6-b>

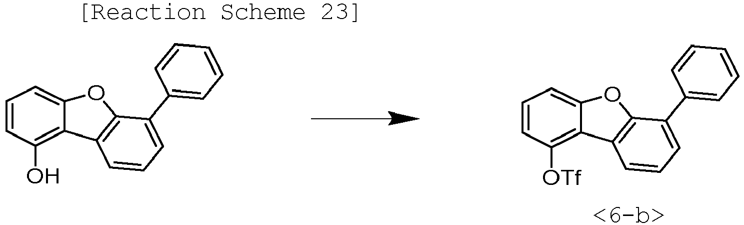

The same procedure as in Synthesis Example 1-3 was carried out, with the exception of using <6-a> instead of <1-b>, to afford <6-b> (yield 86.8%).

### Synthesis Example 6-3. Synthesis of <6-c>

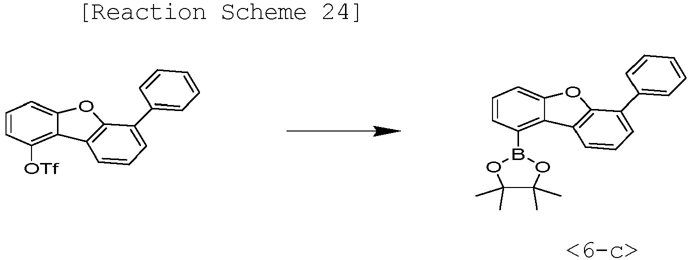

The same procedure as in Synthesis Example 1-4 was carried out, with the exception of using <6-b> instead of <1-c>, to afford <6-c> (yield 79.2%).

### Synthesis Example 6-4. Synthesis of [Chemical Formula 1]

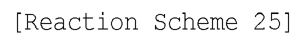

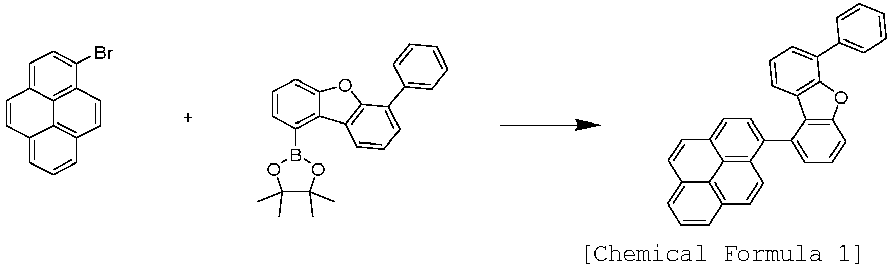

The same procedure as in Synthesis Example 1-5 was carried out, with the exception of using 1-bromopyrene and <6-c> instead of <1-a> and <1-d>, respectively, to afford [Chemical Formula 1] (yield 52.5%).

MS(MALDI-TOF) : m/z 444.15[M]⁺

### SYNTHESIS EXAMPLE 7. Synthesis of Chemical Formula 23

### Synthesis Example 7-1. Synthesis of [Chemical Formula 23]

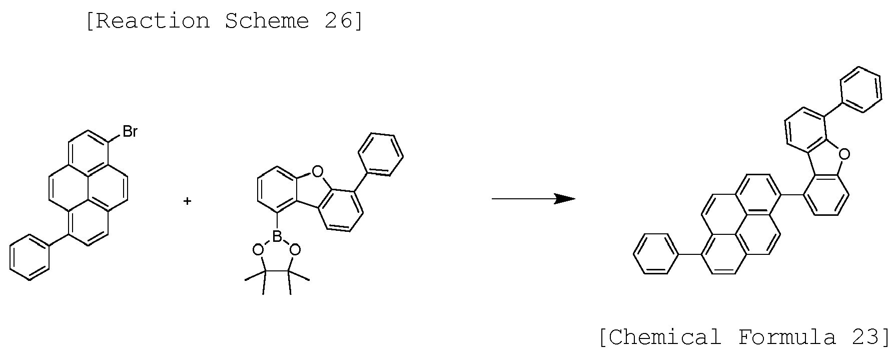

The same procedure as in Synthesis Example 1-5 was carried out, with the exception of using <6-c> instead of <1-d>, to afford [Chemical Formula 23] (yield 53.8%).

MS(MALDI-TOF) : m/z 520.18[M]⁺

### SYNTHESIS EXAMPLE 8. Synthesis of Chemical Formula 41

### Synthesis Example 8-1. Synthesis of [Chemical Formula 41]

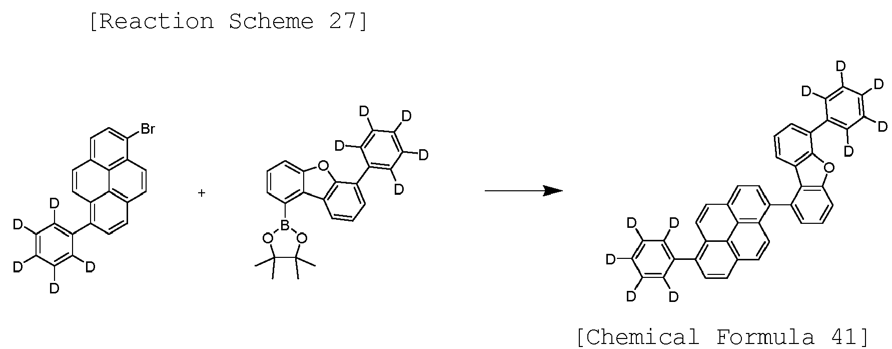

The same procedure as in Synthesis Example 1-5 was carried out, with the exception of using <2-a> instead of <1-a>, to afford [Chemical Formula 41] (yield 54.2%).

MS(MALDI-TOF) : m/z 530.25[M]⁺

### SYNTHESIS EXAMPLE 9. Synthesis of Chemical Formula 57

### Synthesis Example 9-1. Synthesis of [Chemical Formula 57]

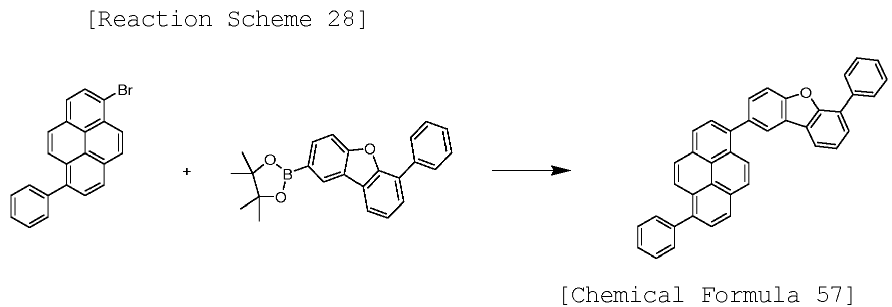

The same procedure as in Synthesis Example 1-5 was carried out, with the exception of using <3-c> instead of <1-d>, to afford [Chemical Formula 57] (yield 53.5 %).

MS(MALDI-TOF) : m/z 520.18[M]⁺

### EXAMPLES 1 TO 13: Fabrication of Organic Light-Emitting Diodes

An ITO glass substrate was patterned to have a translucent area of 2 mm×2 mm and cleansed. The ITO glass was mounted in a vacuum chamber that was then set to have a base pressure of 1×10⁻⁷ torr. On the ITO glass substrate, films were sequentially formed of DNTPD (700 Å) and α-NPD (300 Å). Subsequently, a light-emitting layer (300 Å) was formed of a combination of the host according to the present disclosure and the dopant (BD) (3 wt %) described below. Then, [Chemical Formula E-1] and [Chemical Formula E-2] were deposited at a weight ratio of 1:1 to form an electron transport layer (300 Å) on which an electron injection layer of [Chemical Formula E-1] (10 Å) was formed and then covered with an Al layer (1,000 Å) to fabricate an organic light-emitting diode. The organic light-emitting diodes thus obtained were measured at 0.4 mA for luminescence properties.

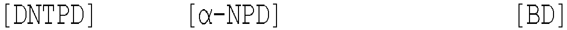

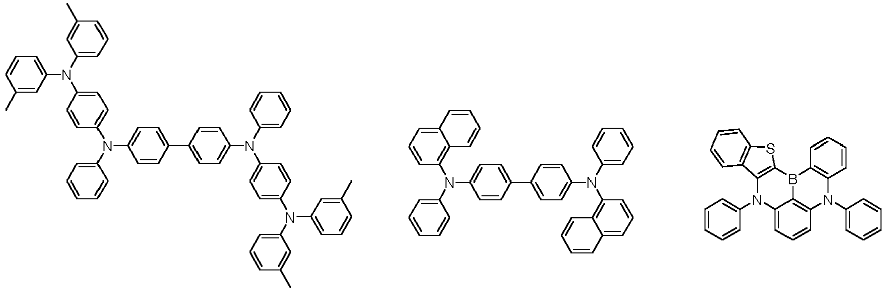

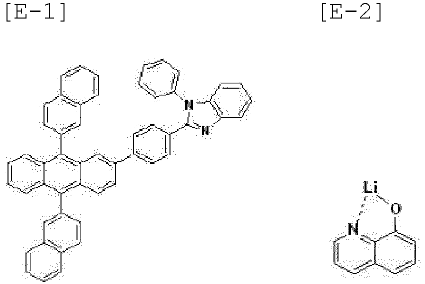

### COMPARATIVE EXAMPLES 1 TO 4

Organic light emitting diodes were fabricated in the same manner as in the Example, with the exception of using [BH1] and [BH2] as hosts instead of the compounds according to the present disclosure. The luminescence of the organic light-emitting diodes thus obtained was measured at 0.4 mA. Structures of compounds [BH1] and [BH2] are as follows:

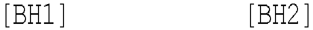

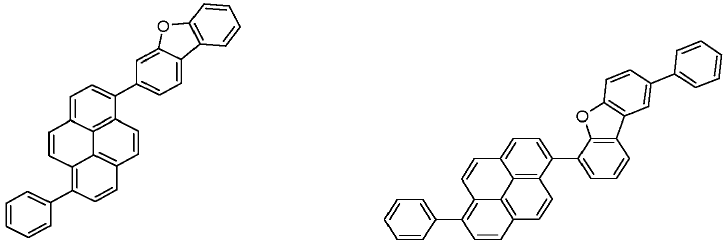

**TABLE 1 Luminous Efficiency**

| | Host | V | cd/A | CIEx | CIEy |
|---|---|---|---|---|---|
| Ex. 1 | Chemical Formula 1 | 3.9 | 8.78 | 0.134 | 0.102 |
| Ex. 2 | Chemical Formula 19 | 3.7 | 9.18 | 0.134 | 0.101 |
| Ex. 3 | Chemical Formula 23 | 3.8 | 8.90 | 0.134 | 0.101 |
| Ex. 4 | Chemical Formula 34 | 3.6 | 9.33 | 0.133 | 0.102 |
| Ex. 5 | Chemical Formula 41 | 3.6 | 9.40 | 0.133 | 0.101 |
| Ex. 6 | Chemical Formula 52 | 3.8 | 9.08 | 0.134 | 0.101 |
| Ex. 7 | Chemical Formula 57 | 3.8 | 8.86 | 0.134 | 0.102 |
| Ex. 8 | Chemical Formula 131 | 3.9 | 8.92 | 0.133 | 0.101 |
| Ex. 9 | Chemical Formula 136 | 3.8 | 8.97 | 0.133 | 0.102 |
| C. Ex. 1 | [BH1] | 4.1 | 8.67 | 0.133 | 0.103 |
| C. Ex. 2 | [BH2] | 4.0 | 8.52 | 0.133 | 0.102 |

**TABLE 2 Longevity**

| | Host | LT 97 | CIEx | CIEy |
|---|---|---|---|---|
| Ex. 10 | Chemical Formula 41 | 210 | 0.133 | 0.101 |
| Ex. 11 | Chemical Formula 50 | 181 | 0.134 | 0.101 |
| Ex. 12 | Chemical Formula 35 | 194 | 0.133 | 0.101 |
| Ex. 13 | Chemical Formula 136 | 270 | 0.134 | 0.102 |
| C. Ex. 3 | [BH1] | 143 | 0.133 | 0.103 |
| C. Ex. 4 | [BH2] | 148 | 0.133 | 0.102 |

As is understood from data of Table 1, the organic light-emitting diodes employing in the light-emitting layer the compounds in which a pyrene group is bonded to the position 1 or 2 of the dibenzofuran moiety according to the present disclosure can drive at lower voltages with higher luminous efficiency, compared to those of Comparative Examples 1 and 2 that employ in the light-emitting layer the compound BH1 or BH2 in which a pyrene group is bonded to the position 3 or 4 of the dibenzofuran moiety. In addition, data of Table 2 show higher longevity in the organic light-emitting diodes employing in the light-emitting layer the compounds in which a pyrene group is bonded to the position 1 or 2 of the dibenzofuran moiety according to the present disclosure, compared to those of Comparative Examples 1 and 2 that employ in the light-emitting layer the compound BH1 or BH2 in which a pyrene group is bonded to the position 3 or 4 of the dibenzofuran moiety. Thus, the organic light-emitting diodes according to the present disclosure is highly available in the organic light-emitting diode field.

### Industrial Applicability

Compared to conventional compounds, the compounds of the present disclosure allow organic light-emitting diodes to exhibit superiority in terms of luminous efficiency, driving voltage, and longevity, thus finding applicability in the organic light-emitting diode field and related industrial fields.

## Claims

1. A compound, represented by the following Chemical Formula A or Chemical Formula B: 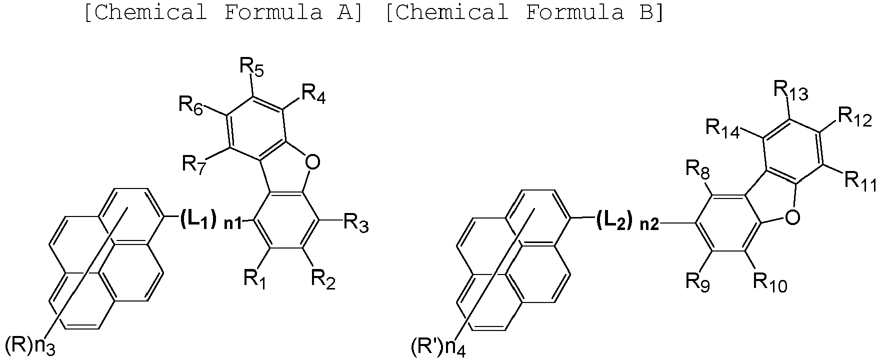 wherein,
R₁ to R₁₄, which are same or different, are each independently at least one selected from a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heterocycloalkyl of 2 to 30 carbon atoms, a substituted or unsubstituted heteroaryl of 2 to 50 carbon atoms, a substituted or unsubstituted alkylamine of 1 to 30 carbon atoms, a substituted or unsubstituted arylamine of 6 to 30 carbon atoms, a substituted or unsubstituted alkylsilyl of 1 to 30 carbon atoms, a substituted or unsubstituted arylsilyl of 6 to 30 carbon atoms, a cyano, a nitro, and a halogen;
linkers L₁ and L₂, which are same or different, are each independently selected from a single bond, a substituted or unsubstituted arylene of 6 to 20 carbon atoms, and a substituted or unsubstituted heteroarylene of 2 to 20 carbon atoms;
n1 and n2, which are same or different, are each independently an integer of 0 to 2 wherein when n1 or n2 is 2, the corresponding linkers L₁'s or L₂'s are same or different,
R and R', which are same or different, are each independently one selected from a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heterocycloalkyl of 2 to 30 carbon atoms, a substituted or unsubstituted heteroaryl of 2 to 50 carbon atoms, a substituted or unsubstituted alkylamine of 1 to 30 carbon atoms, a substituted or unsubstituted arylamine of 6 to 30 carbon atoms, a substituted or unsubstituted alkylsilyl of 1 to 30 carbon atoms, a substituted or unsubstituted arylsilyl of 6 to 30 carbon atoms, a cyano, a nitro, and a halogen;
n3 and n4, which are same or different, are each independently an integer of 1 to 9 where when n3 or n4 are 2 or more, the corresponding Rs or R's are same or different.
wherein the term 'substituted' in the expression "a substituted or unsubstituted" means having at least one substituent selected from the group consisting of a deuterium atom, a cyano, a halogen, a hydroxy, a nitro, an alkyl of 1 to 24 carbon atoms, a hydrogenated alkyl of 1 to 24 carbon atoms, an alkenyl of 1 to 24 carbon atoms, an alkynyl of 1 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 2 to 24 carbon atoms, an alkoxy of 1 to 24 carbon atoms, an alkylamino of 1 to 24 carbon atoms, a diarylamino of 12 to 24 carbon atoms, a diheteroarylamino of 2 to 24 carbon atoms, an aryl(heteroaryl)amino of 7 to 24 carbon atoms, an alkylsilyl of 1 to 24 carbon atoms, an arylsilyl of 6 to 24 carbon atoms, an aryloxy of 6 to 24 carbon atoms, and an arylthionyl of 6 to 24 carbon atoms.

2. The organic compound of claim 1, wherein the compound represented by Chemical Formula A bears at least one a deuterium atom and the compound represented by Chemical Formula B bears at least one deuterium atom.

3. The organic compound of claim 2, wherein at least one of R₁ to R₇ in Chemical Formula A is a substituent bearing a deuterium atom and
at least one of R₈ to R₁₄ in Chemical Formula B is a substituent bearing a deuterium atom.

4. The organic compound of claim 2, wherein at least one R in Chemical Formula A is a substituent bearing a deuterium atom and
at least one R' in Chemical Formula B is a substituent bearing a deuterium atom.

5. The organic compound of claim 1, wherein at least one of R₁ to R₇ in Chemical Formula A is a substituted or unsubstituted aryl of 6 to 18 carbon atoms and
at least one of R₈ to R₁₄ in Chemical Formula B is a substituted or unsubstituted aryl of 6 to 18 carbon atoms.

6. The organic compound of claim 1, wherein the linkers L₁ and L₂ in Chemical Formula A and Chemical Formula B are each a single bond or any one selected from the following Structural Formulas 1 to 5: 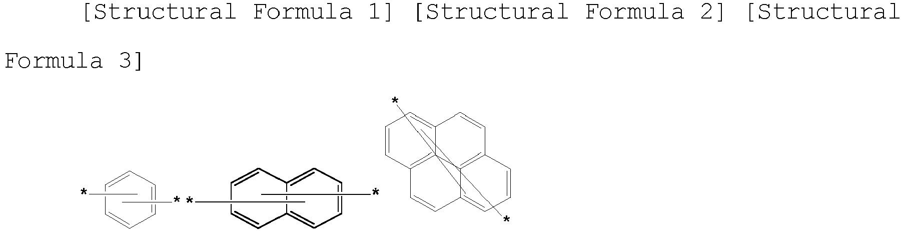 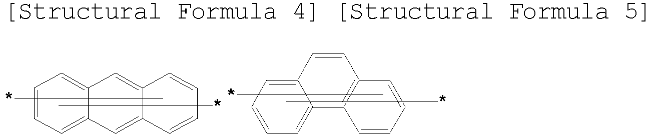 wherein each of the unsubstituted carbon atoms of the aromatic ring moiety is bound with a hydrogen atom or a deuterium atom.

7. The organic compound of claim 6, wherein the linkers L₁ and L₂ are each a single bond.

8. The organic compound of claim 1, wherein at least one R in Chemical Formula A is a substituted or unsubstituted aryl of 6 to 18 carbon atoms and at least one R' in Chemical Formula B is a substituted or unsubstituted aryl of 6 to 18 carbon atoms.

9. The organic compound of claim 8, wherein n3 and n4 in Chemical Formula A and Chemical Formula B are each 1, R in Chemical Formula A is a substituted or unsubstituted aryl of 6 to 18 carbon atoms, and R' in Chemical Formula B a substituted or unsubstituted aryl of 6 to 18 carbon atoms.

10. The organic compound of claim 1, wherein the compound represented by Chemical Formula A or Chemical Formula B is a compound represented by any one of the following Chemical Formula A-1 and Chemical Formula B-1. 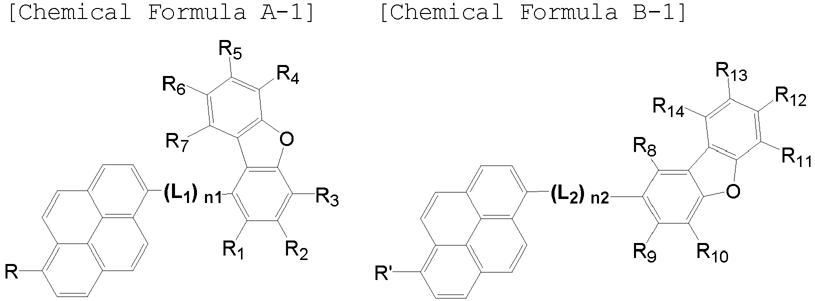
wherein, the substituents R₁ to R₁₄, the linkers L₁ and L₂, and n1 and n2 are as defined in Chemical Formula A or Chemical Formula B, and
R and R' are each a substituted or unsubstituted aryl of 6 to 18 carbon atoms.

11. The organic compound of claim 1, wherein n3 and n4 in Chemical Formula A and Chemical Formula B are each 1,
at least one of R₁ to R₇ and R in Chemical Formula A is a deuterated aryl of 6 to 18 carbon atoms, and
at least one of R₈ to R₁₄ and R' in Chemical Formula B is a deuterated aryl of 6 to 18 carbon atoms.

12. The organic compound of claim 1, wherein n3 and n4 in Chemical Formula A and Chemical Formula B are each 1, R in Chemical Formula A is a substituted or unsubstituted heteroaryl of 2 to 18 carbon atoms, and R' in Chemical Formula B is a substituted or unsubstituted heteroaryl of 2 to 18 carbon atoms.

13. The organic compound of claim 1, wherein the compound is any one selected from the following
Chemical Formulas 1 to 240:

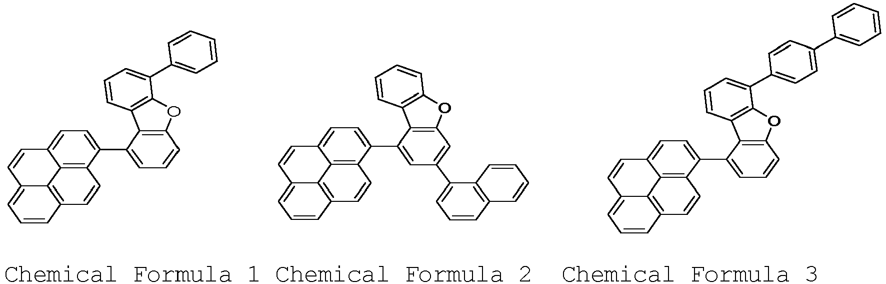

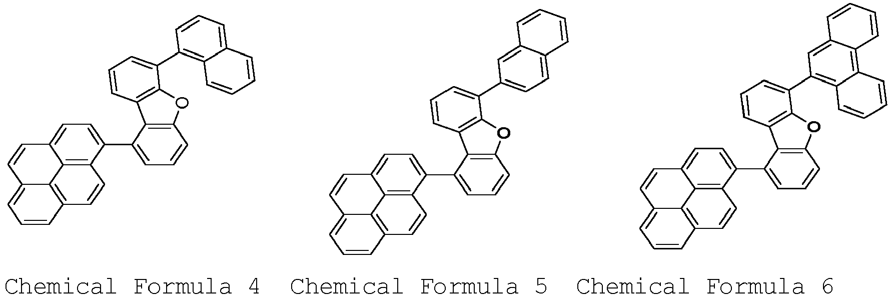

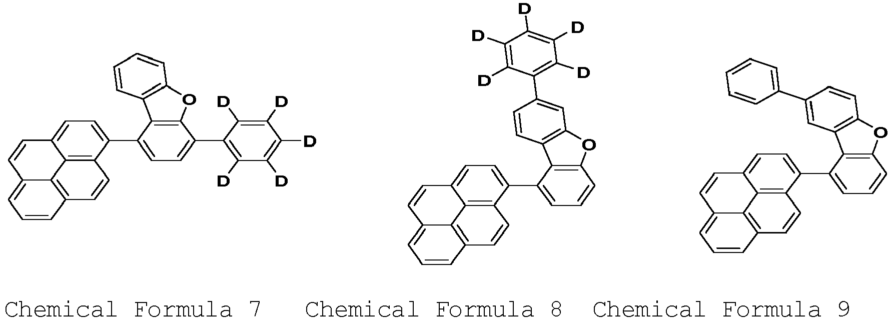

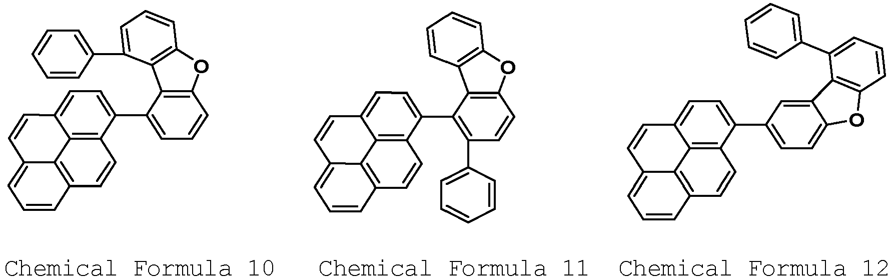

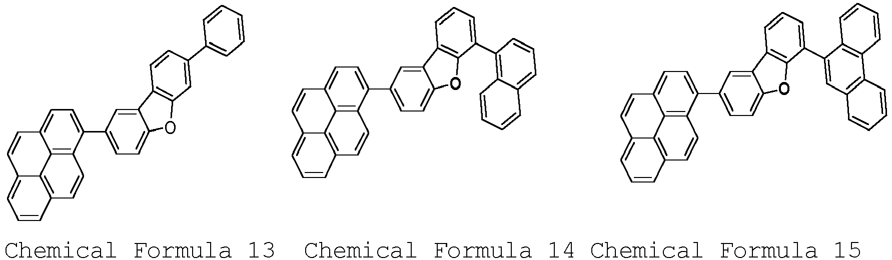

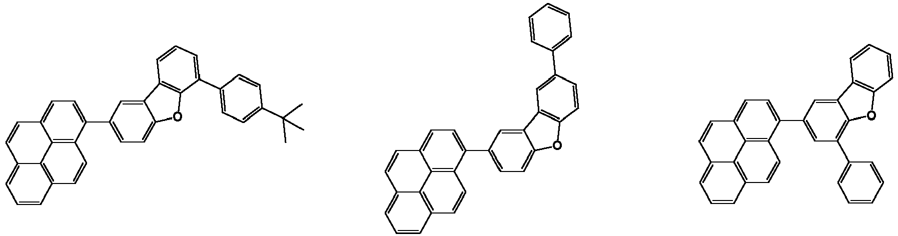

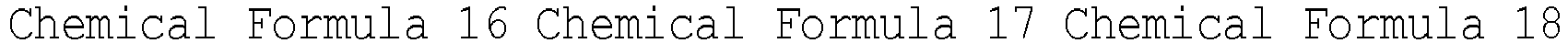

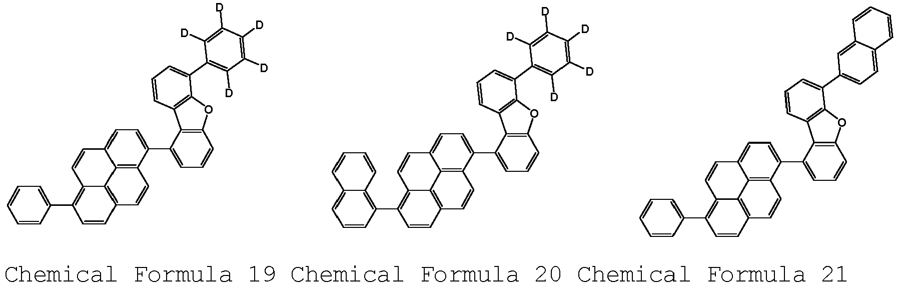

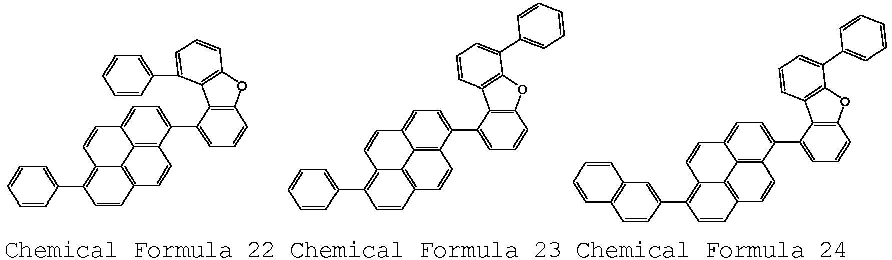

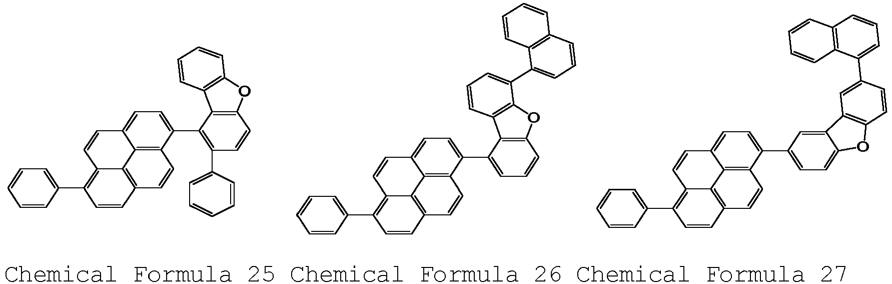

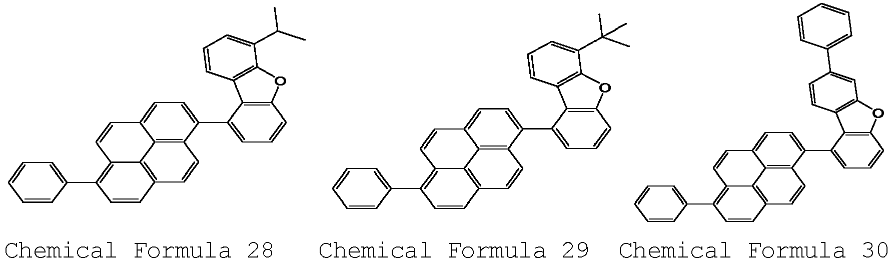

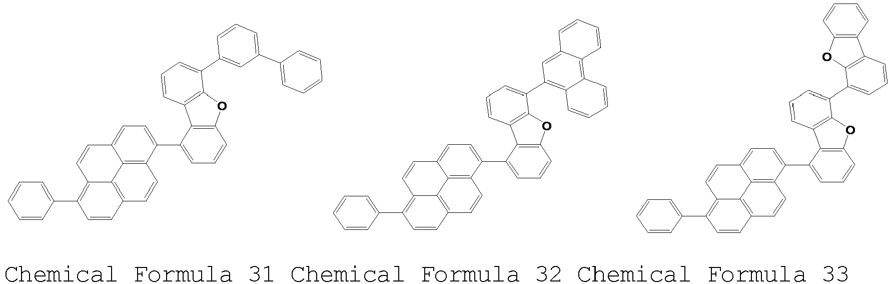

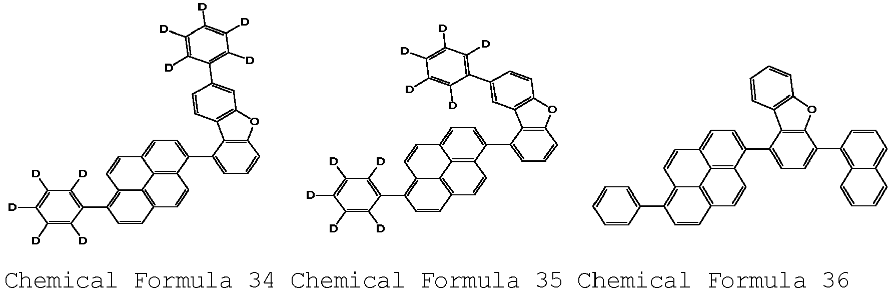

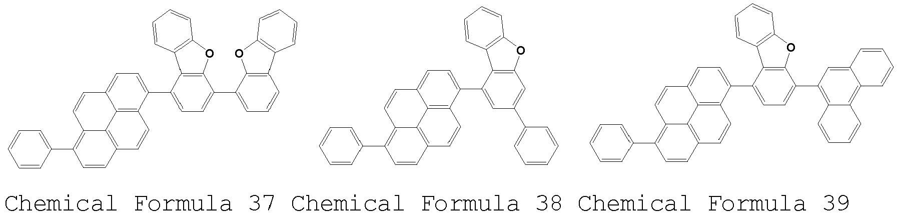

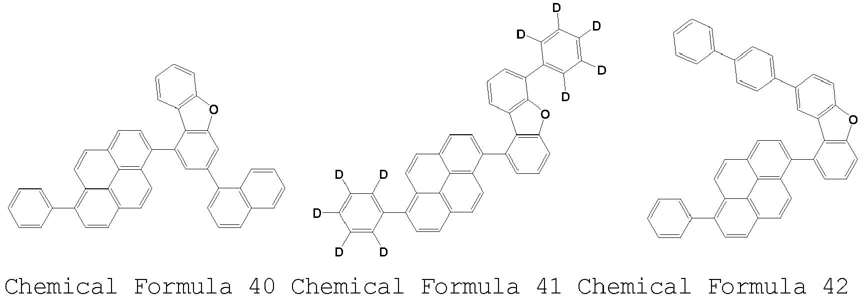

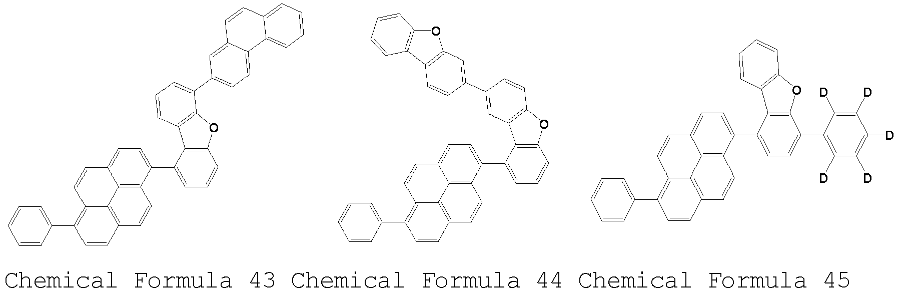

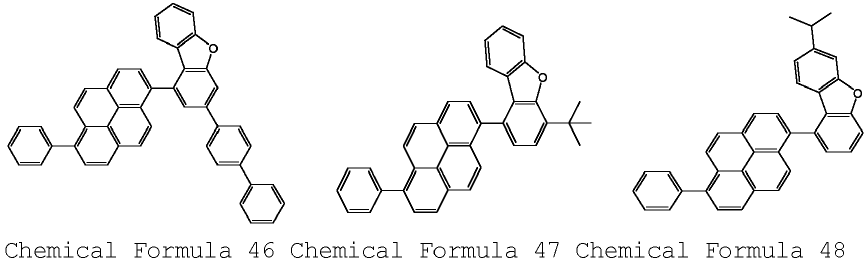

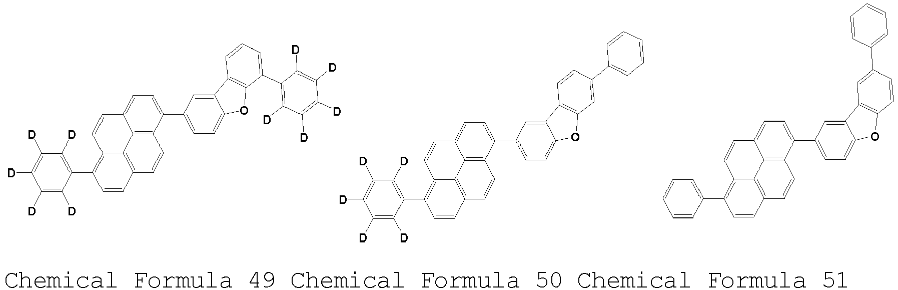

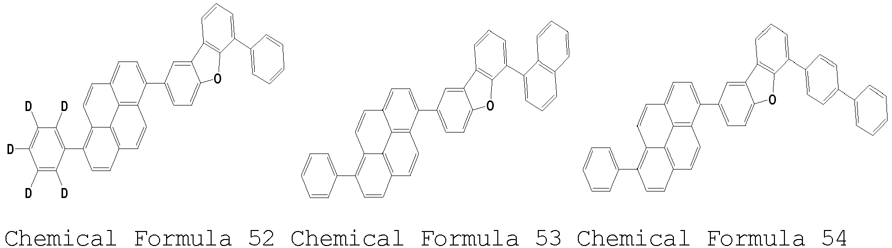

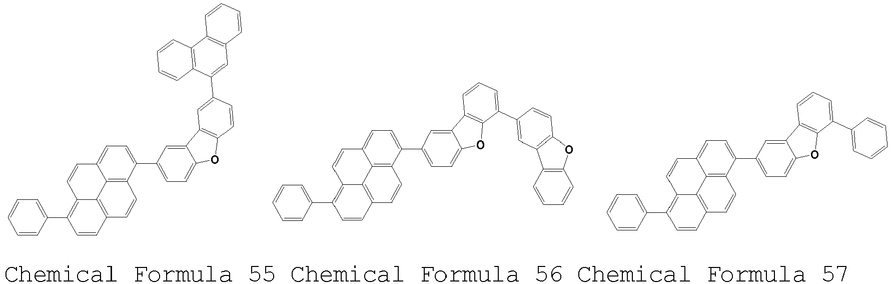

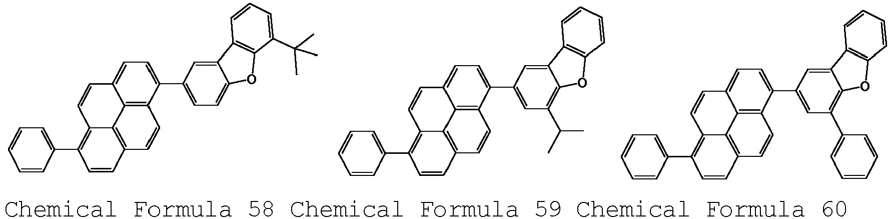

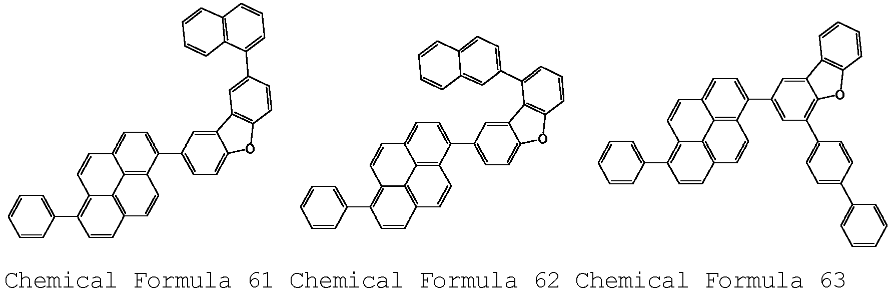

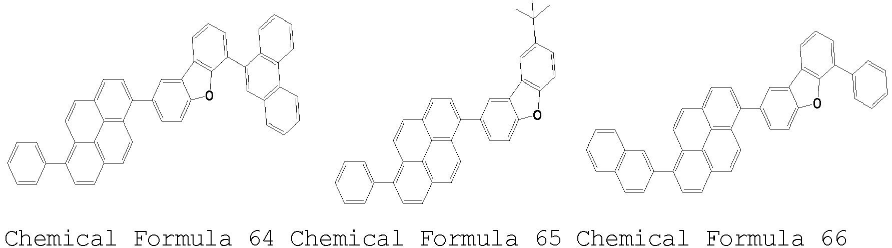

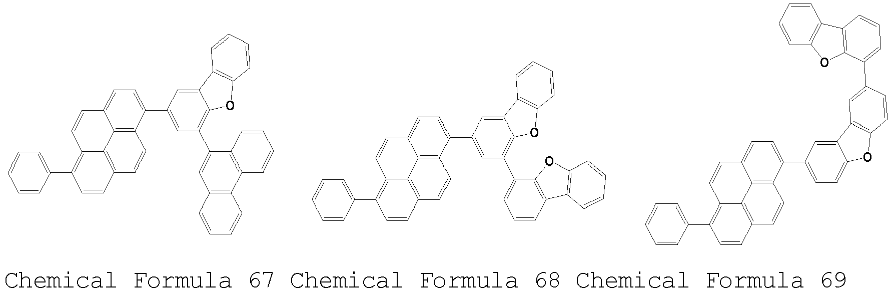

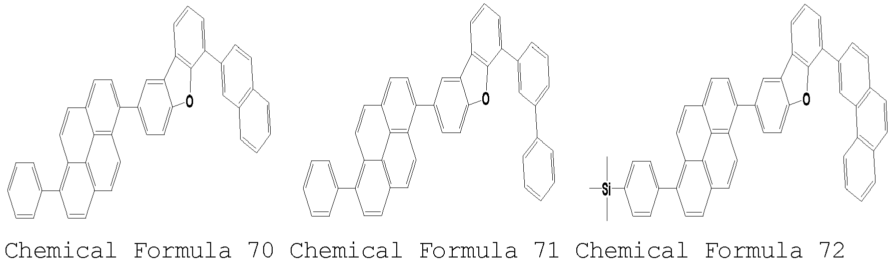

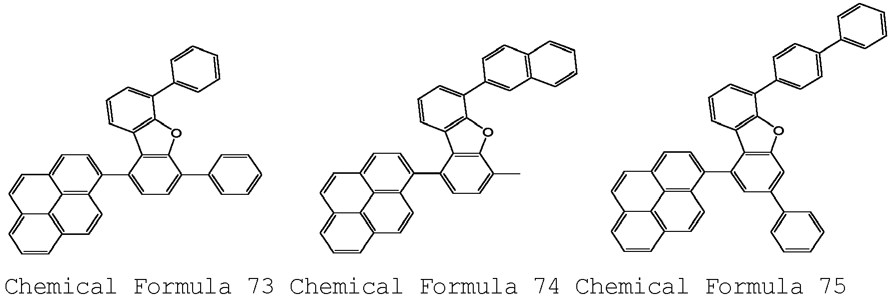

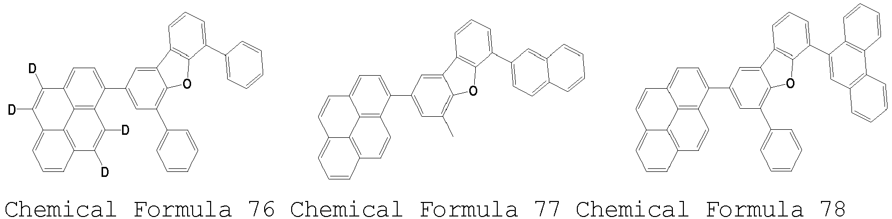

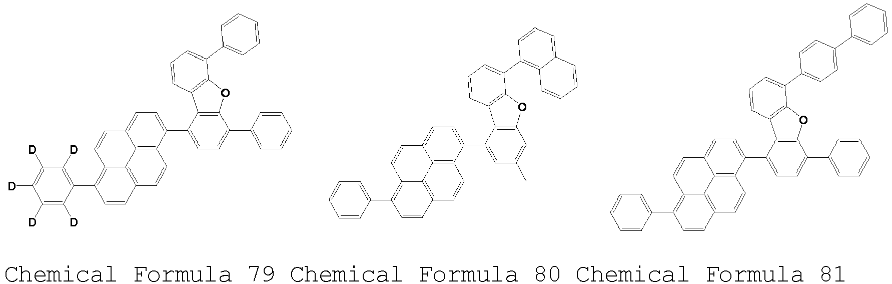

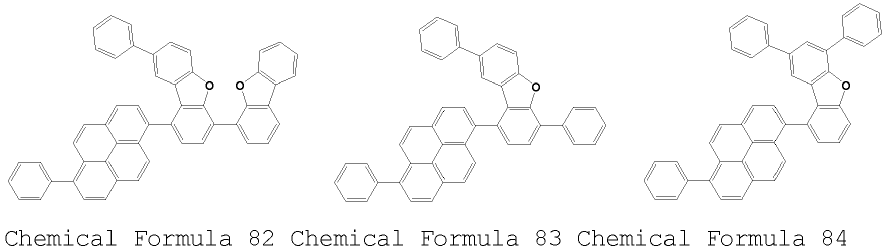

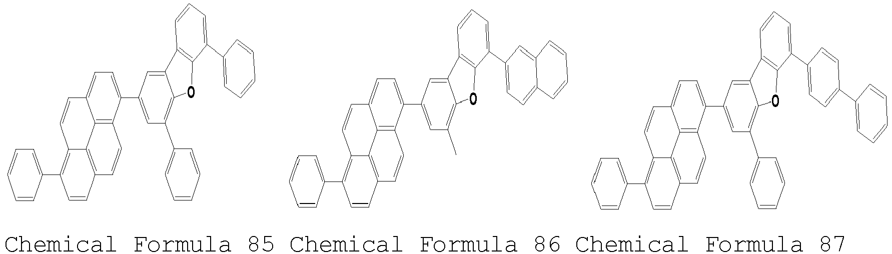

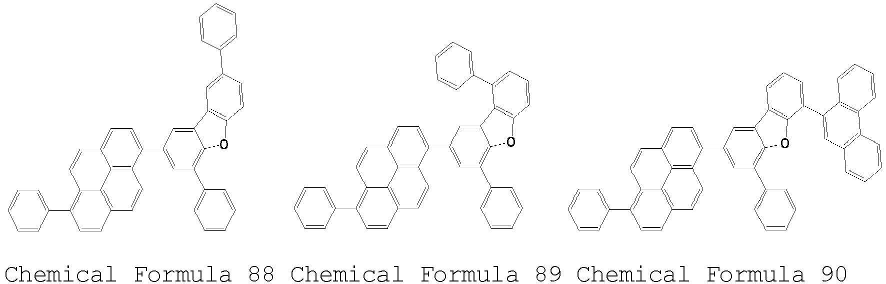

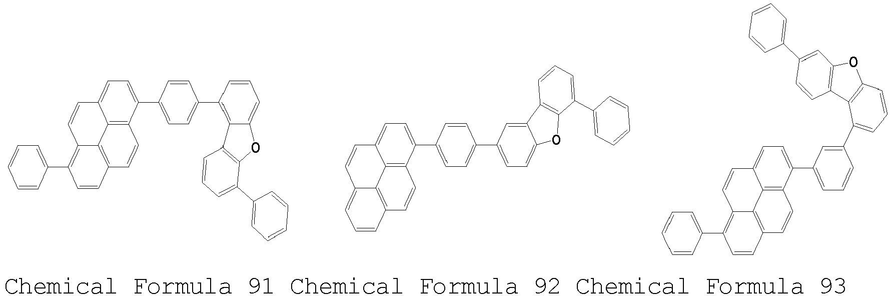

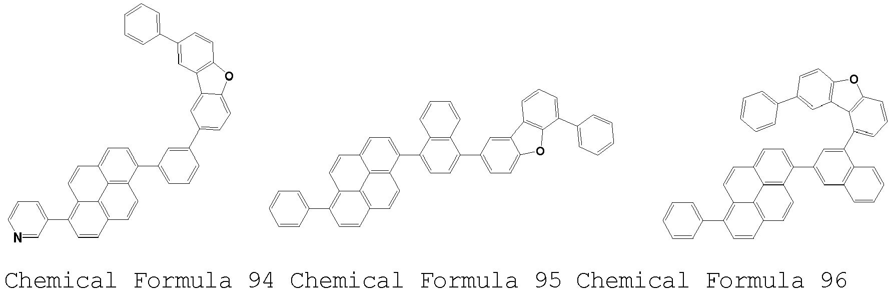

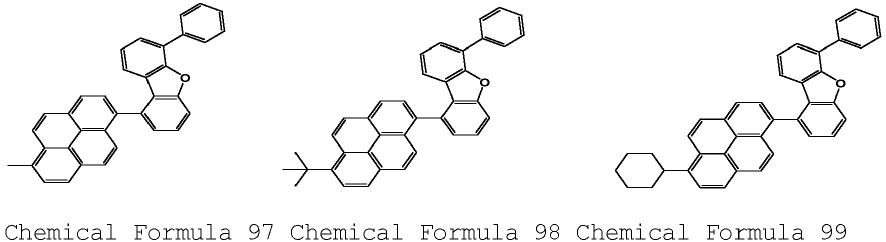

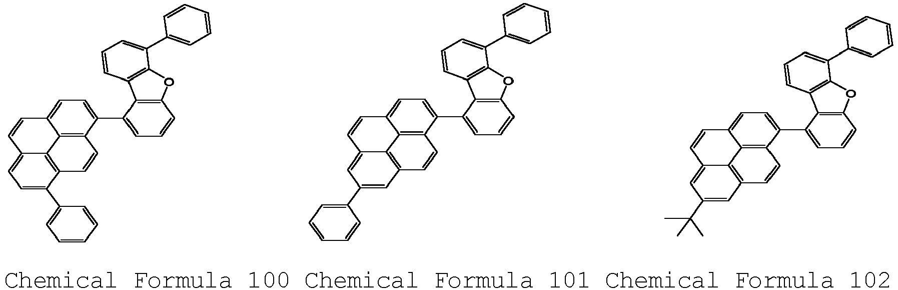

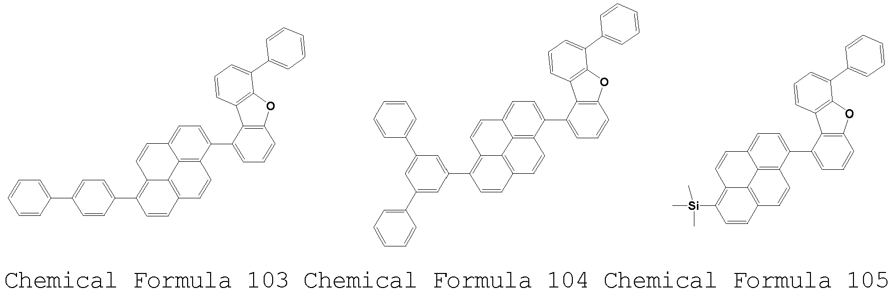

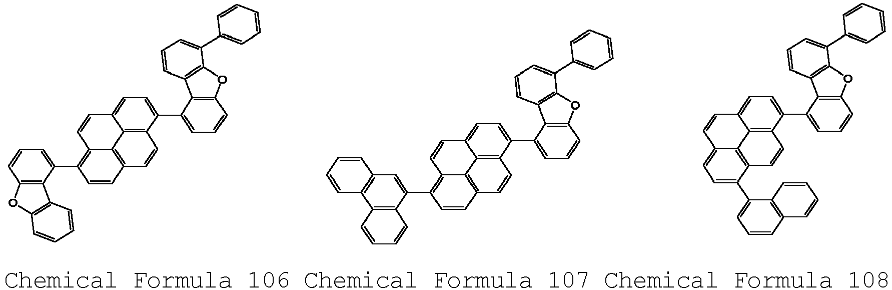

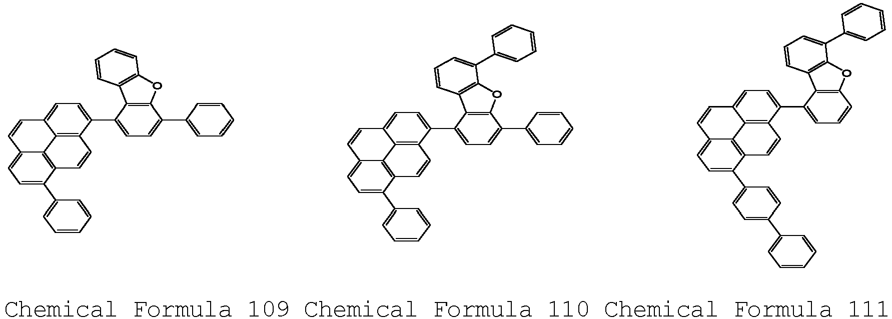

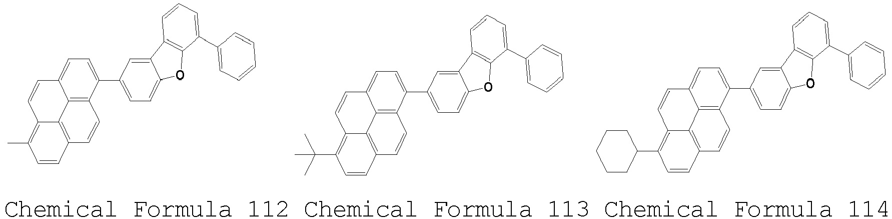

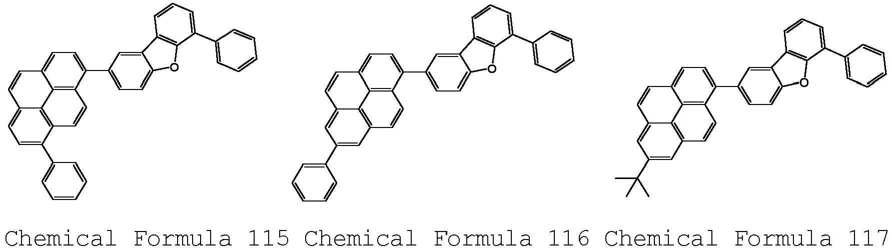

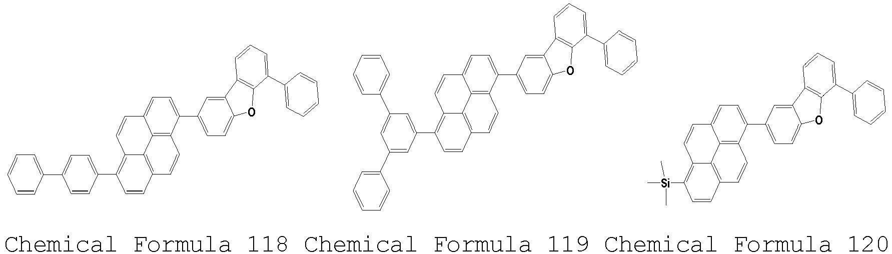

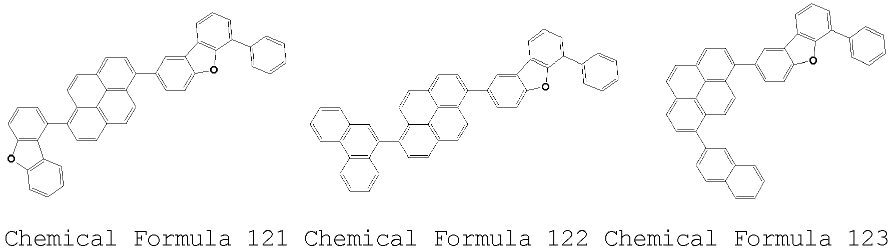

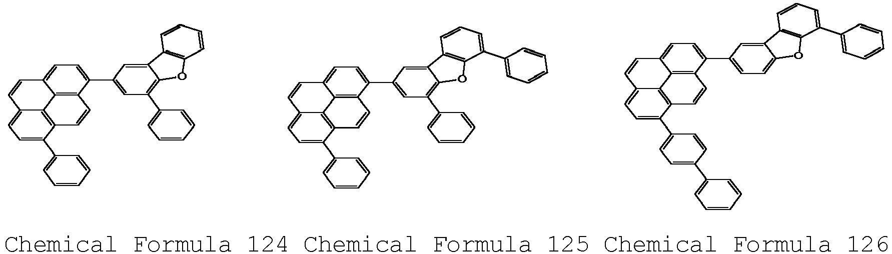

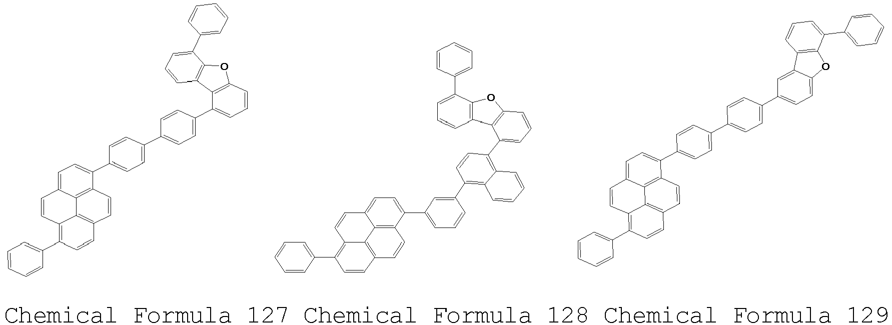

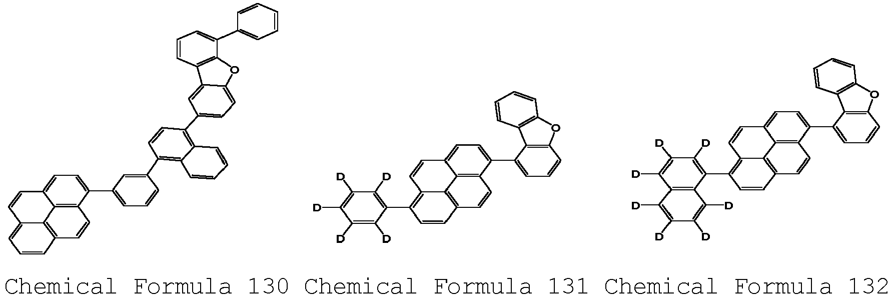

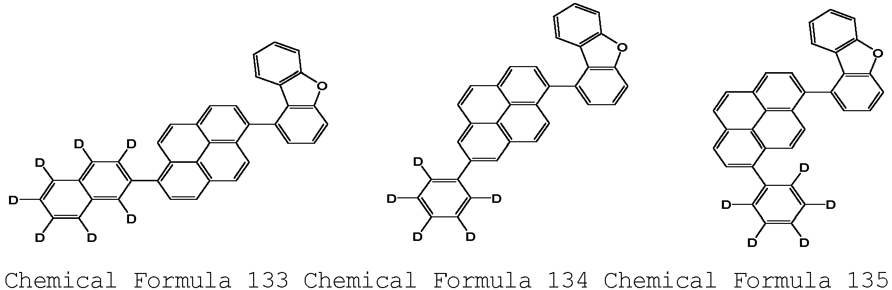

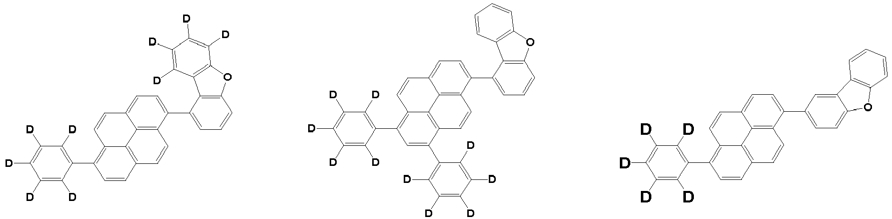

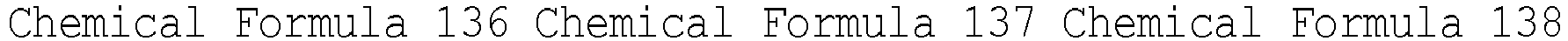

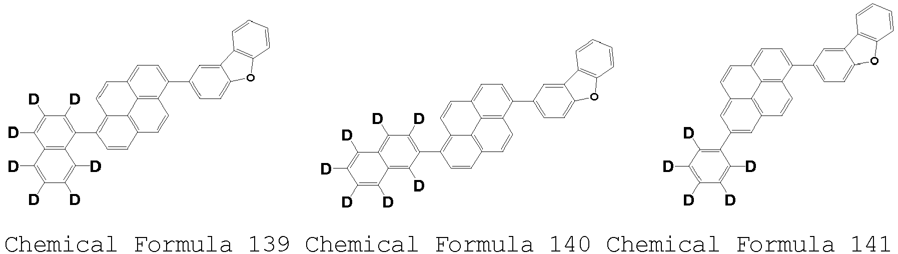

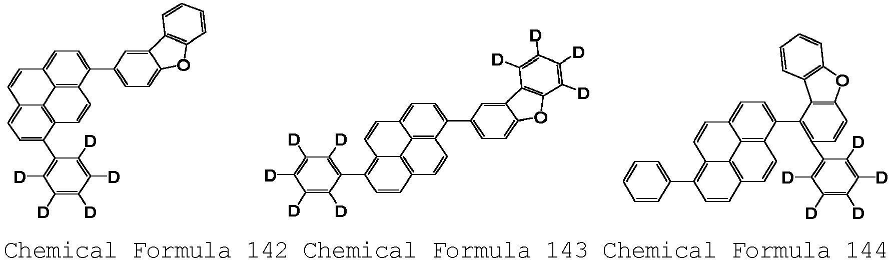

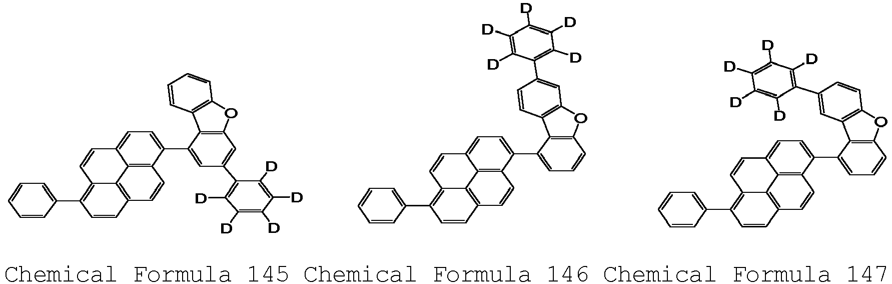

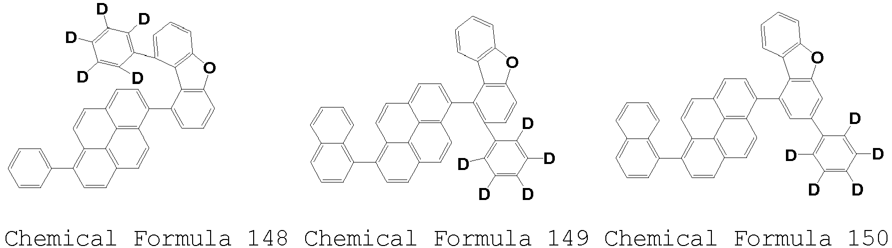

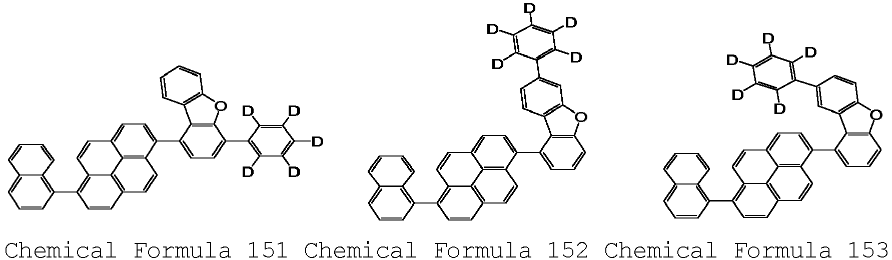

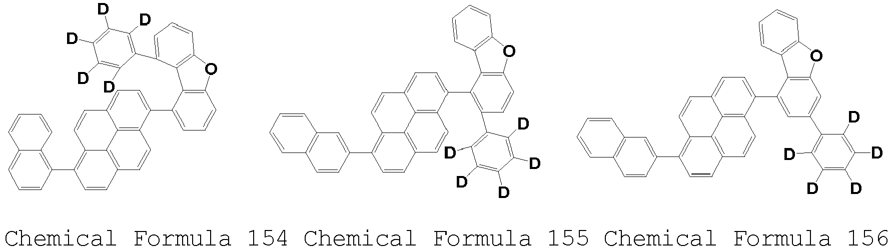

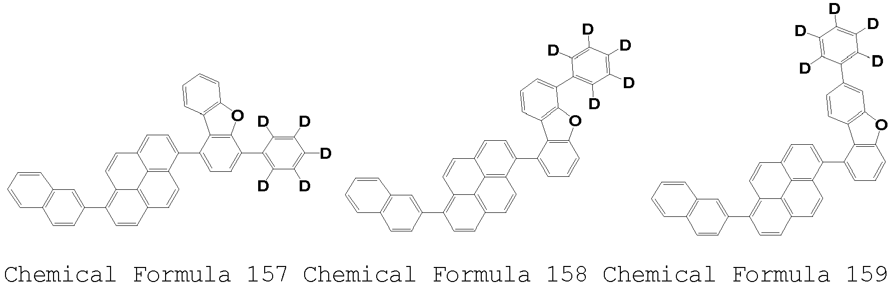

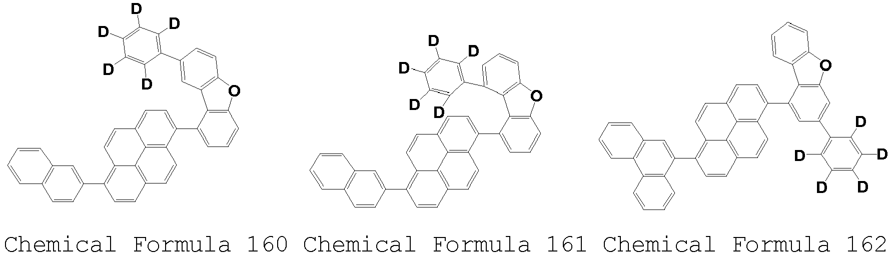

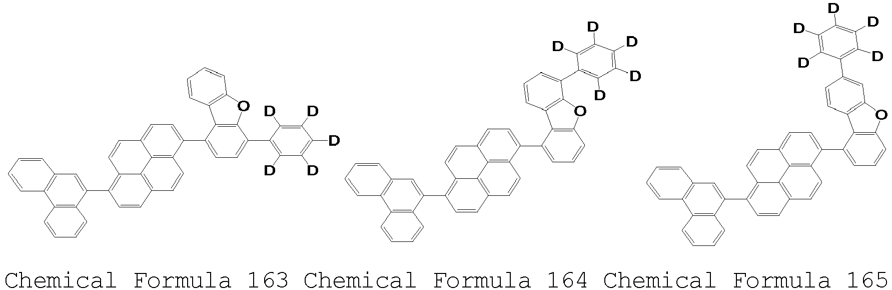

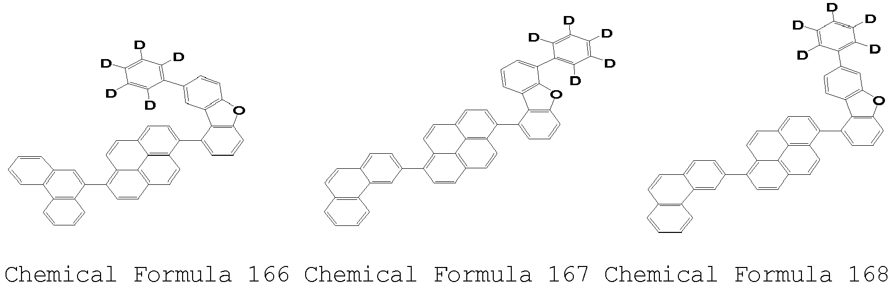

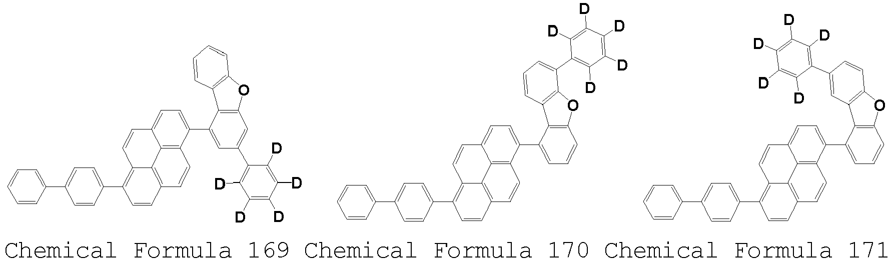

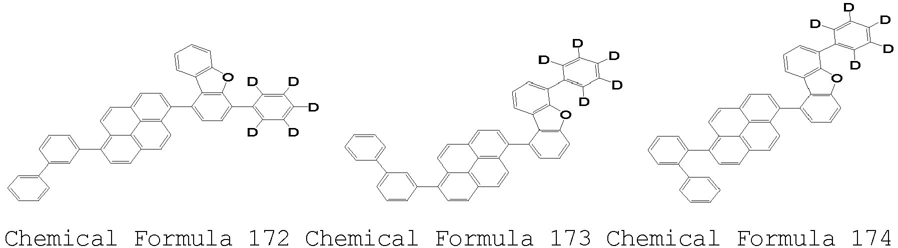

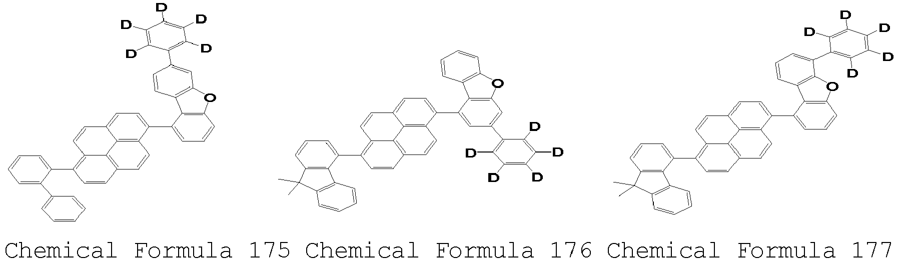

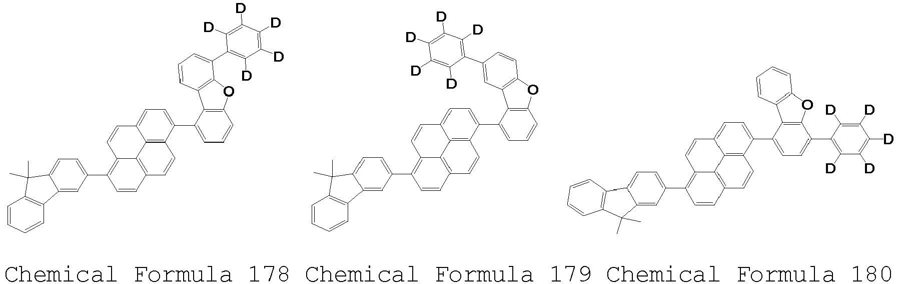

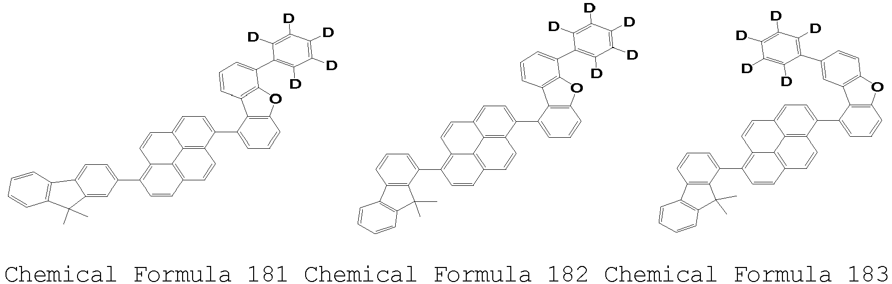

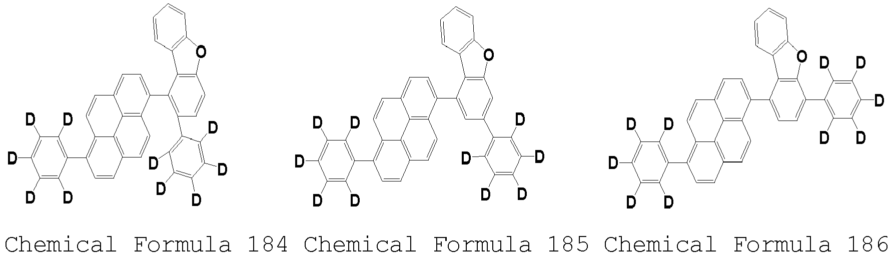

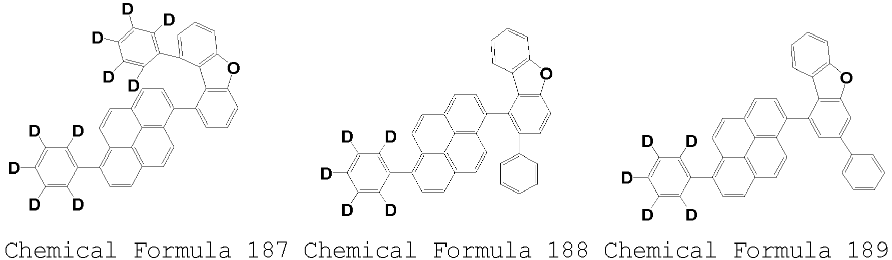

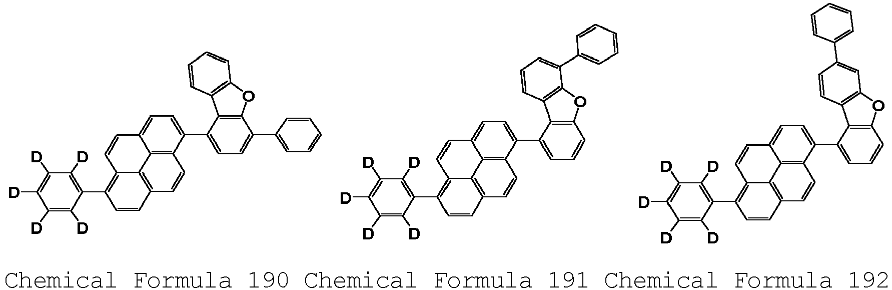

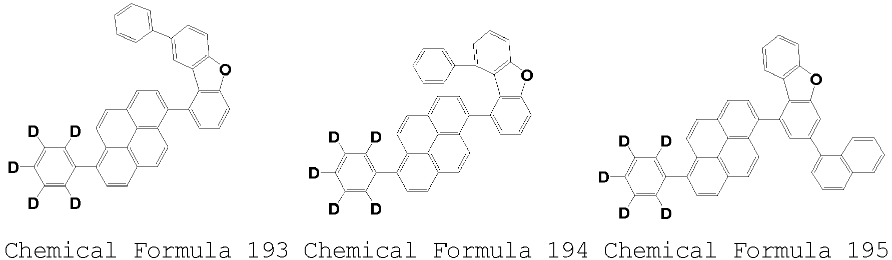

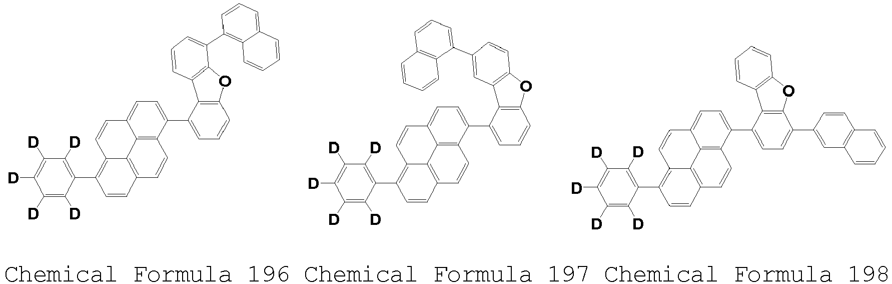

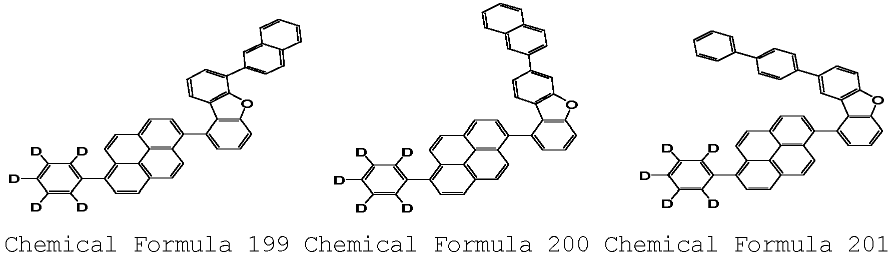

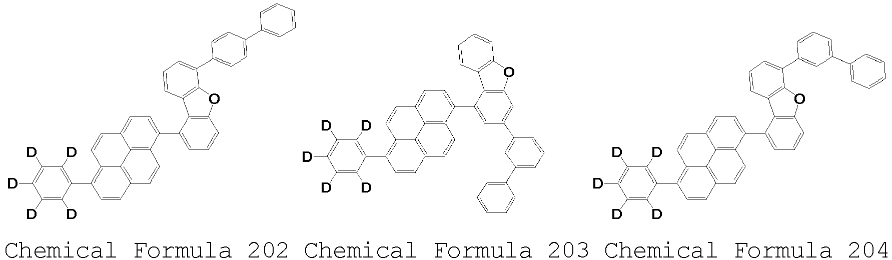

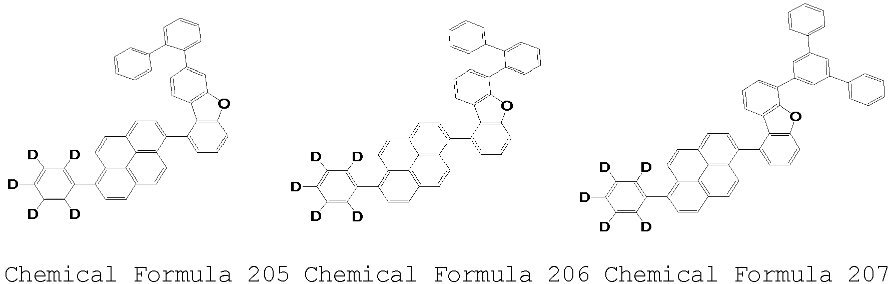

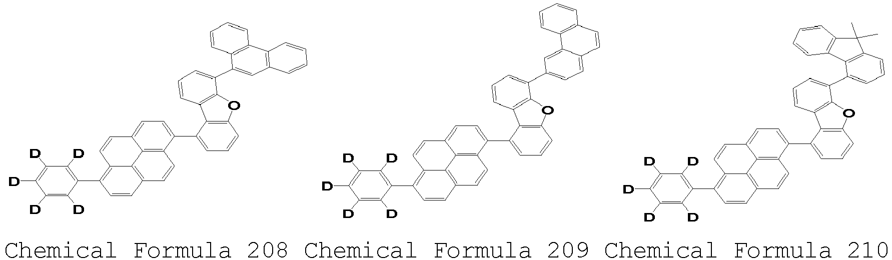

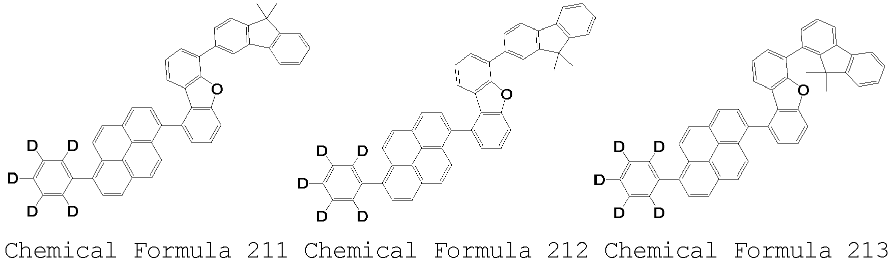

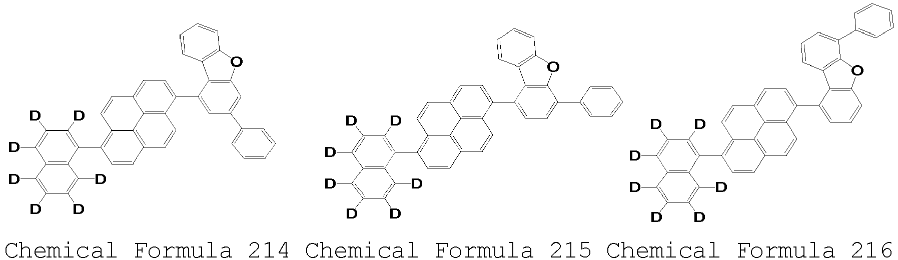

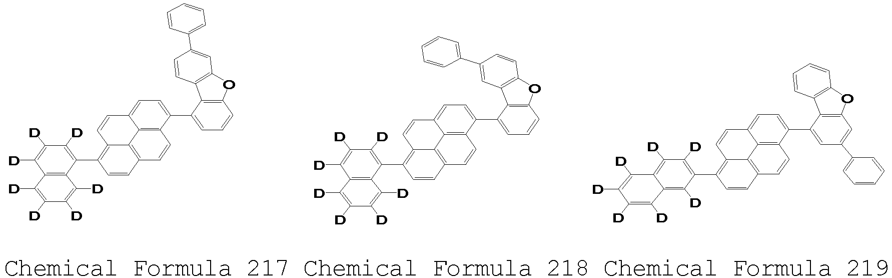

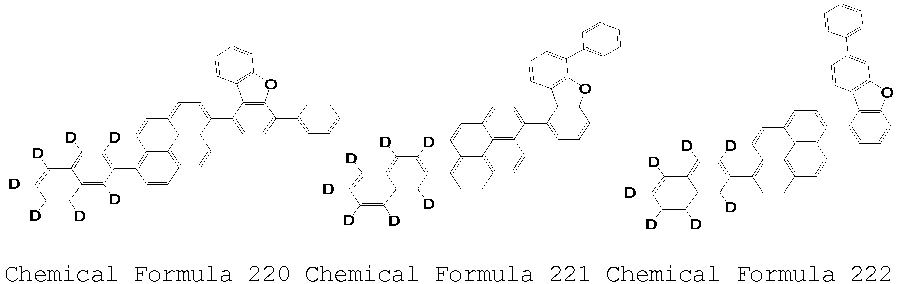

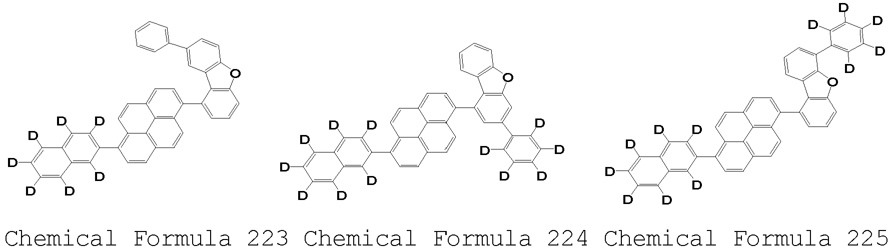

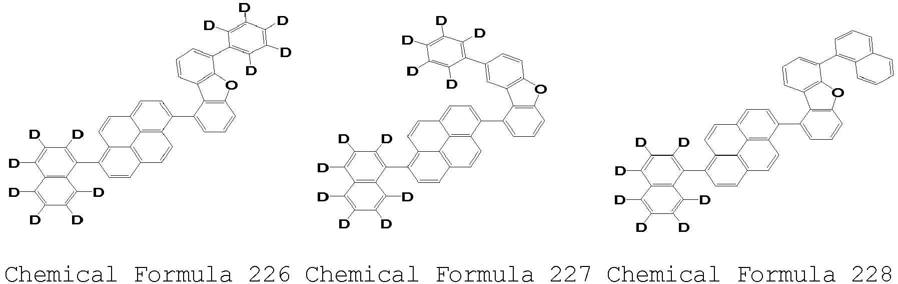

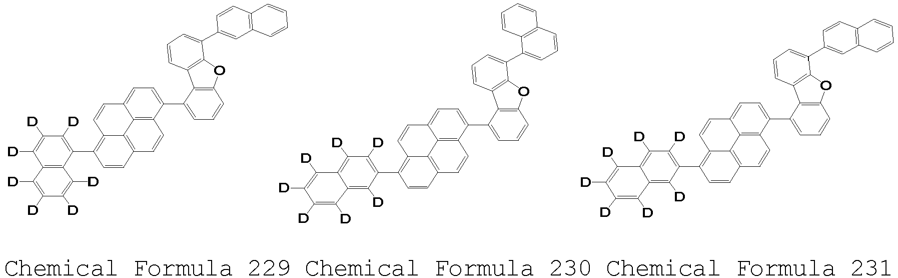

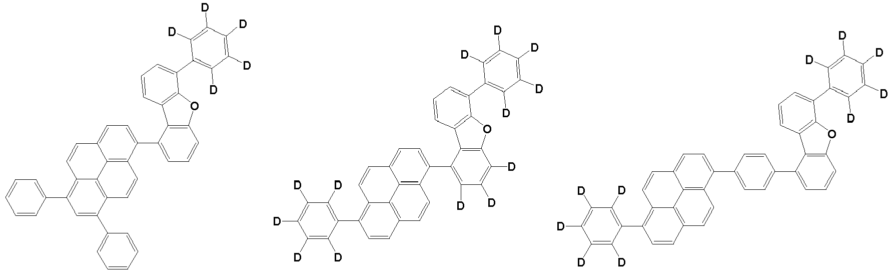

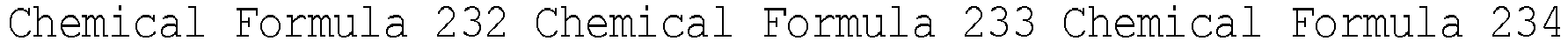

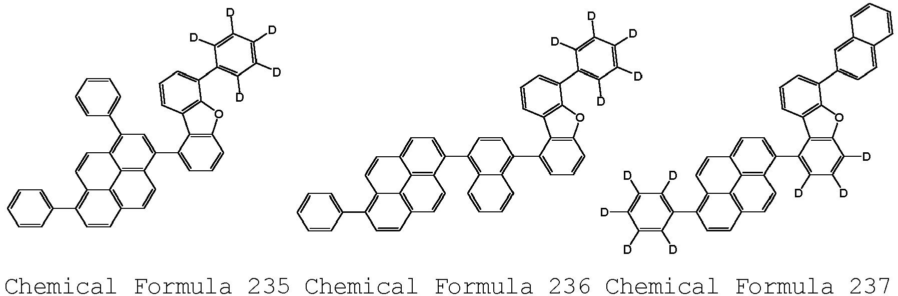

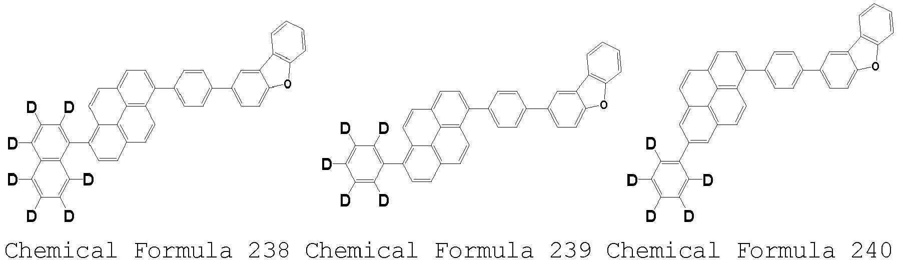

14. An organic light-emitting diode, comprising:
a first electrode;
a second electrode facing the second electrode; and
an organic layer interposed between the first electrode and the second electrode,
wherein the organic layer comprises the compound of any one of claims 1 to 13.

15. The organic light-emitting diode of claim 14, wherein the organic layer comprises at least one of a hole injection layer, a hole transport layer, a functional layer capable of both hole injection and hole transport, a light-emitting layer, an electron transport layer, and an electron injection layer.

16. The organic light-emitting diode of claim 15, wherein the organic layer interposed between the first electrode and the second electrode comprises a light-emitting layer composed of a host and a dopant, the compound servings as the host.

17. The organic light-emitting diode of claim 16, wherein the dopant is at least one represented by any one selected from the following Chemical Formulas D1 to D10: 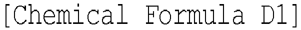 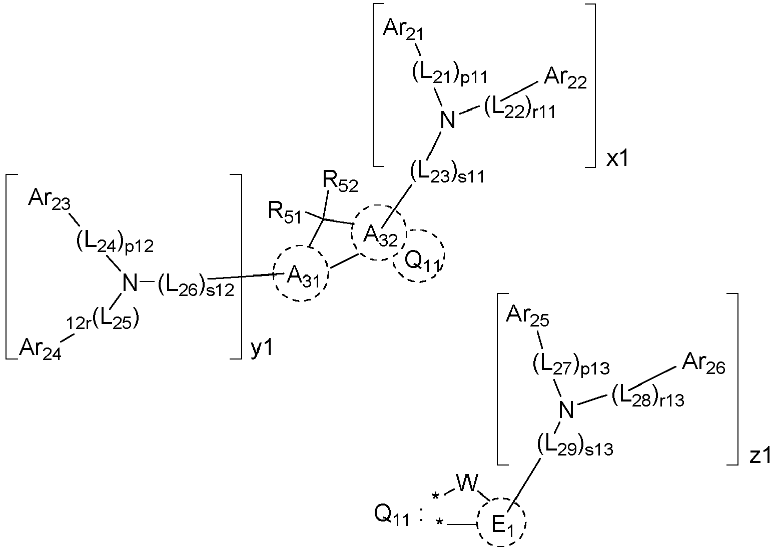 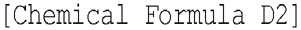 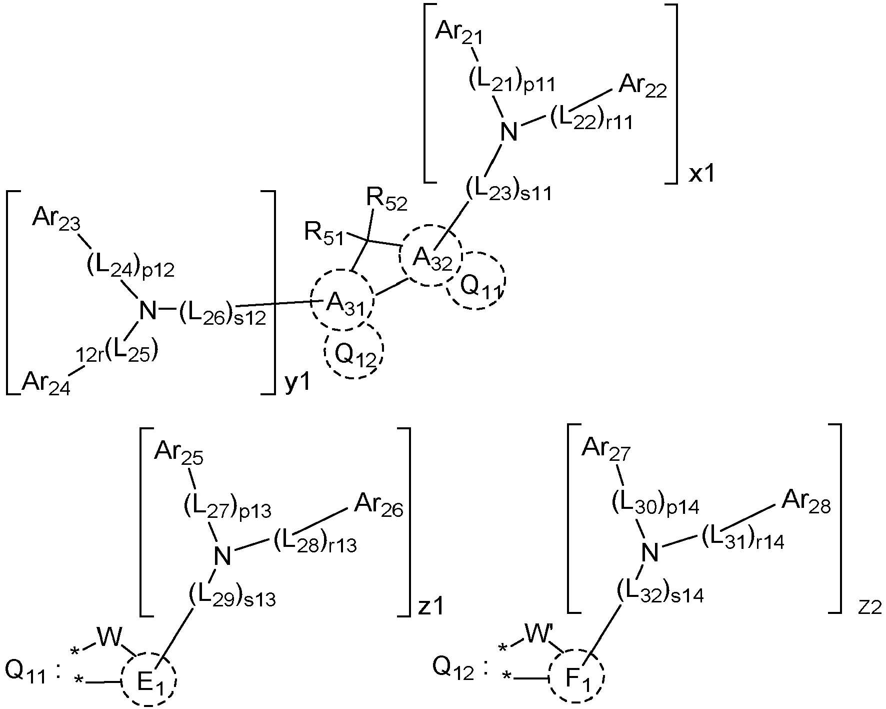
A₃₁, A₃₂, E₁, and F₁, which are same or different, are each independently a substituted or unsubstituted aromatic hydrocarbon ring of 6 to 50 carbon atoms, or a substituted or unsubstituted heteroaromatic ring of 2 to 40 carbon atoms,
wherein two adjacent carbon atoms of the aromatic ring A₃₁ and two adjacent carbon atoms of the aromatic ring A₃₂ form a 5-membered fused ring together with a carbon atom to which substitutents R₅₁ and R₅₂ are bonded;
linkers L₂₁ to L₃₂, which are same or different, are each independently selected from among a single bond, a substituted or unsubstituted alkylene of 1 to 60 carbon atoms, a substituted or unsubstituted alkenylene of 2 to 60 carbon atoms, a substituted or unsubstituted alkynylene of 2 to 60 carbon atoms, a substituted or unsubstituted cycloalkylene of 3 to 60 carbon atoms, a substituted or unsubstituted heterocycloalkylene of 2 to 60 carbon atoms, a substituted or unsubstituted arylene of 6 to 60 carbon atoms, and a substituted or unsubstituted heteroarylene of 2 to 60 carbon atoms;
W and W', which are same or different, are each independently any one selected from among N-R₅₃, CR₅₄R₅₅, SiR₅₆R₅₇, GeR₅₈R₅₉, O, S, and Se;
R₅₁ to R₅₉, and Ar₂₁ to Ar₂₈, which are same or different, are each independently any one selected from among a hydrogen atom, a deuterium atom, a substituted or unsubstituted alkyl of 1 to 30 carbon atoms, a substituted or unsubstituted aryl of 6 to 50 carbon atoms, a substituted or unsubstituted alkenyl of 2 to 30 carbon atoms, a substituted or unsubstituted alkynyl of 2 to 20 carbon atoms, a substituted or unsubstituted cycloalkyl of 3 to 30 carbon atoms, a substituted or unsubstituted cycloalkenyl of 5 to 30 carbon atoms, a substituted or unsubstituted heteroaryl of 2 to 50 carbon atoms, a substituted or unsubstituted heterocycloalkyl of 2 to 30 carbon atoms, a substituted or unsubstituted alkoxy of 1 to 30 carbon atoms, a substituted or unsubstituted aryloxy of 6 to 30 carbon atoms, a substituted or unsubstituted alkylthioxy of 1 to 30 carbon atoms, a substituted or unsubstituted arylthioxy of 5 to 30 carbon atoms, a substituted or unsubstituted alkylamine of 1 to 30 carbon atoms, a substituted or unsubstituted arylamine of 5 to 30 carbon atoms, a substituted or unsubstituted alkylsilyl of 1 to 30 carbon atoms, a substituted or unsubstituted arylsilyl of 5 to 30 carbon atoms, a substituted or unsubstituted alkylgermyl of 1 to 30 carbon atoms, a substituted or unsubstituted arylgermyl of 1 to 30 carbon atoms, a cyano, a nitro, and a halogen, wherein R₅₁ and R₅₂ together can form a mono- or polycyclic aliphatic or aromatic ring that can be a heterocyclic ring bearing a heteroatom selected from among N, O, P, Si, S, Ge, Se, and Te as a ring member;
p11 to p14, r11 to r14, and s11 to s14 are each independently an integer of 1 to 3, wherein when any of them is 2 or greater, the corresponding linkers L₂₁ to L₃₂ are same or different,
x1 is 1, and y1, z1, and z2, which are same or different, are each independently an integer of 0 to 1; and
Ar₂₁ can form a ring with Ar₂₂, Ar₂₃ can form a ring with Ar₂₄, Ar₂₅ can form a ring with Ar₂₆, and Ar₂₇ can form a ring with Ar₂₈,
two adjacent carbon atoms of the A₃₂ ring moiety of Chemical Formula D1 can occupy respective positions * of Structural Formula Q₁₁ to form a fused ring, and
two adjacent carbon atoms of the A₃₁ ring moiety of Chemical Formula D2 can occupy respective positions * of structural Formula Q₁₂ to form a fused ring, and two adjacent carbon atoms of the A₃₂ ring moiety of Chemical Formula D2 can occupy respective positions * of Structural Formula Q₁₁ to form a fused ring, 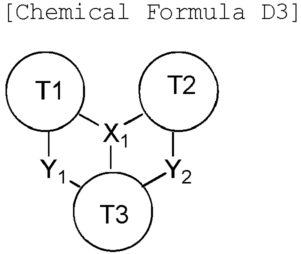
wherein,
X₁ is any one selected from among B, P, and P=O
T₂ to T₃, which are same or different, are each independently a substituted or unsubstituted aromatic hydrocarbon ring of 6 to 50 carbon atoms, or a substituted or unsubstituted heteroaromatic ring of 2 to 40 carbon atoms;
Y₁ is any one selected from among N-R₆₁, CR₆₂R₆₃, O, S, and SiR₆₄R₆₅;
Y₂ is any one selected from among N-R₆₆, CR₆₃R₆₈, O, S, and SiR₆₉R₇₀;
R₆₁ to R₇₀, which are same or different, are each independently any one selected from among a hydrogen atom, a deuterium atom, a substituted or unsubstituted alkyl of 1 to 30 carbon atoms, a substituted or unsubstituted aryl of 6 to 50 carbon atoms, a substituted or unsubstituted cycloalkyl of 3 to 30 carbon atoms, a substituted or unsubstituted heteroaryl of 2 to 50 carbon atoms, a substituted or unsubstituted alkoxy of 1 to 30 carbon atoms, a substituted or unsubstituted aryloxy of 6 to 30 carbon atoms, a substituted or unsubstituted alkylthioxy of 1 to 30 carbon atoms, a substituted or unsubstituted arylthioxy of 5 to 30 carbon atoms, a substituted or unsubstituted alkylamine of 1 to 30 carbon atoms, a substituted or unsubstituted arylamine of 5 to 30 carbon atoms, a substituted or unsubstituted alkylsilyl of 1 to 30 carbon atoms, a substituted or unsubstituted arylsilyl of 5 to 30 carbon atoms, a cyano, and a halogen, and wherein at least one of R₆₁ to R₇₀ can be connected to at least one of T₁ to T₃ to form an additional mono- or polycyclic aliphatic or aromatic ring; 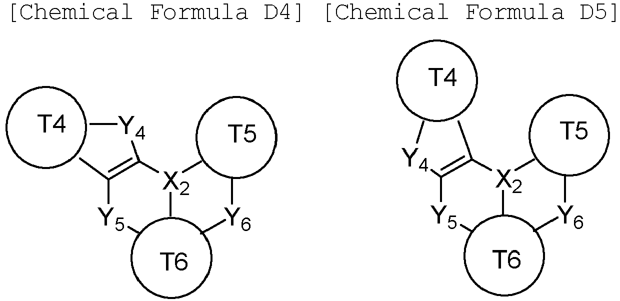
wherein,
X₂ is any one selected from among B, P, and P=O,
T4 to T6 are as defined for T1 to T3 in Chemical Formula D3,
Y₄ is any one selected from among N-R₆₁, CR₆₂R₆₃, O, S, and SiR₆₄R₆₅;
Y₅ is any one selected from among N-R₆₆, CR₆₇R₆₈, O, S, and SiR₆₉R₇₀;
Y₆ is any one selected from among N-R₇₁, CR₇₂R₇₃, O, S, and SiR₇₄R₇₅; and
R₆₁ to R₇₅ being as defined for R₆₁ to R₇₀ in Chemical Formula D3; 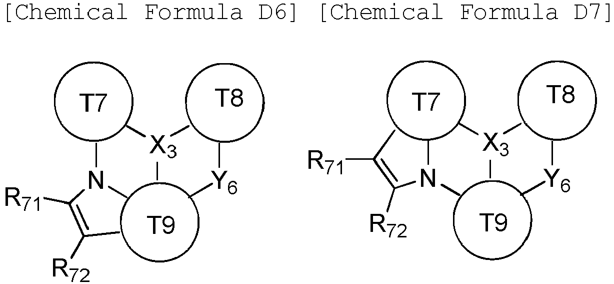
X₃ is any one selected from among B, P, and P=O,
T7 to T9 are defined as for T1 to T3 in Chemical Formula D3,
Y₆ is any one selected from among N-R₆₁, CR₆₂R₆₃, O, S, and SiR₆₄R₆₅;
R₆₁ to R₆₅ and R₇₁ to R₃₂ are each as defined for R₆₁ to R₇₀ in Chemical Formula D3,
wherein R₇₁ and R₇₂ may be connected to each other to form an additional mono- or polycyclic aliphatic or aromatic ring, or may be connected to the T₇ ring moiety or T₉ ring moiety to form an additional mono- or polycyclic aliphatic or aromatic ring; 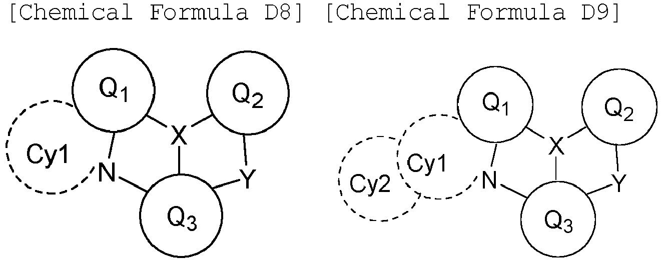 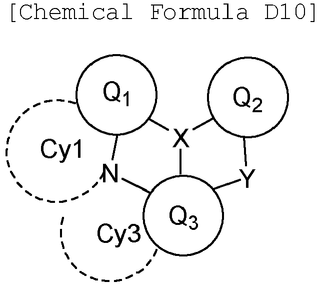
wherein,
X is any one selected from among B, P, and P=O,
Q₁ to Q₃ are each as defined for T1 to T3 in Chemical Formula D3,
Y is any one selected from among N-R₃, CR₄R₅, O, S, and Se,
R₃ to R₅ are each as defined for R₆₁ to R₇₀ in Chemical Formula D3,
R₃ to R₅ can each be connected to the Q₂ or Q₃ ring moiety to form an additional mono- or polycyclic aliphatic or aromatic ring,
R₄ and R₅ can be connected to each other to form an additional mono- or polycyclic aliphatic or aromatic ring,
the ring formed by Cy1 is a substituted or unsubstituted alkylene of 1 to 10 carbon atoms, except for the nitrogen (N) atom, the aromatic carbon atom of Q₁ to which the nitrogen (N) atom is connected, and the aromatic carbon atom of Q₁ to which Cy1 is to bond,
"Cy2" in Chemical Formula D9 forms a saturated hydrocarbon ring added to Cy1 wherein the ring formed by Cy2 is a substituted or unsubstituted alkylene of 1 to 10 carbon atoms, except for the carbon atoms included in Cy1, and
the ring formed by Cy3 in Chemical Formula D10 is a substituted or unsubstituted alkylene of 1 to 10 carbon atoms, except for the aromatic carbon atom of Q₃ to which Cy3 is to bond, the aromatic carbon atom of Q₃ to which the nitrogen (N) atom is connected, the nitrogen (N) atom, and the carbon atom of Cy1 to which the nitrogen (N) atom is connected,
wherein the term "substituted" in the expression "substituted or unsubstituted" used for compounds of Chemical Formulas D1 to D10 means having at least one substituent selected from the group consisting of a deuterium atom, a cyano, a halogen, a hydroxy, a nitro, an alkyl of 1 to 24 carbon atoms, a hydrogenated alkyl of 1 to 24 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 2 to 24 carbon atoms, an alkoxy of 1 to 24 carbon atoms, an alkylamino of 1 to 24 carbon atoms, a diarylamino of 12 to 24 carbon atoms, a diheteroarylamino of 2 to 24 carbon atoms, an aryl(heteroaryl)amino of 7 to 24 carbon atoms, an alkylsilyl of 1 to 24 carbon atoms, an arylsilyl of 6 to 24 carbon atoms, an aryloxy of 6 to 24 carbon atoms, and an arylthionyl of 6 to 24 carbon atoms.

18. The organic light-emitting diode of claim 15, wherein at least one selected from among the layers is deposited using a single-molecule deposition process or a solution process.

19. The organic light-emitting diode of claim 14, wherein the organic light-emitting diode is used for a device selected from among a flat display device; a flexible display device; a monochrome or grayscale flat illumination; and a monochrome or grayscale flexible illumination.
